(19) Europäisches Patentamt
European Patent Office
Office européen des brevets

(11) EP 2 149 370 A1

(12) EUROPEAN PATENT APPLICATION

(43) Date of publication:
**03.02.2010 Bulletin 2010/05**

(21) Application number: **09161266.3**

(22) Date of filing: **27.05.2009**

(51) Int Cl.:
*A61K 9/107* (2006.01) *C07D 487/08* (2006.01)
*C07D 471/08* (2006.01) *A61K 31/551* (2006.01)
*A61P 25/04* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **31.07.2008 IT MI20081428**
**25.02.2009 IT MI20090260**

(71) Applicant: **Neuroscienze Pharmaness S.C. A R.L.**
**09010 Pula (CA) (IT)**

(72) Inventors:
- **Lazzari, Paolo**
  **09010, Pula (CA) (IT)**
- **Loriga, Giovanni**
  **07100, Sassari (IT)**
- **Ruiu, Stefania**
  **09012, Capoterra (CA) (IT)**
- **Manca, Ilaria**
  **07100, Sassari (IT)**
- **Pani, Luca**
  **09100, Cagliari (IT)**
- **Pinna, Gerard Aime**
  **07100, Sassari (IT)**

(74) Representative: **Sama, Daniele et al**
**Sama Patents**
**Via Morgagni, 2**
**20129 Milano (IT)**

(54) **Diazabicyclic compounds and microemulsions thereof**

(57) Pharmaceutical compositions in the form of microemulsions comprising the following components, in amounts expressed as % by weight, the sum of the components being 100%:
S) from 0.01 to 95% of one or more pharmaceutically acceptable compounds, selected from the following classes: surfactants; polymers forming organized structures as micelles, liquid crystals, vesicles in the liquid in which they are solubilized;
O) from 0.01 to 95% of one or more oils,
PA) from 0.001 to 90% of diazabicyclic compounds having formula A':

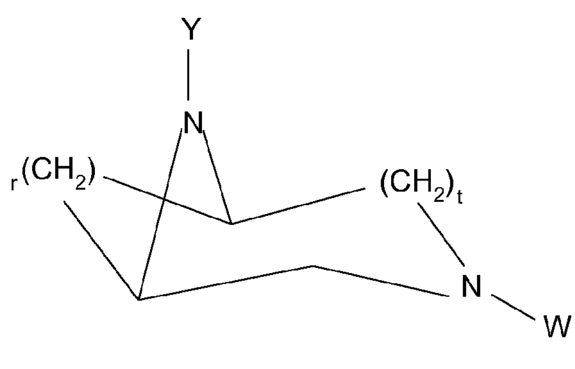

A'

wherein t, r, Y, W have the meanings reported in the description,
AD) from 0 to 60% by weight of one or more compounds selected from the following classes:
- modifiers of water and/or oil polarity,
- modifiers of the curvature of the film of component S),
- co-surfactants,



Printed by Jouve, 75001 PARIS (FR)

(Cont. next page)

water from 0.01 to 99.9%.

## Description

The present invention relates to pharmaceutical compositions in the form of microemulsions comprising as active principles diazabicyclic compounds having affinity for the opioidergic receptors μ ad/or δ and/or k and/or for the receptorial subclasses thereof, the corresponding solvates and pharmaceutically acceptable salts.

More specifically the present invention relates to pharmaceutical compositions in the form of microemulsion wherein the diazabicyclic active principles are diazabicyclic heptane, octane, nonane and decane compounds.

More specifically the invention relates to pharmaceutical compositions in the microemulsion form comprising the above mentioned diazabicyclic compounds and an oil phase, the ratio by weight surfactant/tricyclic compound being lower than that of the microemulsions wherein the oil phase is absent.

Diazabicyclic compounds having affinity for opioidergic receptors are known in the prior art. In US patent application 2003/0195,217 3,9-diazabicyclo[3.3.1]nonane compounds having central analgesic activity mediated by the opioidergic receptors, are described. The analgesic activity is comparable to that induced by morphine, but with lower side effects. In this patent application also the pharmaceutical forms containing these compounds are described. The reported liquid pharmaceutical forms are drops, elixirs, syrups and injectable forms. The study of these diazabicyclic compounds has also been treated in the publication in Bioorganic & Medicinal Chemistry, 10 (2002) 1929-1937 wherein it is pointed out the effect of various substituents of the bicyclic structure on the affinity towards the opioidergic receptors μ, δ and k. Another class of diazabicyclic compounds having affinity towards the opioidergic receptors μ, δ and k, and the corresponding pharmaceutical forms, is described in patent application WO 2004/011,468. In particular, said class of compounds is formed of diazabicyclo nonanes and decanes of general formula

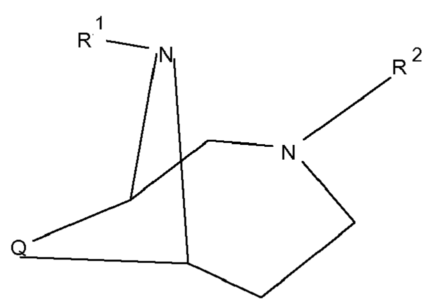

(A)

wherein Q is $-CH_2-CH_2-$ or $-CH_2-CH_2-CH_2-$ and one between $R^1$ and $R^2$ is $-CH_2-CH_2-CH_2-R^3$ or $-CH_2-CH=CH-R^3$ or $-CH_2-C{\equiv}C-R^3$ wherein $R^3$ is aryl or heteroaryl and the other between $R^1$ and $R^2$ is $-C(O)R^4$ wherein $R^4$ is alkyl, or cycloalkyl, or cycloalkylalkyl, or aryl or arylalkyl. The liquid pharmaceutical forms mentioned in said patent application are solutions, suspensions and emulsions.

US Patent 5,672,601 describes 3,8-diaza-bicyclo[3.2.1]-octane compounds, and the corresponding pharmaceutical forms, having central analgesic activity mediated by the opioidergic receptors μ. The mentioned liquid pharmaceutical forms are drops, elixirs, syrups and injectable forms.

Patent application WO 2005/108,402 relates to 3,6-diazabicyclo[3.1.1]heptane derivatives having central analgesic activity selectively mediated by the receptors μ. The mentioned liquid pharmaceutical forms are drops, elixirs, syrups and injectable forms.

The above mentioned patents and patent applications describe the use of the diazabicyclic compounds having affinity for the opioidergic receptors for the pain treatment.

In the above mentioned patent application WO 2004/011,468 it is stated that the opioidergic compounds, besides the use for the treatment of different kinds of pain (post-surgery pain, chronic pain such as neuropathic pain), can be used for the therapeutical treatment of other diseases and disorders such as allergic dermatitis, sexual disfunctions, alcoholism, nausea, vomit, depression, tabagism, obesity and disorders associated to the food intake, use of abuse substances (for ex. heroin, cocaine), spinal lesion, cerebral trauma, shock, stroke, gastrointestinal disorders. Eur. J. Pharmacol. 296 (1996) 199-207 reports the antiproliferative activity of agonist compounds of opioidergic receptors on a human cellular line of breast tumour. The article therefore discloses the antitumoural activity of said agonist compounds. In the articles Veterinary Ophthalmology (2003) 6, 1, 73-76; Exp. Eye Res. 2007 January 84(1) 185-190; British Journal of Anaesthesia 1998, 81 606-607 it is pointed out the capability of agonist compounds of the opioidergic receptors of reducing the intraocular pressure and consequently the use of said compounds for eye diseases, such as glaucoma.

In the article published in Neuropeptides (1999) 33(5) 360-368 it is reported the effect of compounds modulating the opioidergic receptors on the food intake, in particular it is stated that agonists and antagonists of the opioidergic receptors, can respectively, increase and decrease the food intake.

Patent application WO 06/113,468 describes the use of compounds modulating the opioidergic receptors for the treatment of arthritis, psoriasis, asthma, cardiac disorders, sexual disfunctions, pain, incontinence and disorders of the urogenital tract.

Patent application US 2005/0203,123 relates to compounds antagonist of the opioidergic receptors and their use for the treatment of gastrointestinal disorders, pain, obesity, Alzheimer and Parkinson diseases. The use of opioidergic compounds for the treatment of diabetes and atherosclerosis is described in patent applications WO 05/092,836 and WO 05/066,164.

Patent application WO 04/089,372 describes the use of compounds capable of modulating the opioidergic receptors for the treatment and prevention of central nervous system disorders, such as anxiety and depression.

Patent application WO 04/060,321 relates to therapeutic compositions comprising agonists of the opioidergic receptors with cardioprotective effects.

Patent applications WO 02/42,309, WO 01/46,198 describe the use of opioidergic compounds as immunostimulants or immunosuppressants.

The compounds of the above mentioned patents and patent applications are obtained in oil form. For their use in therapy said compounds must be salified to increase their bioavailability. As a matter of fact the compounds as such are substantially insoluble in water. Further the corresponding salts show however a limited water-solubility. Therefore the solubility characteristics of these compounds and of the corresponding salts can hinder or limit the use of these classes of compounds as effective therapeutic agents in the treatment of the above mentioned pathologies and disorders, both when using the compounds as such or salified, and when using the above mentioned pharmaceutical dosage forms.

The known water-based pharmaceutical dosage forms of the diazabicyclic compounds or their salts do not have a high stability and show therefore a limited shelf life.

It is known to use surfactants pharmaceutical formulations. It is well known that surfactants, especially if present in significant amounts, can give side effects as for example anaphylactic shock.

The need was felt to have available liquid pharmaceutical dosage forms comprising diazabicyclic compounds having an improved shelf life, showing the following combination of properties:

- re-establishment of the homogeneity and shelf life of the starting composition even when separated phases are formed in the formulation,
- dilutable with water or with aqueous solutions,
- solubilization in said liquid pharmaceutical composition of the active ingredients at concentrations at least equal to those suitable for an effective therapeutic treatment in human beings and in mammals,
- reduced ratio by weight surfactant/active principle, to avoid the prior art drawbacks.

Pharmaceutical compositions have been surprisingly and unexpectedly found by the Applicant solving the above mentioned technical problem.

It is an object of the present invention pharmaceutical compositions in the form of microemulsions comprising the following components, in amounts expressed as % by weight,:

S) from 0.01 to 95% of one or more pharmaceutically acceptable compounds, selected from the following classes:

- surfactants selected from non-ionic, anionic, cationic and amphoteric surfactants, optionally containing fluorine atom,
- polymers forming organized structures such as the following: aggregates, micelles, liquid crystals, vesicles, in the liquid in which they are solubilized;

O) from 0.01 to 95% of one or more oils selected from the following classes of pharmaceutically acceptable compounds:

- $C_4$-$C_{32}$ acid esters, optionally containing one or more unsaturations of ethylenic type,
- $C_4$-$C_{32}$ acids, optionally containing one or more unsaturation of ethylenic type, which are used when the final composition has a pH such that the acid is not converted into the corresponding salt,

PA) from 0.001 to 90% of diazabicyclic compounds of formula A':

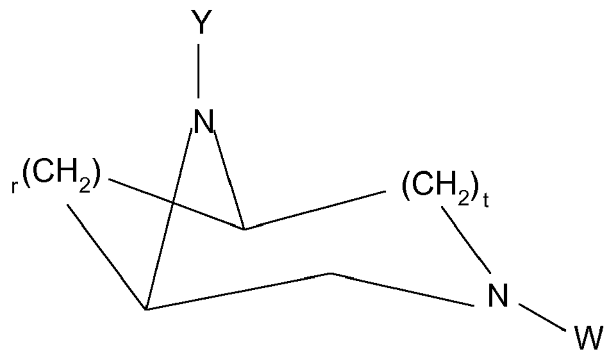

A'

wherein:

t is an integer equal to 1 or 2,
r is an integer equal to 1, 2 or 3 and has the following values, depending on those of t:

- r= 1, 2 or 3 when t=1,
- r= 2, 3 when t=2,

when t=1, one between the substituents W, Y of the nitrogen atoms of the diazabicyclic ring is an acyl group -C(O)-$R_B$, wherein $R_B$ is a $C_1$-$C_{10}$ alkyl group, linear or branched when possibile, the other substituent is selected from:

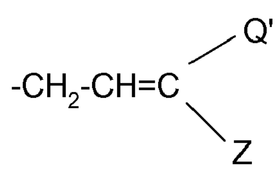

(XIV)

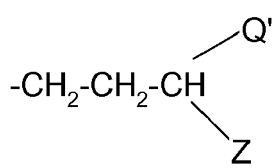

(XV)

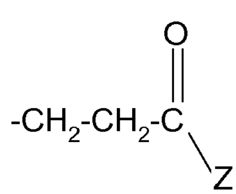

(XVI)

wherein:

Z has the following meanings:

- $C_6$-$C_{10}$ aryl group,
- $C_5$-$C_7$ cycloalkyl group,
- aromatic heterocyclic group with a 5 or 6 atom ring, containing at least an heteroatom selected from nitrogen, oxygen, sulphur,

Q' is selected among: hydrogen, $C_1$-$C_4$ alkyl, linear or branched when possible, $C_5$-$C_7$ cycloalkyl, phenyl,

when t=2, one of the substituents W, Y of the nitrogen atoms of the diazabicyclic ring is an acyl group -C(O)-$R_B$, wherein $R_B$ is as defined above, the other substituent remained between W and Y has the following meanings:

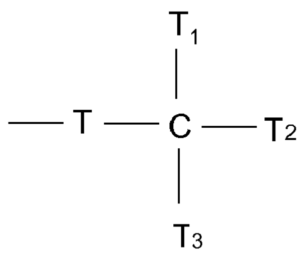 (XVII)

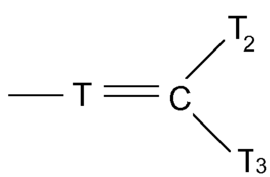 (XVIII)

-T≡C-$T_4$ (XIX)

wherein:

- T is a saturated or unsaturated $C_2$-$C_9$ aliphatic chain, linear or branched when possible,
- $T_1$ has the following meanings: hydrogen, isothiocyanate, CN, OR', C(O)OR', C(O)R', C(O)NR'R", NR'R", R' and R", equal to or different from each other, linear or branched are selected from hydrogen, linear or branched when possible $C_1$-$C_7$ alkyl, $C_1$-$C_7$ alkoxy, $C_1$-$C_7$ alkylthio, $C_1$-$C_7$ haloalkyl, $C_1$-$C_7$ haloalkoxy, $C_3$-$C_{15}$ cycloalkyl, aryl, heteroaryl,
- $T_2$ and $T_3$, equal to or different from each other, are substituents selected from:

  - hydrogen, with the following provisos:

    - when one substituent between W and Y has formula (XVII), at least one of $T_1$, $T_2$ and $T_3$ is not hydrogen,
    - when one substituent between W and Y has formula (XVIII), at least one of $T_2$ and $T_3$ is not hydrogen,
    - linear or branched when possible $C_1$-$C_{10}$ alkyl,
    - aryl or heteroaryl,
    - $C_3$-$C_{15}$ cycloalkyl,

  - $T_4$ has the same meanings of $T_2$ and $T_3$, but excluding hydrogen,

AD) from 0 to 60% by weight of one or more compounds selected from the following classes:

- modifiers of the water and/or oil polarity,
- modifiers of the curvature of the film of component S),
- co-surfactants,

WA) from 0.01 to 99.9% of water or a saline aqueous solution, optionally buffered,

the sum of the components being 100%,
wherein the ratio by weight S)/PA) is lower than at least 10%, preferably lower than at least 30%, with respect to the ratio by weight S)/PA) in the microemulsions having the same composition but not including component O).

The compositions of the invention in the form of microemulsions are limpid and transparent, preferably liquid. When the viscosity is very high, the compositions of the invention can be in gel form.

In component S) the surfactants containing fluorine atoms can have (per)fluorinated chains, for example (per) fluoropolyether chains.

The liquids wherein the polymers of component S) are solubilized to form the organized structures are water and/or oil. The oils that can be used are indicated hereinafter and may be of both natural and synthetic origin.

In component PA) $R_B$ can optionally contain $C_3$-$C_{10}$ cycloalkyl or $C_6$-$C_{10}$ aryl rings, or saturated or unsaturated $C_3$-$C_{10}$ heterocycles containing one or more heteroatoms selected from N, S and O.

In Z the $C_6$-$C_{10}$ aryl group is optionally substituted with one or more groups equal to or different from each other, selected from: $C_1$-$C_3$ alkoxy, $C_1$-$C_2$ haloalkoxy, $C_1$-$C_3$ alkyl, halogen, carboxy, cyano, nitro, -CONHZ', wherein Z' has the meaning of $C_1$-$C_4$ alkyl, linear or branched when possible.

In Z the aromatic heterocyclic group with a 5 or 6 atom ring is optionally fused with a benzene ring. Furthermore the aromatic heterocyclic group with a 5 or 6 atom ring can optionally be substituted with one or more substituents equal to or different from each other selected from those mentioned above when Z has the meaning of $C_6$-$C_{10}$ aryl group.

When Q' has the meaning of $C_5$-$C_7$ cycloalkyl or phenyl, these groups can optionally be substituted with one or more groups equal to or different from each other selected from those above mentioned when Z has the meaning of $C_6$-$C_{10}$ aryl group.

When $T_1$ has the meaning of OR', C(O)OR', C(O)R', C(O)NR'R", NR'R", and R' and/or R" have the meaning of $C_3$-$C_{15}$ cycloalkyl, this cycloalkyl can optionally contain one or more heteroatoms, preferably selected from O, S, N.

When $T_1$ has the meaning of OR', C(O)OR', C(O)R', C(O)NR'R", NR'R", and R' and/or R" have the meaning of $C_3$-$C_{15}$ cycloalkyl, aryl and heteroaryl, these structures can optionally be substituted with one or more groups selected from hydroxy, halogen, $C_1$-$C_{10}$ alkyl, linear or branched when possible.

When $T_2$ and $T_3$ have the meaning of $C_1$-$C_{10}$ alkyl, linear or branched when possible, the alkyl can optionally be substituted with one or more groups, equal to or different from each other, selected from hydroxy, halogen, CN, $C_3$-$C_{15}$ cycloalkyl, aryl or heteroaryl. The $C_3$-$C_{15}$ cycloalkyl can optionally contain one or more heteroatoms, preferaly selected from O, S, N. The $C_3$-$C_{15}$ cycloalkyl, aryl and heteroaryl can optionally be substituted with one or more groups selected from hydroxy, halogen, $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy, all these structures being linear or branched when possible.

When $T_2$ and $T_3$ have the meaning of aryl or heteroaryl, said aryl or heteroaryl can optionally be substituted with one or more of the following groups, equal to or different from each other, selected from:

linear or branched when possible $C_1$-$C_{10}$ alkyl, optionally substituted with one or more groups, equal to or different from each other, selected from hydroxy, halogen, CN;

$C_3$-$C_{15}$ cycloalkyl, optionally containing one or more heteroatoms, preferably selected from O, S, N. The cycloalkyl can optionally be substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy;

alkylaryl or aryl or heteroaryl or alkylheteroaryl, optionally substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

When $T_2$ and $T_3$ have the meaning of $C_3$-$C_{15}$ cycloalkyl, said cycloalkyl can optionally contain one or more heteroatoms, preferably selected from O, S, N. Furthermore said cycloalkyl can optionally be substituted with one or more of the following groups, equal to or different from each other, selected from:

linear or branched when possible $C_1$-$C_{10}$ alkyl, optionally substituted with one or more groups, equal to or different from each other, selected from hydroxy, halogen, CN,

$C_3$-$C_{15}$ cycloalkyl, optionally containing one or more heteroatoms, preferably selected from O, S, N. Furthermore said cycloalkyl can optionally be substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy,

arylalkyl or aryl or heteroaryl or heteroarylalkyl, optionally substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

The preferred compounds PA) are those wherein one of the substituents between W and Y is selected from $-C(O)-CH_3$ or $-C(O)-C_2H_5$, and that remained between W and Y:

- when t=1, is selected from the group (XIV) or (XV),
- when t=2, is selected from the group (XVII) wherein $T_1$=H, or group (XVIII).

The most preferred compounds PA) are those wherein one of the substituents between W and Y is selected from $-C(O)-CH_3$ or $-C(O)-C_2H_5$, and the other substituent:

- when t=1, is selected from the group (XIV) or (XV), wherein at least one of the substituents Q' and Z has the meaning of aryl,
- when t=2, is selected from the group (XVII) wherein $T_1$=H, or group (XVIII), wherein at least one among the substituents $T_2$ and $T_3$ has the meaning of aryl.

By microemulsion a system formed of two or more immiscible phases with each other, transparent, isotropic, comprising at least an aqueous phase and at least an oil phase is meant, wherein the various phases are stabilized by component S), optionally in the presence of one or more co-surfactants. The latter compounds are defined hereinafter. See for example R.K. Mitra, Physicochemical investigations of microemulsification of eucalyptus oil and water using mized surfactants (AOT+ Brij-35) and butanol, J. Colloid and Interface Science, 283 (2005) 565-577.

The preferred microemulsions of the invention are the following (% by weight):

- Component S) from 0.01 to 60%,
- Component O) from 0.01 to 90%,
- Component PA) from 0.001 to 50%,
- Component AD) from 0 to 20%,
- Component WA) from 0.1 to 99.9%,

the sum of the components being 100%.

The microemulsions having the following composition are more preferred:

- Component S) from 0.01 to 50%,
- Component O) from 0.01 to 90%,
- Component PA) from 0.05 to 50%,
- Component AD) from 0 to 10%,
- Component WA) from 10 to 99.9%,

the sum of the components being 100%.

The microemulsions having the following composition are still more preferred:

- Component S) from 0.01 to 50%,
- Component O) from 0.01 to 50%,
- Component PA) from 0.05 to 50%,
- Component AD) from 0 to 10%,
- Component WA) from 20 to 99.9%,

the sum of the components being 100%.

The preferred surfactants component S) are the non-ionic and anionic ones. Among the non-ionic surfactants the most preferred are those containing polyoxyalkylene chains, preferably polyoxyethylene chains. The following can for example be mentioned:

polyoxyl 35 castor oil, known for example by the commercial name Cremophor® EL (BASF), produced by ethoxylation of castor oil,

polyoxyl 40 hydrogenated castor oil, known for example by the commercial name Cremophor® RH40 (BASF), produced by ethoxylation of hydrogenated castor oil, polyethylenglycol 15 hydroxystearate, known for example by the commercial name Solutol® HS15 (BASF), produced by reaction of 15 moles of ethylene oxide with 1 mole of 12-hydroxystearic acid,

polyoxyethylene polysorbate, such as Tween® 80, Tween® 20, Tween® 60, Tween® 85,

sorbitan esters of fatty acids, such as sorbitan monolaurate and sorbitan monostearate, commercialized for example

with the name Span® 20 and Span® 60, respectively,
vitamin E/TPGS: tocopheryl propylenglycol 1000 succinate,
polyoxyethylen ethers of fatty acids, such as those of the series Brij®, for example Brij® 35, Brij® 76, Brij® 98,
PEG-12-acyloxy-stearates, see for example C.E. McNamee et al, in "Physicochemical Characterization of PEG 1500-12-acyloxy-stearate micelles and liquid cristalline phases", Langmuir, 2005, 21, 8146-8154, among the polyoxyethylene ethers of fatty acids the following ones can for example be mentioned:

- PEG 1500 mono-12-capryloyloxy stearate (PEG 1500-$C_{18}C_8$)
- PEG 1500 mono-12-caproyloxy stearate (PEG 1500-$C_{18}C_{10}$)
- PEG 1500 mono-12-lauroyloxy stearate (PEG 1500-$C_{18}C_{12}$)
- PEG 1500 mono-12-myristoyloxy stearate (PEG 1500-$C_{18}C_{14}$)
- PEG 1500 mono-12-palmitoyloxy stearate (PEG 1500-$C_{18}C_{16}$)

Among the anionic surfactants the following can for example be mentioned: soya lecithin, for example known by the commercial name Epikuron® 200, bis-2-ethylhexylsulpho-succinate, (AOT), sodium taurocholate.
Among the cationic surfactants, hexadecyl-trimethylammonium bromide (CTAB) and didodecylammonium bromide (DDAB) can for example be mentioned.
The polymers which can be used as component S) must be soluble in the aqueous phase and/or in the oily phase. By soluble it is meant that the polymers must reach in the phase in which they are soluble concentrations at least equal to those allowing the formation of organized structures such as aggregates, micelles, liquid crystals, vesicles. The presence of said organized structures can be detected by specific techniques of the physical chemistry of the dispersed systems, such as Laser Light Scattering (LLS), Neutron Scattering, microscopy.
As said, the polymers component S) can be used also in combination with the above mentioned surfactants. Also in this case the concentration of the solubilized polymer in the liquid phase used must be such to form the above mentioned organized structures.
The polymers component S) are for example polyvinylpyrrolidone and vinylpyrrolidone/vinyl acetate copolymers, commercialized for example by the name Kollidon®, as Kollidon® 12PF and Kollidon® 17PF (BASF), and the block copolymers containing polyoxyethylene chains, more preferably containing polyoxyethylene chains (PEO), such as the PEO block copolymers with polyoxyprpylene chains (PPO) characterized by PEO-PPO-PEO structures, commercially available for example with the trademark Pluronic® or Poioxamer® or Lutrol®, as Lutrol® F68 and Lutrol® F127 commercialized by Basf.
The esters of component O) are preferably obtained by esterification of the corresponding acid with an alcohol having an aliphatic chain, preferably a $C_1$-$C_5$ chain, or a polyoxyalkylene chain, or with glycerine. In this case mono-, di- or triglycerides are obtained.
The following esters can for example be mentioned:

oleoyl macrogol 6 glyceride (unsaturated polyglycosylated glyceride), commercialized for example by the trademark Labrafil® 1944 CS, (Gattefossé),
propylenglycol caprylate caprate, known for example by the commercial name Labrafac® PG (Gattefossé),
propylenglycol monoester of the caprylic acid, commercialized for example by the trademark Capmul® PG-8 (Abitec),
glycerol oleate (ex. Peceol® (Gattefossé)),
medium chain mono- and diglycerides, for example glycerides of the capric and caprylic acid (ex. Capmul® MCM (Abitec), Imwitor® 308 (Sasol)),
polyglycerol oleate (ex. Pluro® oleic (Gattefossé)),
triglycerides of the capric/caprylic acid (ex. Miglyol® 812 e Miglyol® 810 (Sasol), Labrafac® CC CS (Gattefossé)),
ethyl butyrate, ethyl caprylate, ethyl oleate,
tripalmitine, commercialized for example by the name DYNASAN® 116 by Sasol.

Also vegetable oils of pharmaceutical purity grade. containing one or more esters mentioned above can be used. The soya oil can be for example mentioned.
Among the acids of component O), the stearic acid, the omega-3 and omega-6 acids can be mentioned.
In component AD) the modifiers of the water and/or oil polarity can for example be polyethylenglycols. Lutrol®E300 and Lutrol®E400 (BASF) can be mentioned. Aliphatic alcohols, for example ethanol, can also be used.
In component AD) the modifiers of the curvature of the component S) film are for example aliphatic alcohols, preferably $C_2$-$C_5$.
In component AD) the co-surfactants can for example be surfactant compounds as defined above, or aliphatic alcohols, preferably having a chain with at least 6 carbon atoms. There can be mentioned for example:

propylene glycol monolaurate, known for example by the commercial name Capmul® PG12 (Gattefossé) or Lauroglycol® 90 (Gattefossé),
caprylocaproyl macrogol 8 glyceride (saturated ethyldiglycosylated glyceride) commercialized for example by the trademarks Labrasol®, Gelucire 44-14 (Gattefossé),
diethylenglycol monoethyl ether, known for example by the commercial name Transcutol® (Gattefossé).

The compositons according to the present invention in the form of microemulsions are stable in a wide range of temperature, generally from 0˚C to 80˚C, preferably from 4˚C to 45˚C.
The microemulsions of the present invention are prepared with a a process comprising the following steps:

(IP) solubilization of the diazabicyclic compound of formula A' in oil,
(IIP) addition of component S) to the solution in oil,
(IIIP) optional addition of component AD) to the oily phase,
(IVP) addition of water or saline solution to the oily phase, obtaining a limpid solution.

In step (IVP) the water or the saline solution are added to the oily phase preferably obtained under stirring.
The steps of the processes can be carried out at temperatures in the range 0˚C - 80˚C.
It is possible to obtain a microemulsion in the form of a limpid solution also by varying the order of performance of the above mentioned steps, or, for example, by proceeding as follows:

(I') solubilization of the diazabicyclic compound of formula A' in oil,
(II') addition of component S) to the aqueous phase (water or saline),
(III') optional addition of component AD) to the aqueous phase,
(IV') mixing the oily phase of step (I') with the aqueous phase of step (II') or optionally with (III').

In step (IV') mixing is preferably carried out under stirring.
The temperature range at which one operates is the same as indicated above.
It has been surprisingly and unexpectedly found by the Applicant that the microemulsions of the invention have a content of surfactants, with respect to the active principle, lower than that of the microemulsions not containing component O). This is extremely advantageous since it reduces the undesired potential effects of the surfactants, especially if these are present in significant amounts with respect to PA).
The Applicant has surprisingly and unexpectedly found that it is possible to prepare pharmaceutical formulations having a better shelf life containing the diazabicyclic compounds of formula A'. It has been surprisingly and unexpectedly found that it is possible to prepare microemulsions with the compounds of formula A'. The pharmaceutical formulations of the invention show the following combination of properties:

- possibility of being dilutable with water or with aqueous solutions,
- solubilization in the liquid pharmaceutical composition of the active principles at concentrations at least equal to those effective for a therapeutic treatment in human beings and in mammals,
- improved shelf life,
- re-establishment of the homogeneity and shelf life of the starting composition also when separated phases are formed in the formulation,
- reduced ratio by weight surfactant/active principle to avoid the drawbacks of the prior art.

The Applicant has furthermore surprisingly and unexpectedly found that it is possible to obtain a concentrated microemulsion of the active principle, for example having concentration higher than 50%, which is stable in the time. Further it is possible to dilute the concentrated microemulsion with water and/or oil, obtaining diluted compositions. This is a remarkable advantage since it is possible, starting from a concentrated microemulsion, to obtain, for example by diluting with water or isotonic saline solution, a ready-to-use pharmaceutical composition containing an effective amount of the active principle.
The compounds A' can be present in the form of isomers cis and trans, and/or optical isomers when one or more chiral centres are present in the compounds.
The present invention refers also to a specific class of diazabicyclic nonane and decane derivatives with homopiperazine main ring which have a high affinity and selectivity for one or more opioidergic receptors μ, δ, k or their receptorial subclasses, so that one can act either substantially only on one receptor of this receptorial class or simultaneously on more opioid receptors.
It is therefore a further object of the present invention diazabicyclic nonane and decane derivatives with homopiperazine main ring, having affinity for the opioidergic receptors μ and/or δ and/or k and/or for their receptorial subclasses,

having activity on the central nervous system and/or peripheral system, of formula (I), comprising the isomeric forms and the mixtures thereof, wherein the ring atoms can be optionally in different isotopic forms:

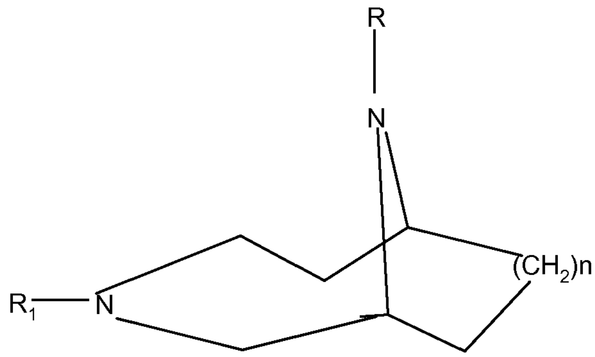

(I)

wherein:

- n is an integer equal to 1 or 2,
- one of the substituents R and $R_1$ of the nitrogen atoms of the diazabicyclic ring, is a -C(O)-$R_B$ group, wherein $R_B$ is a $C_1$-$C_{10}$ alkyl group, linear or branched when possible,
- the other substituent is selected from the following groups from (II) to (X):

structure (II)

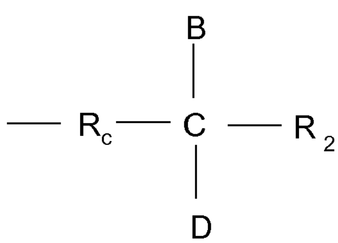

(II)

wherein: linear or branched

- $R_c$ is a bivalent saturated $C_3$-$C_{10}$ aliphatic chain, linear or branched when possible,
- B is a group selected from hydrogen, isothiocyanate, CN, OR', C(O)OR', C(O)R', C(O)NR'R", NR'R", R' and R", equal to or different from each other, being selected from hydrogen, linear or branched when possible, $C_1$-$C_7$ alkyl, $C_1$-$C_7$ alkoxy, $C_1$-$C_7$ alkylthio, $C_1$-$C_7$ haloalkyl, $C_1$-$C_7$ haloalkoxy, $C_3$-$C_{15}$ cycloalkyl, aryl or heteroaryl,
- D and $R_2$, equal to or different from each other, are substituents selected from:
  - hydrogen, with the proviso that in formula (II) at least one substituent among B, D and $R_2$ is different from hydrogen,
  - $C_1$-$C_{10}$ alkyl, linear or branched when possible,
  - aryl or heteroaryl,
  - $C_3$-$C_{15}$ cycloalkyyl,

formula (III):

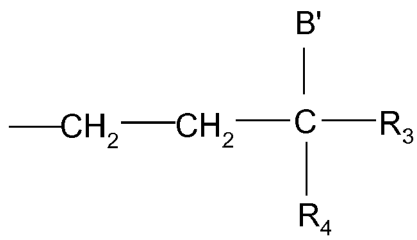

(III)

wherein:

- B' is equal to B as defined above,
- $R_3$ is hydrogen, or, respectively, an alkyl, aryl, heteroaryl or cycloalkyl substituent as defined for $R_2$,
- $R_4$ has the following meanings:

  - $C_1$-$C_{10}$ alkyl, linear or branched when possible,
  - $C_3$-$C_{15}$ cycloalkyl, optionally containing one or more heteroatoms, preferably selected from O, S, N,
  - when B' and $R_3$ are not both hydrogen, $R_4$ has the further the meaning of aryl or heteroaryl,

formula (IV):

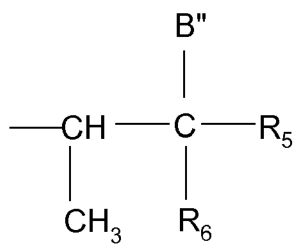

(IV)

wherein:

- B" has the same meaning of B' as defined above,
- $R_5$ has the same meaning of $R_3$ as defined above,
- $R_6$ has the same meaning of $R_4$ as defined above, or is an aryl or heteroaryl,

formula (V):

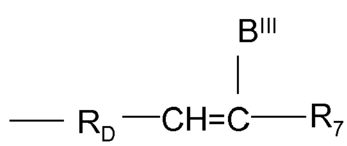

(V)

wherein:

- $R_D$ is a bivalent saturated or unsaturated $C_2$-$C_8$ aliphatic chain, linear or branched when possible,
- $B^{III}$ and $R_7$, equal to or different from each other, have the same meaning of $R_2$ as defined above, with the

proviso that $B^{III}$ and $R_7$ are not both hydrogen,

formula (VI):

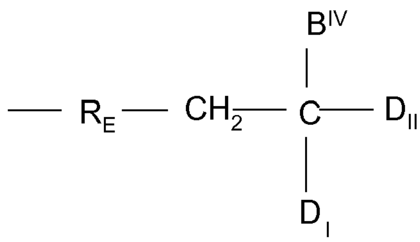

(VI)

wherein:

- $R_E$ is an unsaturated bivalent $C_2$-$C_8$ aliphatic chain, linear or branched when possible,
- $B^{IV}$ has the same meaning of B as defined above,
- $D_I$ and $D_{II}$ have the same meaning of $R_2$ as defined above, with the proviso that in formula (VI) at least one substituent among $B^{IV}$, $D_{II}$ and $D_I$ is different from hydrogen,

formula (VII):

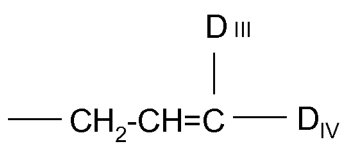

(VII)

wherein:

- $D_{III}$ and $D_{IV}$, equal to or different from each other, have the same meaning of $R_2$ as defined above, but excluding hydrogen,

formula (VIII):

$-CH_2-CH=CH-D_V$ (VIII)

wherein $D_V$ has the meanings of $R_4$ as defined above but excluding aryl or heteroaryl,
formula (IX):

$-R_F-C\equiv C-R_8$ (IX)

wherein $R_F$ is a bivalent, saturated or unsaturated bivalent, $C_2$-$C_8$ aliphatic chain, linear or branched when possible and $R_8$ has the same meaning as $R_2$ as defined above, but excluding the meaning of $R_8$ equal to hydrogen,
formula (X):

$-CH_2-C\equiv C-R_9$ (X)

wherein $R_9$ has the meanings of $R_4$ as defined above but excluding aryl or heteroaryl.

When the substituent B in formula (II) is a group selected from OR', C(O)OR', C(O)R', C(O)NR'R", NR'R" and R' and/or R" have the meaning of $C_3$-$C_{15}$ cycloalkyl, said cycloalkyl can optionally contain one or more heteroatoms, preferably selected from O, S, N.

When the substituent B in formula (II) is a group selected from OR', C(O)OR', C(O)R', C(O)NR'R", NR'R" and R' and/or R" have the meaning of $C_3$-$C_{15}$ cycloalkyl, aryl and heteroaryl, said $C_3$-$C_{15}$ cycloalkyl, aryl and heteroaryl can optionally be substituted with one or more groups selected from hydroxy, halogen, $C_1$-$C_{10}$ alkyl linear or branched when possible.

When D and/or $R_2$ in formula (II) have the meaning of $C_1$-$C_{10}$ alkyl, linear or branched when possible, the alkyl can optionally be substituted with one or more groups, equal to or different from each other, selected from hydroxy, halogen, CN, $C_3$-$C_{15}$ cycloalkyl, aryl, heteroaryl. Said cycloalkyl can optionally contain one or more heteroatoms, preferably selected from O, S, N. Said $C_3$-$C_{15}$ cycloalkyl, aryl and heteroaryl can optionally be substituted with one or more groups selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

When D and/or $R_2$ in formula (II) and/or $R_6$ in formula (IV) have the meaning of aryl or heteroaryl, these structures can optionally be substituted with one or more of the following groups, equal to or different from each other, selected from: $C_1$-$C_{10}$ alkyl, linear or branched when possible, optionally substituted with one or more groups, equal to or different from each other, selected from hydroxy, halogen, CN,
$C_3$-$C_{15}$ cycloalkyl, optionally containing one or more heteroatoms, preferably selected from O, S, N. Said cycloalkyl can optionally be substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy,
arylalkyl or aryl or heteroaryl or heteroarylalkyl, these groups are optionally substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

When D and/or $R_2$ in formula (II) have the meaning of $C_3$-$C_{15}$ cycloalkyl, said cycloalkyl optionally contains one or more heteroatoms, preferably selected from O, S, N. Furthermore cycloalkyl can optionally be substituted with one or more of the following groups, equal to or different from each other, selected from:

$C_1$-$C_{10}$ alkyl, linear or branched when possible, optionally substituted with one or more groups, equal to or different from each other, selected from hydroxy, halogen, CN,
$C_3$-$C_{15}$ cycloalkyl, optionally containing one or more heteroatoms, preferably selected from O, S, N. Said cycloalkyl can optionally be substituted with one or more groups, equal to or different from each other, selected from hydroxy, halogen, linear and branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy,
arylalkyl or aryl or heteroaryl or heteroarylalkyl, said groups optionally substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

In formula (III) when $R_4$ has the meaning of $C_1$-$C_{10}$ alkyl, linear or branched when possibile, said $C_1$-$C_{10}$ alkyl can optionally be substituted with one or more groups equal to or different from each other selected from hydroxy, halogen, CN, aryl, heteroaryl or $C_3$-$C_{15}$ cycloalkyl, optionally containing one or more heteroatoms, preferably selected from O, S, N. Said $C_3$-$C_{15}$ cycloalkyl, aryl or heteroaryl can optionally be substituted with one or more groups equal to or different from each other selected from hydroxy, halogen, $C_1$-$C_{10}$ alkyl, linear or branched when possible, arylalkyl or aryl or heteroaryl or heteroarylalkyl. Said arylalkyl or aryl or heteroaryl or heteroarylalkyl can optionally be substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

In formula (III) when $R_4$ has the meaning of $C_3$-$C_{15}$ cycloalkyl, optionally containing one or more heteroatoms, preferably selected from O, S, N, said cycloalkyl can optionally be substituted with one or more of the following groups, equal to or different from each other:

$C_1$-$C_{10}$ alkyl, linear or branched when possible, optionally substituted with one or more groups, equal to or different from each other, selected from the following: hydroxy, halogen, CN,
$C_3$-$C_{15}$ cycloalkyl, optionally containing one or more heteroatoms, equal to or different from each other, preferably selected from O, S, N, said cycloalkyl optionally be substituted with one or more groups, equal to or different from each other: hydroxy, halogen, $C_1$-$C_{10}$ alkyl, linear or branched when possible, arylalkyl or aryl or heteroaryl or heteroarylalkyl. Said arylalkyl or aryl or heteroaryl or heteroarylalkyl can optionally be substituted with one or more groups, equal to or different from each other, selected from: hydroxy, halogen, linear and branched when possible

$C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy,
arylalkyl or aryl or heteroaryl or heteroarylalkyl, optionally substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

In formula (III) when B' and $R_3$ are not both hydrogen and $R_4$ can also have the meaning of aryl or heteroaryl, these structures can optionally be substituted with one or more of the following groups, equal to or different from each other:

$C_1$-$C_{10}$ alkyl, linear or branched when possible, optionally substituted with one or more groups, equal to or different from each other, selected from the following: hydroxy, halogen, CN,
$C_3$-$C_{15}$ cycloalkyl, optionally containing one or more heteroatoms, equal to or different from each other, preferably selected from O, S, N. Said cycloalkyl can optionally be substituted with one or more of the following groups, equal to or different from each other: hydroxy, halogen, $C_1$-$C_{10}$ alkyl, linear or branched when possible, arylalkyl or aryl or heteroaryl or heteroarylalkyl. Said arylalkyl or aryl or heteroaryl or heteroarylalkyl can optionally be substituted with one or more groups, equal to or different from each other, selected from hydroxy, halogen, linear and branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy,
arylalkyl or aryl or heteroaryl or heteroarylalkyl, optionally substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

Where not otherwise specified, the following meanings are meant in the present invention.
By alkyl or alkyl chain it is meant a saturated $C_1$-$C_{40}$, preferably $C_1$-$C_{10}$, aliphatic chain, linear or branched when possible, the aliphatic chain having a free valence.
By bivalent saturated aliphatic chain it is meant a linear or branched when possible hydrocarbon chain, formed of carbon atoms saturated with hydrogen atoms, and linked to each other by single bonds, having at each end of the backbone a free valence.
By bivalent unsaturated aliphatic chain it is meant a hydrocarbon chain formed of carbon atoms linked to each other by single bonds and by at least a double or triple bond, the chain having at each end a free valence, the other valences of the carbon atoms are saturated by hydrogen atoms.
By cycloalkyl it is meant a cycloalkyl with a ring, for example from 3 to 8 carbon atoms, preferably from 5 to 6 carbon atoms, or a structure having more condensed cycloalkyl rings, preferably with 8-19 carbon atoms.
By saturated heterocycle it is meant a cycloalkyl as defined above wherein at least one carbon atom of a cycloalkyl ring is substituted by an heteroatom preferably selected from S, O, N.
By unsaturated heterocycle it is meant a saturated heterocycle as defined above but with one or more double bonds in one or more cycloalkyl rings, with the proviso that the ring is not aromatic.
By halogen, an atom selected from fluorine, chlorine, bromine, iodine is meant.
By haloalkyl or haloalkyl chain it is meant an alkyl as defined above wherein one or more hydrogen atoms are substituted with halogen atoms, for example trifluoromethyl, 1-bromo-n-butyl, pentachloroethyl.
By aryl it is meant a $C_6$ aromatic monocyclic radical or a $C_7$-$C_{19}$ polycyclic radical wherein at least one ring is aromatic, said radicals containing carbon and hydrogen atoms.
By alkylaryl it is meant a $C_1$-$C_7$ alkyl group, linear or branched when possible, wherein one hydrogen atom is substituted with an aryl group as defined above, wherein the free valence is on the aryl.
By arylalkyl it is meant a $C_1$-$C_{10}$ alkyl linear or branched when possible, linked to an aryl as defined above, wherein the free valence is in the alkyl moiety, for example benzyl can be mentioned.
By heteroaryl it is meant an aryl as defined above, but even with 5 atom rings, wherein at least one atom of the ring is an heteroatom preferably selected from S, O, N.
By alkylheteroaryl it is meant a $C_1$-$C_7$ alkyl group, linear or branched when pssible, wherein one hydrogen atom is substituted with an heteroaryl group as defined above.
By heteroarylalkyl it is meant an arylalkyl as defined above wherein at least one atom of the ring is an heteroatom preferably selected from S, O, N, but even with 5 atom rings.
By compound having affinity towards the receptors it is meant a compound having in vivo and/or in vitro and/or in ex-vivo agonist activity, or antagonist, or partial agonist, or partial antagonist, or inverse agonist, or inverse antagonist, or inverse partial agonist, or inverse partial antagonist activity towards the receptors. The meaning of said terms is known to the skilled in the field.
By opioid receptors and opioidergic receptors are meant the receptors $\mu$ and/or $\delta$ and/or k and/or their receptorial subclasses.
By receptorial subclasses of the opioidergic receptors $\mu$, $\delta$ and k, the receptors $\mu 1$, $\mu 2$, $\delta 1$, $\delta 2$, k1, k2 and k3

are meant.

The preferred compounds of formula (I) are those wherein one of the substituents R and $R_1$ of the nitrogen atoms of the diazabicyclc ring is a group -C(O)-$R_B$, the other substituent remained between R and $R_1$ is selected from the structures of formula (II) to (X) wherein:

in formula (II) $R_c$ is a bivalent saturated $C_3$-$C_7$ aliphatic chain, linear or branched when possible, B is hydrogen,
in formula (III) B' is hydrogen, $R_3$ is a substituent selected from alkyl, aryl, heteroaryl or cycloalkyl as defined in $R_2$, wherein $R_4$ is as above defined, and further comprising the meaning of aryl or heteroaryl as defined above,
in formula (IV) B" is hydrogen,
in formula (V) $R_D$ is a saturated or unsaturated bivalent $C_2$-$C_5$ aliphatic chain, linear or branched when possible, $B^{III}$ and $R_7$, equal to or different from each other, have the same meaning of $R_2$ with the proviso that $B^{III}$ and $R_7$ are not both hydrogen,
in formula (VI) $R_E$ is a bivalent unsaturated $C_2$-$C_5$ aliphatic chain, linear or branched when possible, $B^{IV}$ is hydrogen, $D_{II}$ and $D_I$ have the same meaning of $R_2$, with the proviso that in formula (VI) at least one substituent between $D_{II}$ and $D_I$ is different from hydrogen,
formulae (VII) to (X) are as defined above.

The most preferred compounds of formula (I) are those wherein:

one of the substituents R and $R_1$ is a group -C(O)-$R_B$, wherein $R_B$ is a $C_1$-$C_4$ alkyl group linear or branched when possible, the other substituent remained between R and $R_1$ is selected from the structures of formulae (II) to (X) wherein:
in formula (II) $R_c$ is a bivalent saturated $C_3$-$C_7$ aliphatic chain, linear or branched when possible, B is hydrogen,
in formula (III) B' is hydrogen, $R_3$ is a substituent selected from alkyl, aryl, heteroaryl or cycloalkyl as defined for $R_2$, wherein $R_4$ comprises also the meaning of aryl or heteroaryl, $R_3$ being different from hydrogen and B being equal to hydrogen,
in formula (IV) B" is hydrogen,
in formula (V) $R_D$ is a bivalent saturated or unsaturated $C_2$-$C_5$ aliphatic chain, linear or branched when possible, $B^{III}$ and $R_7$, equal to or different from each other, have the same meaning of $R_2$, with the proviso that $B^{III}$ and $R_7$ are not both hydrogen,
in formula (VI) $R_E$ is an unsaturated bivalent $C_2$-$C_5$ aliphatic chain, linear or branched when possible, $B^{IV}$ is hydrogen, $D_{II}$ and $D_I$ have the same meaning of $R_2$, with the proviso that in formula (VI) at least one substituent between $D_{II}$ and $D_I$ is different from hydrogen,
formulae (VII) to (X) are as defined above.

The still more preferred compounds of formula (I) are those wherein:

one of the substituents R and $R_1$ is a group -C(O)-$R_B$, wherein $R_B$ is methyl or ethyl, the other substituent remained between R and $R_1$ is selected from the structures of formula (II) to (X) wherein:
in formula (II) $R_c$ is a bivalent saturated $C_3$ alkyl chain, linear or branched when possible, B is hydrogen,
in formula (III) B' is hydrogen, $R_3$ is a substituent selected from alkyl, aryl, heteroaryl or cycloalkyl as defined in $R_2$, wherein $R_4$ comprises also the meaning of aryl or heteroaryl, $R_3$ being different from hydrogen and B being equal to hydrogen,
in formula (IV) B" is hydrogen,
in formula (V) $R_D$ is a bivalent saturated or unsaturated $C_2$-$C_5$ aliphatic chain, linear or branched when possible, $B^{III}$ and $R_7$, equal to or different from each other, have the same meaning of $R_2$, with the proviso that $B^{III}$ and $R_7$ are not both hydrogen,
in formula (VI) $R_E$ is an unsaturated bivalent $C_3$ aliphatic chain, linear or branched when possible, $B^{IV}$ is hydrogen, $D_{II}$ and $D_I$ have the same meaning of $R_2$, with the proviso that in formula (VI) at least one substituent between $D_{II}$ and $D_I$ is different from hydrogen,
formulae (VII) to (X) are as defined above.

In particular the most preferred compounds are those of formula (I) wherein: one of the substituents R and $R_1$ is a group -C(O)-$R_B$, wherein $R_B$ is methyl or ethyl, the other substituent remained between R and $R_1$ is selected from the structures of the following formulae:

formula (III) wherein B' is hydrogen, $R_3$ is a substituent selected from alkyl, aryl, heteroaryl or cycloalkyl as defined in $R_2$, wherein $R_4$ comprises also the meaning of aryl or heteroaryl,

formula (VII) wherein $D_{III}$ and $D_{IV}$ equal to or different from each other, have the meaning of $R_2$ but excluding hydrogen, formula (VIII) wherein $D_V$ has the meaning of $R_4$ but excluding aryl or heteroaryl, formula (X) wherein $R_9$ has the meanings of $R_4$ but excluding aryl or heteroaryl.

The specific compounds of formula (I) are the following:

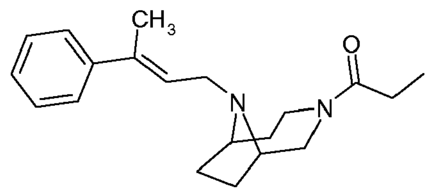

(XX)

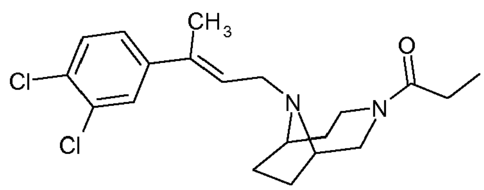

(XXI)

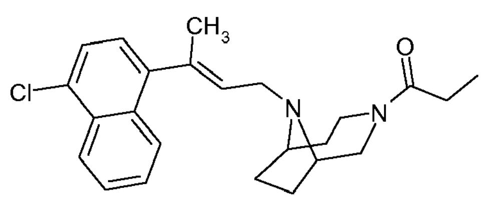

(XXII)

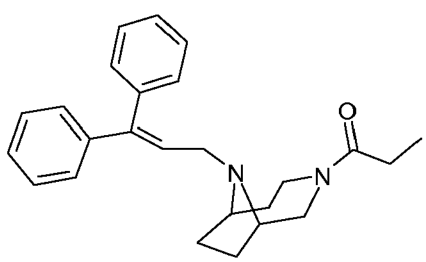

(XXIII)

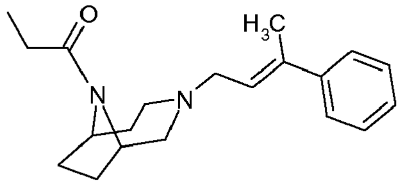

(XXIV)

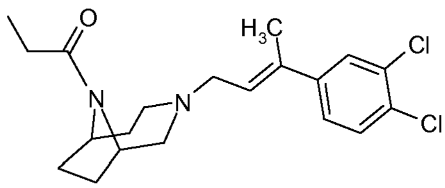

(XXV)

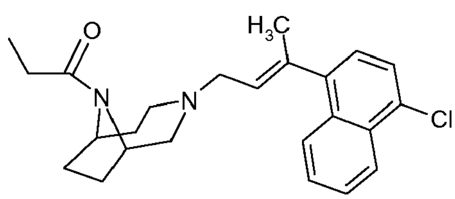

(XXVI)

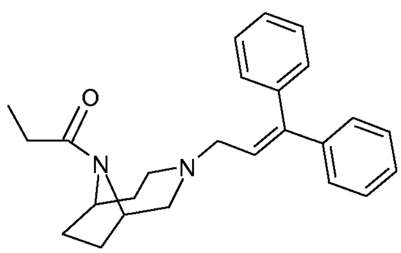

(XXVII)

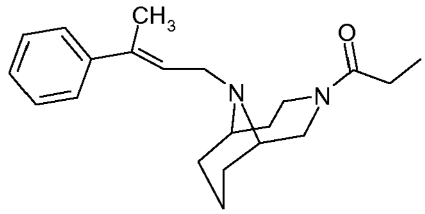

(XXVIII)

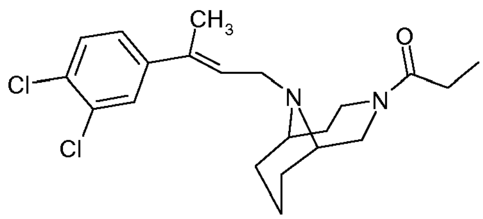

(XXIX)

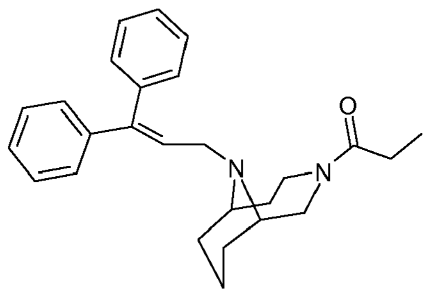

(XXX)

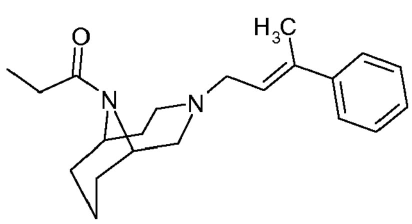

(XXXI)

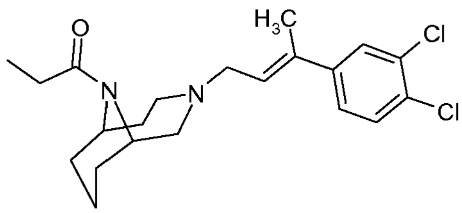

(XXXII)

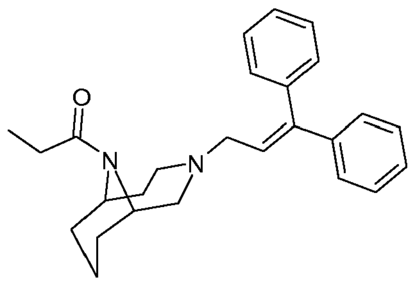

(XXXIII)

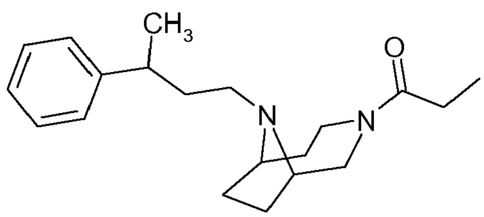

(XXXIV)

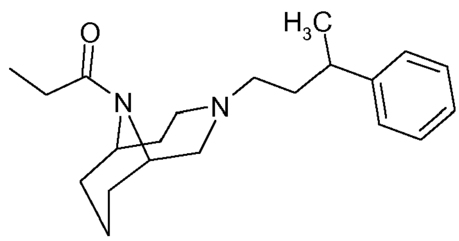

(XXXV)

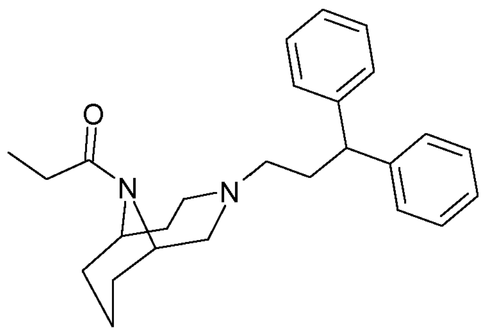

(XXXVI)

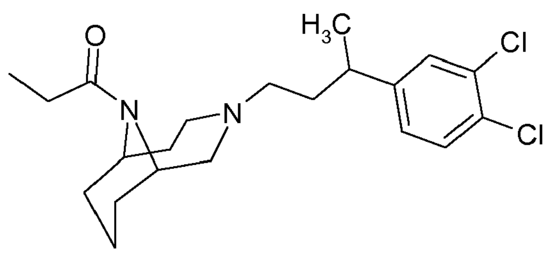

(XXXVII)

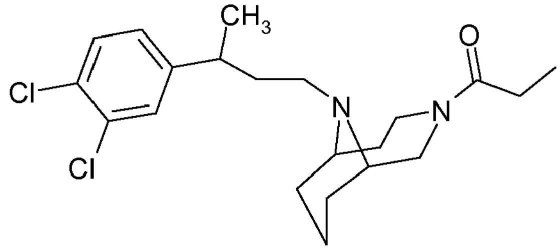

(XXXVIII)

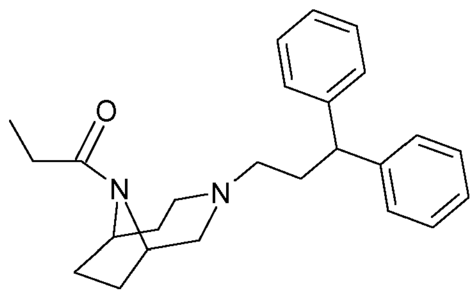

(XXXIX)

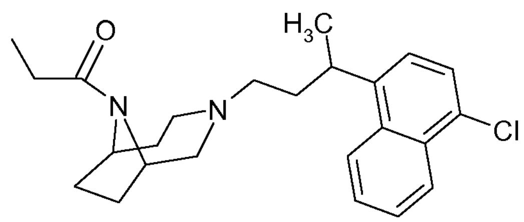

(XXXX)

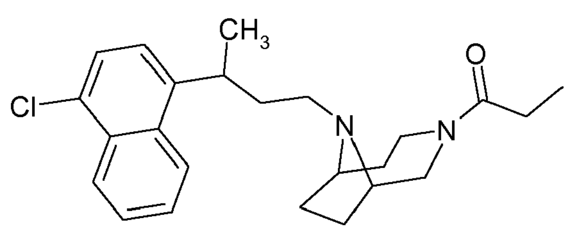

(XXXXI)

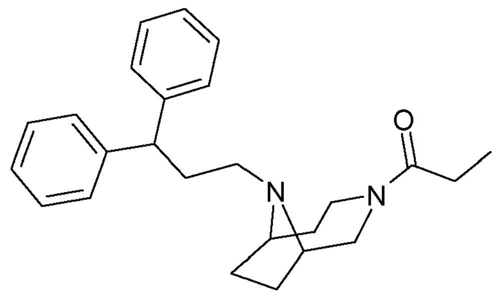

(XXXXII)

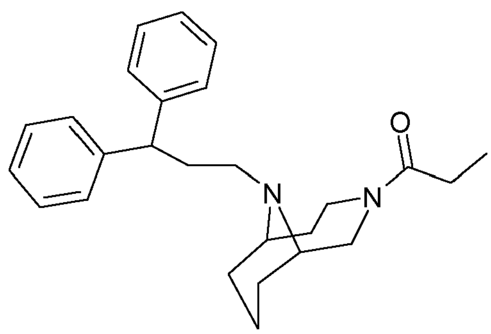

(XXXXIII)

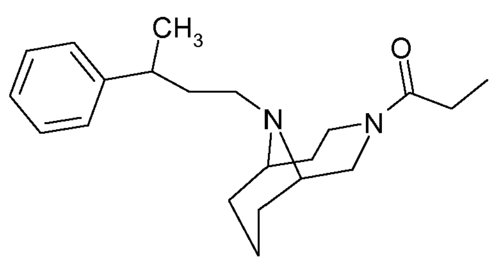

(XXXXIV)

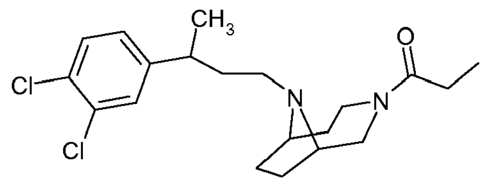

(XXXXV)

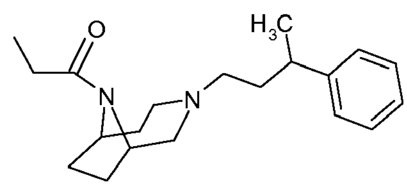

(XXXXVI)

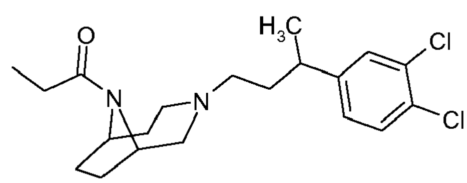

(XXXXVII)

As said, the hydrates, solvates and the pharmaceutically acceptable salts of the compounds of formula (I), including the various geometrical isomers and/or stereoisomers (for example cis and trans isomers, optical isomers when one or more chiral centres are present in the compounds) and corresponding mixtures of the compounds of formula (I), are a further object of the present invention. The meaning of the hydrate and solvate terms is well known to the skilled in the field. In particular by hydrate it is meant the compound containing one or more molecules of hydration water, generally from 1 to 10 water molecules. By solvate it is meant that the compound contains one or more molecules of solvent different from water, preferably from 1 to 10 solvent molecules.

By pharmaceutically acceptable salts are meant all the salts obtained by treating the compounds of formula (I) with organic or inorganic acids acceptable from the pharmaceutical point of view. For example hydrochlorides, sulphates, fumarates, oxalates, citrates, hydrogensulphates, succinates, paratoluensulphonates can be mentioned. See the volume: "Remington, The Science and Practice of Pharmacy", vol. II, 1995, page 1457.

The metabolites derived from the administration in an individual and in an animal of the compounds of formula (I) are a further object of the present invention.

It has been surprisingly and unexpectedly found by the Applicant that the compounds of formula (I) of the invention have both in vitro and/or in vivo one or more of the following activities towards the opioidergic receptors $\mu$ and/or $\delta$ and/or k (and all the receptorial subclasses thereof, such as the receptors $\mu_1$, $\mu_2$ and $\mu_3$): agonist, or antagonist,

or partial agonist, or partial antagonist, or inverse agonist, or inverse antagonist, or inverse partial agonist, or inverse partial antagonist. In particular, when the compounds of formula (I) show agonist activity towards one or more receptors of the opioidergic system, they can be used in the treatment of pain.

It is a further object of the present invention a process for preparing the compounds of formula (I).

The process, see scheme 1, comprises the following steps:

a) reaction of an aldehyde of formula 2 with an amine of formula 1 wherein Rg has the meaning of methyl or benzyl, and with the 1,3-acetonedicarboxylic acid of formula 3, and obtainment of a bicyclic ketone of formula 4,
b) treatment of the ketone of formula 4 with inorganic acids in the presence of an azide, or a derivative thereof, and obtainment of the aminolactam of formula 5,
c) reduction of the aminolactam of formula 5 to obtain the diazabicyclic amine of formula 6,
d) acylation of the diazabicyclic amine of formula 6 with an alkyl anhydride, or an acyl chloride, wherein in the anhydride or in the acyl chloride the alkyl chain is $R_B$ as defined above in formula (I), obtaining the amide of formula 7, wherein the acyl substituent -C(O)-$R_B$ has the meaning of $R_1$ in formula (I),
e) hydrogenation of the amide of formula 7 by obtaining an amidic compound of formula 8,
f) substitution of the hydrogen present on the amine nitrogen of the compound of formula 8 with the substituent R as defined in formula (I), and obtainment of the compound of formula 9.

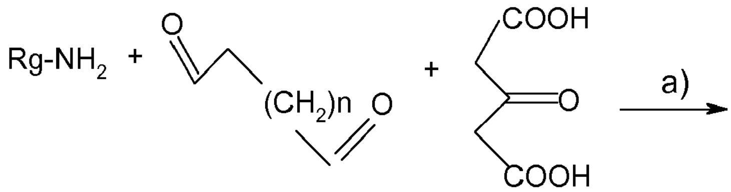


1 2 3

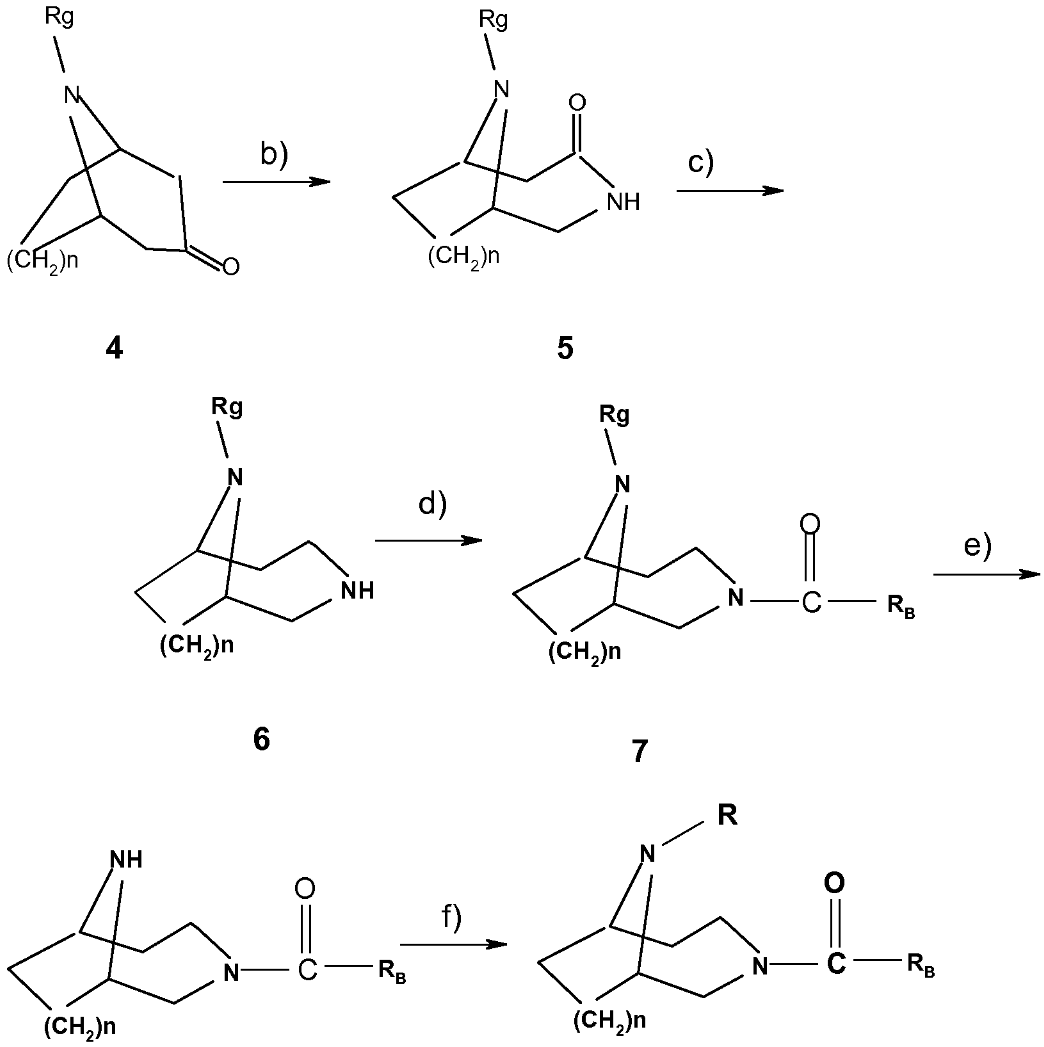


4 5

6 7

8 9

Scheme 1

The reaction of step a) can be carried out in an aqueous solution in the presence of sodium acetate, by operating at a temperature about 0°C. The obtained mixture is left under stirring at room temperature for at least one hour and then heated to 50°C for 3-7 hours. The pH is brought to values comprised between 4 and 5. Compound 4 is then extracted from the aqueous phase with an organic solvent, for example dichloromethane, and then recovered by solvent evaporation.

Step a) can be avoided when the ketones of formula 4 are commercially available.

In step b) the used inorganic acids, preferably concentrated, are for example sulphuric acid. Besides, the azide can be used under the form of a derivative thereof, for example $NaN_3$. Generally one operates at temperatures lower than 40°C, preferably lower than 35°C.

In step c) the reduction of the aminolactam 5 is preferably carried out with metal hydrides in an inert organic solvent, in an inert atmosphere. As metal hydride, $LiAlH_4$ can for example be used, the inert organic solvent can be for example tetrahydrofuran (THF). Generally one operates at temperatures of about 0°C in an inert atmosphere, for example by using argon.

In step d) the acylation reaction of the diazabicyclic amine is preferably carried out in the presence of an acceptor of protons, such as a tertiary amine.

Step e) of the hydrogenation of the amide of formula 7 can be carried out by using 2,2,2-trichloro ethylchloroformate and metal zinc in acetic acid, or by catalytic hydrogenation with Pd/C.

Step f) can be carried out for example by reaction with a compound of formula R-X wherein X is a leaving group, preferably selected from halogen, mesyl or tosyl. In place of the compound R-X, an aldehyde having formula $R_T$-C(O)H can also be used. The substituent $R_T$-$CH_2$- which is formed in the reaction by using the aldehyde, corresponds to group R of formula (I). The reaction conditions are those known in the prior art. The reactants R-X are generally commercially available or can be prepared according to the processes of the prior art. For example they can be obtained by synthesis schemes as reported hereinunder.

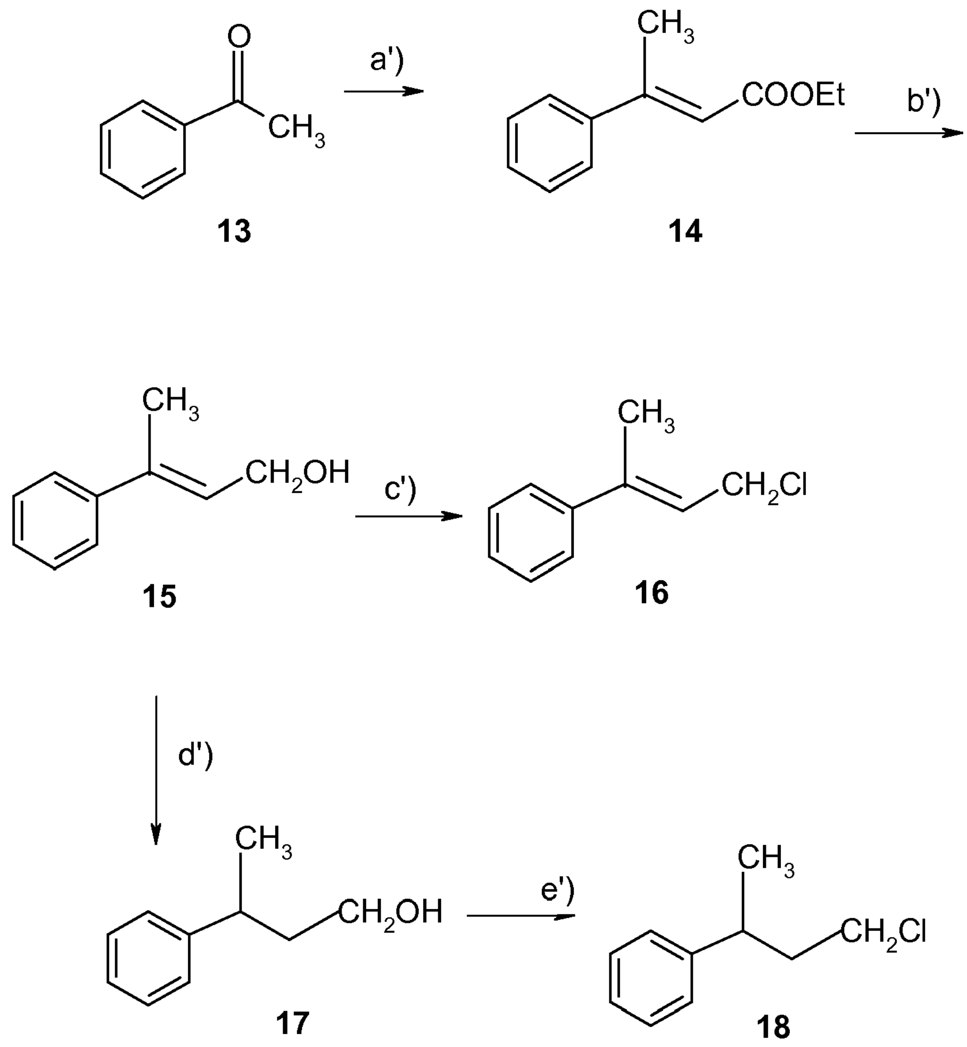


13 14 15 16 17 18

Scheme 1'

According to scheme 1', chlorides of formula 16 and 18 are obtained by the following steps:

a') 1.5 equivalents of triethyl-phosphonium acetate in toluene are reacted at room temperature with 1.5 equivalents of sodium hydride for one hour. One equivalent of the ketone of formula 13 dissolved in toluene is dropwise added to the obtained mixture. The obtained mixture is reacted at room temperature under stirring for two-three days, obtaining the ester of the acrylic acid derivative of formula 14,

b') the ester of the acrylic acid derivative of formula 14 is reduced to the corresponding alcohol of formula 15, preferably by using metal hydrides, for example $LiAlH_4$,

c') the alcohol of formula 15 is halogenated preferably by treatment with concentrated hydrochloric acid, obtaining the corresponding chloride of formula 16,

d') alternatively to step c'), the alcohol of formula 15 can be transformed into the corresponding alcohol of formula 17 having a saturated aliphatic chain, for example by catalytic hydrogenation with Pd/C,

e') the alcohol of formula 17 is treated with concentrated HCl obtaining the chloride of formula 18.

According to an alternative process, compound 8 of Scheme 1, instead of being subjected to step f), is subjected to steps f') and g'), see scheme 2:

f') obtainment of the diazabicyclic compounds of formula 8a, wherein the acyl substituent -C(O)-$R_B$ of the amidic nitrogen has the meaning of R in formula (I), by subjecting the amidic compounds of formula 8 to thermal transposition,
g') obtainment of the compound of formula 9a by substitution of the hydrogen present on the aminic nitrogen of the compound of formula 8a with the substituent $R_1$ as defined in formula (I).

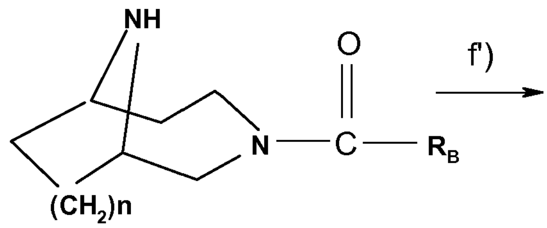


**8**

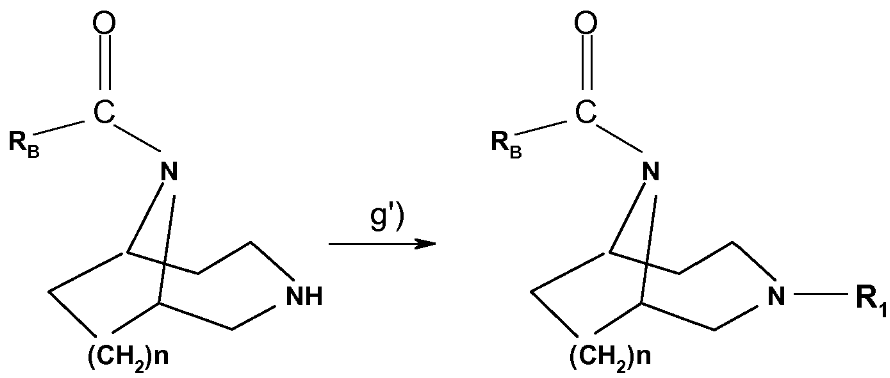


**8a** **9a**

Scheme 2

In step f') the thermal transposition can be carried out at temperatures comprised between 100˚C and 200˚C, preferably from 120˚C to 150˚C. The reaction time is generally comprised between 60 and 180 minutes.

Step g') is preferably carried out by using a compound of formula $R_1$-X wherein X is a leaving group, preferably selected from halogen, mesyl or tosyl. In place of the compound $R_1$-X an aldeyde of formula $R_{w}$-C(O)H can be used. In this case the substituent $R_w$-$CH_2$- that is formed corresponds to the group $R_1$ of formula (I). The reaction conditions of this step are those known in the prior art. The compounds $R_1$-X are generally commercially available or can be prepared according to known synthesis schemes of the prior art.

According to a further alternative of the described process of Scheme 1, instead of steps d)-f), steps from g) to k) can be used according to scheme 3 reported herein below:

g) protection of the nitrogen atom at position 3 of the diazabicyclic amine of formula 6 obtaining the compound of formula 10,
h) hydrogenation of the compound of formula 10, obtaining the diazabicyclic aminocarbamate compound of formula 11,
i) acylation of the diazabicyclic amine of formula 11 with an alkyl anhydride, or an acyl chloride, wherein in both compounds the alkyl group is $R_B$ as defined in formula (I), and obtainment of the amide of formula 12, wherein the acyl substituent -C(O)-$R_B$ of the amidic nitrogen has the meaning of R in formula (I),
j) deprotection of the amine group, obtaining the compound of formula 8a,
k) obtainment of compound 9a by performing the reaction described in step g').

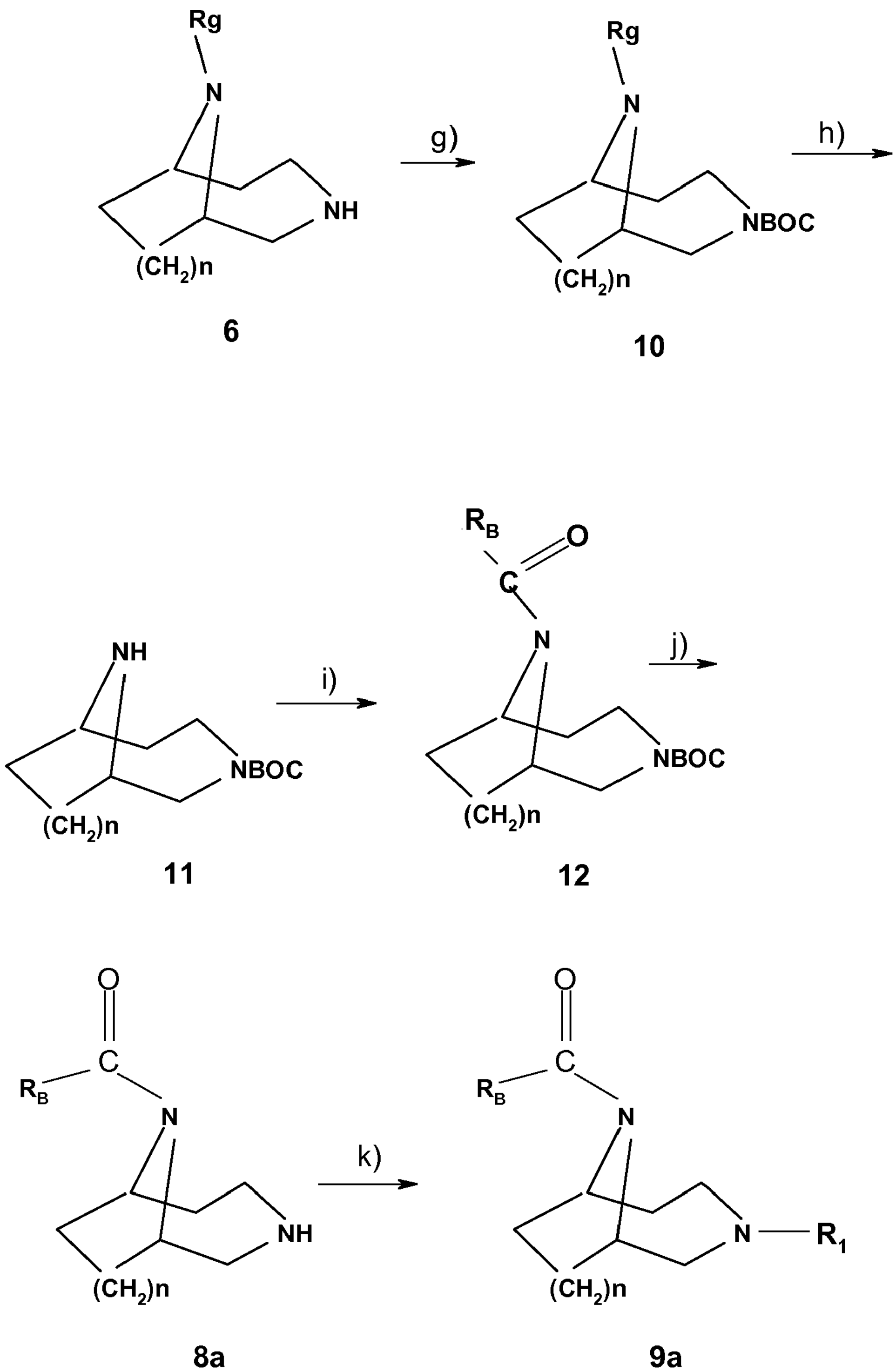


6 10 11 12 8a 9a

Scheme 3

In step g) protection of the nitrogen atom is preferably carried out with di-tert-butyldicarbonate ($(BOC)_2O$)) in an inert solvent.

In step h) the hydrogenation reaction is carried out with Pd/C catalyst.

In step j) deprotection is carried out by treatment with trifluoroacetic acid in inert solvent. This is particularly used when the amine has been protected with BOC.

The other compounds of formula A' different from the compounds of formula (I) are prepared as follows.

When t=1, r=1, the diazabicycloheptane compounds are defined, which can be prepared as described in patent application WO 2005/108,402.

When t=1, r=2, the diazabicyclooctane compounds are defined, which can be prepared as described in USP 5,672,601.

When t=1, r=3, the diazabicyclononane compounds are defined, which can be prepared as described in patent application US 2003/196,217.

When t=2, r=2 or r=3, the diazabicyclononane and diazabicyclodecane compounds are respectively defined, which can be prepared as described in patent application WO 2004/11,468.

It is a further object of the present invention the microemulsions of the invention for preparing pharmaceutical compositions.

A further object is the use of the microemulsions of the invention for preparing pharmaceutical compositions. The pharmaceutical compositions can include the microemulsions as such, wherein the amount of active principle is that requested for the specific pharmaceutical application. If necessary the starting microemulsion can be diluted to obtain the desired concentration.

The pharmaceutical compositions of the invention can be only partly constituted of the microemulsions of the present invention. Preferably the microemulsions of the present invention are present in amounts (% by weight) from 0.1 % to 95%, more preferably 5-85%, still more preferably 10-60%, depending on the desidered amount of active principle in the final formulation.

In the pharmaceutical compositions component PA) can be present as such or in the form of salt or solvate, or also as isomer.

The additives contained in the pharmaceutical compositions comprising the microemulsions of the invention are excipients, carriers, dyestuffs, preservatives, aromas, etc., the use of which in pharmaceutical field is known. The used amounts of these various additives and excipients are those known for the specific applications.

The administration of the pharmaceutical compositions can be made by oral, subcutaneous, sublingual, intramuscular, intravenous, topic, transdermal, rectal, ophthalmic, intranasal, vaginal, intraperitoneal route.

When the pharmaceutical compositions are only partly constituted by the microemulsions of the present invention, the compositions comprise for example dispersions, solutions, emulsions, powders, capsules, aerosol, suppositories, tablets, syrups, elixirs, creams, gels, ointments, plasters, etc. See for example those described in patent application WO 2004/011,468. The pharmaceutical compositions can be obtained according to known processes of the pharmaceutical art. For example, the pharmaceutical compositions can be obtained according to processes mentioned in USP 6,028,084.

The compounds of formula A', comprising the various isomers and the corresponding hydrates or solvates and pharmaceutically acceptable salts and the pharmaceutical compositions thereof, have an high affinity in vitro for the opioidergic receptors $\mu$ and/or $\delta$ and/or k, and/or all their receptorial subclasses. See the examples. More specifically the compounds of formula A' have a *K*i value for the opioidergic receptors $\mu$ and/or $\delta$ and/or k, lower than 0.5 $\mu$M.

It is a further object of the present invention the use of the pharmaceutical compositions of the present invention for the prophylaxis and therapy in mammals and in human beings of diseases and disorders wherein the opioidergic receptors $\mu$ and/or $\delta$ and/or k are involved.

The diseases and disorders which can be treated with the pharmaceutical compositions of the present invention are: pain, post surgery pain, chronic pain, neuropathic pain, treatment of abuse (as for example heroin and cocaine abuse), alcoholism, constipation, diarrhoea and other disorders of the gastrointestinal tract, nausea, vomit, dermatitis, obesity and other disorders connected with appetite, depression, smoke dependence (tabagism), sexual disfunctions, shocks, cerebral trauma, spinal damages and eye pathologies and disorders as for example glaucoma and intraocular hypertension, tumours as for example breast cancer.

The use of the pharmaceutical compositions of the present invention for the treatment of the various pathologies can be made by using the known methods employed for said treatments.

In particular the administration of the compositions of the invention must be carried out so that the amount of active principle is effective for the specific treatment. The dosages, the administration routes and the posologies are set depending on the type of disease, its severity, the physical conditions and characteristics of the patient, such as age, weight, response to the active principle, the pharmacokinetics and toxicology of the active principle for the specific treatment.

The preferred daily dosage is 0.01-1,000 mg of compound of formula A' per Kg of weight of mammals to be treated. In the case of human beings, the preferred daily range is 0.1-800 mg of compound for Kg/weight, still more preferred from 1 to 600 mg.

The present invention relates furthermore to the compounds of formula (I), for preparing drugs for the treatment in mammals and in human beings of diseases and disorders wherein the opioidergic receptors $\mu$ and/or $\delta$ and/or k and/or their receptorial subclasses are involved.

A further object of the present invention is the use of the compounds of formula (I) for the treatment of the

above mentioned diseases and disorders.

The compounds of formula (I), containing radioactive isotopes and the pharmaceutical formulations thereof, can be used for identifying and marking the opioidergic receptors μ and/or δ and/or k and/or all their receptorial subclasses in mammals or in human beings.

Furthermore the compounds of formula (I) containing an hydroxyl group, can be used to obtain ligands. The ligands are detectable by immunochemical methods, and are used in the separation, purification and characterization of the opioidergic receptors μ and/or δ and/or k and/or the receptorial subclasses thereof and in the identification of the corresponding active sites.

The following examples are given for a better understanding of the present invention but are not meant to be limitative of the scope of the invention.

**EXAMPLES**

**EXAMPLE 1**

Synthesis of the compound 9-methyl-3,9-diazabicyclo[4.2.1] nonan-4-one

To a solution (5 g) in chloroform (50 ml) of tropinone (1 a) of formula:

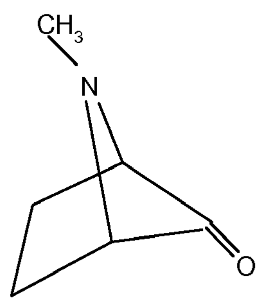

(1a)

cooled at -5˚C, 11.3 ml of conc. $H_2SO_4$ are dropwise added while maintaining the temperature lower than 15˚C. $NaN_3$ (4.67 g) is then slowly added in small amounts lumpwise so to avoid temperatures exceeding 35˚C. Then it is heated at reflux for two hours. The obtained mixture is poured in a vessel containing about 200 ml of ice. Solid $K_2CO_3$ is added up to obtain a strongly alkaline pH. An emulsion is formed. 25 ml of 60% aqueous KOH are added to said emulsion. It is left under stirring for 10 minutes, then the formed inorganic salts are filtered and the reaction mixture is extracted with chloroform. The organic phase is anhydrified on sodium sulphate and the solvent evaporated to obtain 3.64 g of compound 9-methyl-3,9-diazabicyclo-[4.2.1]nonan-4-one (1b) as a white crystalline solid.

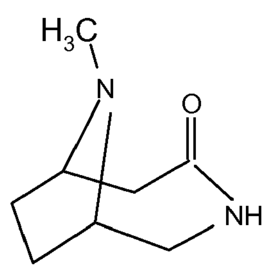

(1b)

Yield: 95%; $R_f$: 0.26 ($CHCl_3$-MeOH 8:2); m.p.: 79-82˚C; $^1$H-NMR ($CDCl_3$): δ (ppm) 1.69-1.85 (m,2H), 2.00-2.20 (m,2H), 2.42 (s,3H), 2.48-2.53 (m,1H), 2.80-2.96 (m,2H), 3.15-3.27 (m,2H), 3.59 (bd,1H,J=14Hz).

**EXAMPLE 2**

Synthesis of 9-methyl-3,9-diazabicyclo-[4.2.1]nonane

To a suspension of $LiAlH_4$ (0.61 g) in anhydrous THF (30 ml), cooled at 0˚C, and kept under an argon inert atmosphere, a solution in THF of 9-methyl-3,9-diazabicyclo[4.2.1]nonan-4-one obtained in example 1 is dropwise added (1.00 g of compound in 10 ml of THF). The obtained mixture is heated at reflux for 48 hours and then cooled at 0˚C. Then water (3 ml) is slowly added to the mixture. After water addition, the mixture is kept under stirring for 10 minutes. A precipitate is formed, that at the end is filtered under vacuum and washed with dichloromethane. The obtained filtrate is evaporated obtaining an oil which is dissolved in dichloromethane. The obtained solution is then anhydrified on sodium sulphate and the solvent evaporated. The residual oil is distilled (45-50˚C/0.1 mmHg) to give 0.63 g of compound 9-methyl-3,9-diazabicyclo[4.2.1]nonane (2a) as a colourless oil.

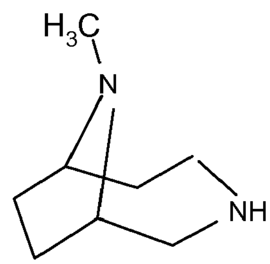

(2a)

Yield: 69%; $R_f$: 0.22 ($CHCl_3$-MeOH 9:1 + drop of $NH_4OH$); b.p.: 45-50˚C/0.1 mmHg;$^1$H-NMR ($CDCl_3$): δ (ppm) 1.40-2.38 (m,4H), 2.05-2.15 (m,2H), 2.44 (s,3H), 2.47 (bs,1H,N*H*), 2.64-3.30 (m,6H);$^{13}$C-NMR ($CDCl_3$): δ (ppm) 28.16; 30.65; 37.30; 43.69; 45.84; 55.57; 63.98; 66.69.

**EXAMPLE 3**

Synthesis of 3-propionyl-9-methyl-3,9-diazabicy-clo[4.2.1] nonane

To a solution in anhydrous dichloromethane (18 ml) of 9-metil-3,9-diazabicyclo [4.2.1] nonane obtained in Example 2 (0.63 g), cooled at 0˚C, a propionic anhydride solution (2.1 ml) in anhydrous dichloromethane (5 ml) is added. The obtained mixture is heated at reflux for one hour, then cooled to room temperature, made alkaline with a NaOH aqueous solution at 40% w. and left under stirring for 16 hours. The so obtained mixture is extracted with dichloromethane; the organic phase is separated, anhydrified on sodium sulphate and evaporated. 0.88 g of compound 3-propionyl-9-methyl-3,9-diazabicyclo[4.2.1]nonane (3a) are obtained under the form of a light yellow oil. The yield is quantitative.

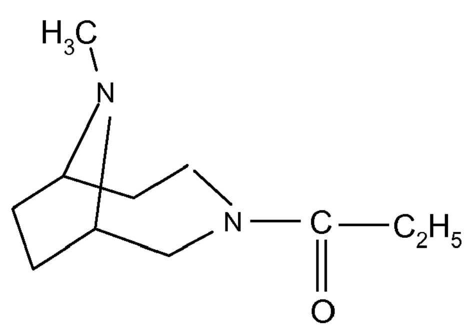

(3a)

$R_f$: 0.44 ($CH_2Cl_2$-MeOH 9:1); $^1$H-NMR ($CDCl_3$): δ (ppm) 1.16 (t,3H,J=7,4Hz), 1.21-2.38 (m,6H), 2.43 (s,3H), 2.80-2.88 (m,1H), 3.19-3.75 (m,5H), 3.85-4.00 (m,1H), 4.35 (bd,1H,J=14Hz).

**EXAMPLE 4**

Synthesis of 3-propionyl-3,9-diazabicyclo-[4.2.1]nonane

To a solution in toluene (25 ml) of 3-propionyl-9-methyl-3,9-diazabicyclo[4.2.1]nonane obtained in Example 3 (0.49 g), kept under an argon inert atmosphere, 0.4 ml of 2,2,2-trichloroethylchloroformate and 0.52 g of $K_2CO_3$ are added. The mixture is heated at reflux for 16 hours, then cooled at room temperature, washed with water, then with an aqueous solution of citric acid at 15% and brine. The organic phase recovered at the end of the washings is anhydrified on sodium sulphate and the solvent evaporated, obtaining 0.76 g of a yellow oil (carbamate). The oil is dissolved in 25 ml of glacial acetic acid. 0.82 g of zinc in powder are added to the so obtained solution. The mixture is kept under stirring at room temperature for 16 hours and then diluted with about 25 ml of toluene, and finally evaporated under vacuum. The residue is dissolved in a minimum volume of dichloromethane and extracted three times with an aqueous solution of citric acid at 15% w. The aqueous phases are washed with dichloromethane, then made alkaline with conc. $NH_4OH$ and extracted again with dichloromethane. The organic phase is anhydrified on sodium sulphate and the solvent evaporated to obtain 0.28 g of the compound 3-propionyl-3,9-diazabicyclo[4.2.1]nonane (4a) as a yellow oil.

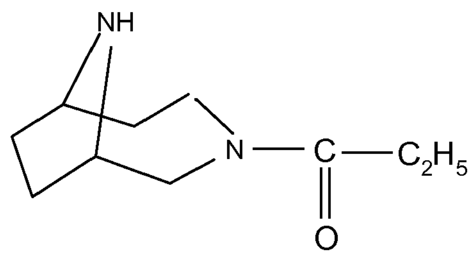

(4a)

Yield: 62%; $R_f$: 0.15 ($CH_2Cl_2$-MeOH 9:1); $^1$H-NMR ($CDCl_3$): δ (ppm) 1.16 (t,3H,J=7.6Hz), 1.25-2.00 (m,6H), 2.06 (bs, 1H,N*H*), 2.20-2.48 (m,2H), 2.75-2.85 (m,1H), 3.25-4.03 (m,4H), 4.39 (bd,1H,J=14Hz).

**EXAMPLE 5**

Synthesis of 9-propionyl-3,9-diazabicyclo-[4.2.1]nonane

0.5 g of the compound 3-propionil-3,9-diazabicyclo[4.2.1]nonane obtained in Example 4 are heated to 110˚C for one hour. The residue is purified by flash chromatography ($CH_2Cl_2$-MeOH 8:2) obtaining 0.21 g of the compound 9-propionyl-3,9-diazabicyclo[4.2.1]nonane (5a)

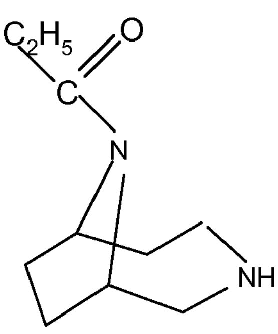

(5a)

Yield: 42%; $R_f$: 0.17 ($CH_2Cl_2$-MeOH 8:2); $^1$H-NMR ($CDCl_3$): δ (ppm) 1.16 (t,3H,J=7.6Hz), 1.20-2.65 (m,9H), 2.46 (bs, 1H,N*H*), 2.70-3.10 (m,3H), 4.13-4.30 (m,1H), 4.60-4.73 (m,1H).

**EXAMPLE 6**

Synthesis of the compound 9-benzyl-9-azabicyclo[3.3.1]nonan-3-one

To a solution in water (400 ml) of pentandial (84.5 g) and benzylamine hydrochloride (36.3 g), cooled at 0˚C, 30.8 g of 1,3-acetonedicarboxylic acid are added. 70 ml of an aqueous solution of sodium acetate at 10% are then added. The obtained mixture is kept under stirring at room temperature for one hour and then heated for 4 hours to 50˚C. The mixture is then acidified at pH 2 with a 10% w. HCl aqueous solution and then washed with diethyl ether. The pH is then brought to 6 with sodium bicarbonate and the aqueous phase extracted with dichloromethane. The organic extracts are anhydrified on sodium sulphate and the solvent evaporated to obtain 38.5 g of the compound 9-benzyl-9-azabicyclo[3.3.1]nonan-3-one (6a) as a light coloured solid.

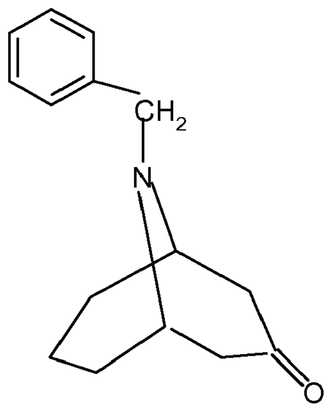

(6a)

Yield: 78%; B.p.: 165-169˚C/0.2 mmHg; $^1$H-NMR ($CDCl_3$): δ (ppm) 1.49-1.59 (m, 6H), 2.26 (d,2H,J=19Hz), 2.76 (dd, 2H,J=8 and 19Hz), 3.28-3.49 (bs,2H), 3.91 (s,2H), 7.22-7.46 (m,5H).

**EXAMPLE 7**

Synthesis of 10-benzyl-3,10-diazabicyclo-[4.3.1]decan-4-one

To a solution of 9-benzyl-9-azabicyclo[3.3.1]nonan-3-one (0.5 g) in chloroform (4.4 ml), cooled at -5˚C, 1 ml of conc. $H_2SO_4$ is dropwise added, while maintaining the temperature under 15˚C. $NaN_3$ (0.28 g) is then slowly added in small amounts each time, so to avoid that the reaction temperature exceeds 35˚C. It is then heated at reflux for 2 hours; the obtained mixture is poured into a vessel containing about 200 ml of ice. Solid $K_2CO_3$ is added up to have a strongly alkaline pH. An emulsion is formed, to which 25 ml of a KOH aqueous solution at 60% are added. It is left under stirring for 10 minutes, then the formed inorganic salts are filtered off and an extraction with chloroform is effected. The organic phase is anhydrified on sodium sulphate and the solvent evaporated to obtain 0.50 g of 10-benzyl-3,10-diazabicyclo[4.3.1]decan-4-one (7a) as a light solid.

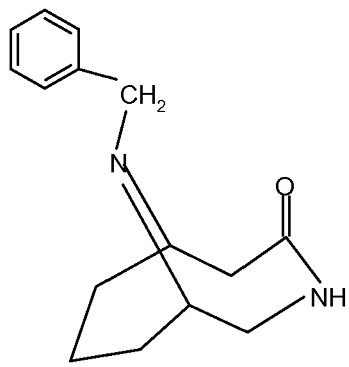

(7a)

Yield: 95%; $R_f$: 0.42 ($CHCl_3$-MeOH 97:3); $^1$H-NMR ($CDCl_3$): δ (ppm) 1.43-1.70 (m,3H), 1.90-2.23 (m,3H), 2.37-2.53 (m, 1H), 2.80-3.15 (m,4H), 3.75 (dt,1H,J=3.8 and 15Hz), 3.93 (s,2H), 5.82 (bs,1H), 7.20-7.40 (m,5H).

**EXAMPLE 8**

Synthesis of 10-benzyl-3,10-diazabicyclo-[4.3.1]decane

To a suspension of $LiAlH_4$ (0.19 g) in anhydrous THF (9 ml), cooled at 0°C, kept under an argon inert atmosphere, a solution of 10-benzyl-3,10-diazabicyclo[4.3.1]decan-4-one obtained in Example 7 (0.50 g) in THF (4 ml) is dripped. The mixture is kept under stirring at room temperature for 14 hours. The mixture is then cooled at 0°C, about 0.9 ml of $H_2O$ are added with caution, leaving afterwards under stirring for 10 min. The obtained precipitate is filtered under vacuum and washed with dichloromethane. The filtrate is evaporated, the obtained oil is dissolved in dichloromethane, the solution is anhydrified with sodium sulphate and the solvent evaporated to give 0.47 g of the compound 10-benzyl-3,10-diazabicyclo[4.3.1]decane (8a).

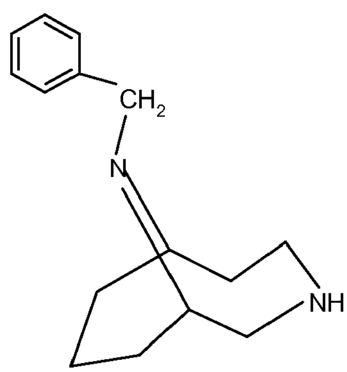

(8a)

Yield: quantitative; $R_f$: 0.62 ($CH_2Cl_2$-MeOH 8:2); $^1$H-NMR ($CDCl_3$): δ (ppm) 1.17-2.18 (m,8H), 2.62 (bs,1H), 2.76-2.92 (m,2H), 2.99-3.18 (m,4H), 3.97 (s,2H), 7.20-7.42 (m,5H).

**EXAMPLE 9**

Synthesis of 10-benzyl-3-propionyl-3,10-diazabicyclo[4.3.1] decane

To a solution of 10-benzyl-3,10-diazabicyclo[4.3.1]-decane obtained in Example 8 (0.63 g) in anhydrous dichloromethane (18 ml), cooled at 0°C, propionic anhydride (1.27 ml) dissolved in anhydrous dichloromethane (5 ml), is added. The mixture is heated at reflux for one hour, then cooled to room temperature, made alkaline with an aqueous

solution of NOH at 40% and left under stirring for 16 hours. The mixture is extracted with dichloromethane, the organic phase separated, anhydrified on sodium sulphate and evaporated. 0.73 g of the compound 10-benzyl-3-propionyl-3,10-diazabicyclo[4.3.1]decane (9a) are obtained as a light yellow oil.

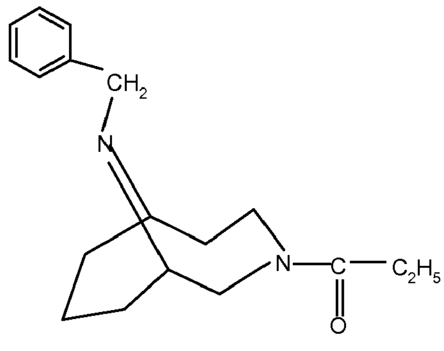

(9a)

Yield: 94%; $R_f$: 0.55 (AcOEt-ligroin 6:4); $^1$H-NMR ($CDCl_3$): δ (ppm) 1.05-1.30 (m,3H), 1.38-2.13 (m,8H), 2.20-2.46 (m, 2H), 2.82-3.18 (m,2H), 3.38-3.65 (m,3H), 3.72-4.00 (m,3H), 7.15-7.40 (m,5H).

**EXAMPLE 10**

Synthesis of the compound 3-propionyl-3,10-diazabicyclo-[4.3.1]decane

A solution in EtOH (39 ml) of 10-benzyl-3-propionyl-3,10-diazabicyclo[4.3.1] decane obtained in Example 9 (2.40 g) was hydrogenated at 45 psi and at room temperature for 19 hours in the presence of Pd/C 10% (0.89 g). The mixture was filtered off on celite and the catalyst washed with EtOH. The solution was concentrated to obtain 1.60 g of a light oil corresponding to the compound 3-propionyl-3,10-diazabicy-clo[4.3.1]decane (10a).

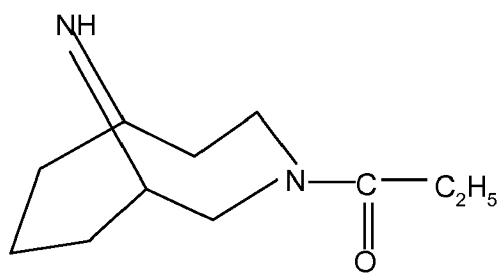

(10a)

Yield: 97%;$R_f$: 0.15 ($CH_2Cl_2$-MeOH 9:1); $^1$H-NMR ($CDCl_3$): δ (ppm) 1.16 (t,3H,J=7.6Hz), 1.35-2.09 (m,8H), 2.38 (q,2H, J=7.4Hz), 2.68-2.83 (m,2H), 3.06-3.96 (m,5H).

**EXAMPLE 11**

Synthesis of 10-benzyl-3-BOC-3,10-diazabicy-clo[4.3.1]decane

To a solution of di-*tert*-butyldicarbonate (BOC) in THF (1.38 g of $BOC_2O$ in 12 ml of THF), cooled at 0°C, a solution of 10-benzyl-3,10-diazabicyclo[4.3.1]decane obtained in Example 8 (0.97 g) in THF (8 ml) is added. The mixture is left under stirring for 10 minutes, warmed to room temperature and then left under stirring for 16 hours. The obtained solution was diluted with $Et_2O$ and washed with an aqueous solution of $NaHCO_3$ at 10% and then with brine. The organic phase was anhydrified on $Na_2SO_4$ and concentrated. The obtained residue was purified by flash chromatography (ligroin/$Et_2O$ 9:1). 1.11 g of the compound 10-benzyl-3-BOC-3,10-diazabicyclo[4.3.1]decane (11a) are obtained as a

colourless oil.

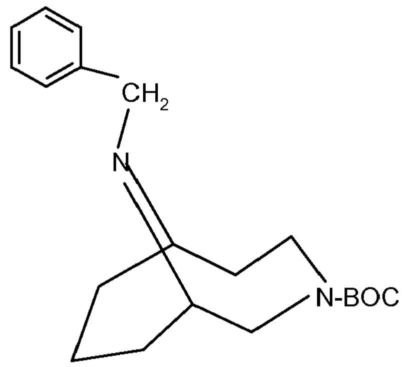

(11a)

Yield: 80%; $R_f$: 0.48 (ligroin/$Et_2O$ 9:1); $^1H$-NMR ($CDCl_3$): δ (ppm) 1.18-2.15 (m,8H), 1.46 (s,3H), 1.50 (bs,6H), 2.80-3.00 (m,1H), 3.05-3.22 (m,1H), 3.23-3.90 (m,4H), 3.92 (s,2H), 7.20-7.50 (m,5H).

**EXAMPLE 12**

Synthesis of 3-BOC-3,10-diazabicyclo-[4.3.1]-decane

A solution of 10-benzyl-3-Boc-3,10-diazabicyclo-[4.3.1]decane obtained in Example 11 (3.50 g) in EtOH (52 ml) underwent hydrogenation at 45 psi and 30˚C for 19 hours in the presence of Pd/C 10% (1.13 g). At the end the mixture was filtered on celite and the catalyst washed with EtOH. The solution was then concentrated. 2.44 g of a light oil corresponding to the compound 3-BOC-3,10-diazabicyclo[4.3.1]decane (12a) are obtained.

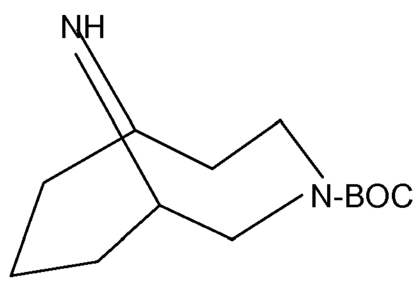

(12a)

Yield: 96%; $R_f$: 0,55 ($CHCl_3$-MeOH 9:1); $^1H$-NMR ($CDCl_3$): δ (ppm) 1.38-2.00 (m,8H), 1.48 (s,9H), 2.30 (bs,1H), 3.05-3.42 (m,4H), 3.60-3.88 (m,2H).

**EXAMPLE 13**

Synthesis of 3-BOC-10-propionyl-3,10-diazabicy-clo[4.3.1] decane

To a solution in anhydrous dichloromethane (8 ml) of 3-BOC-3,10-diazabicyclo[4.3.1]decane obtained in Example 12 (0.34 g), cooled at 0˚C, propionic anhydride (0.66 ml) dissolved in anhydrous dichloromethane (3 ml), is added. The mixture is heated at reflux for one hour, then cooled to room temperature, then made alkaline with an aqueous solution of NaOH at 40% and left under stirring for 16 hours. The mixture is extracted with dichloromethane, the organic phase separated, anhydrified on sodium sulphate and evaporated to give 0.32 g of a colourless oil corresponding to 3-BOC-10-propionyl-3,10-diazabicyclo[4.3.1] decane (13a).

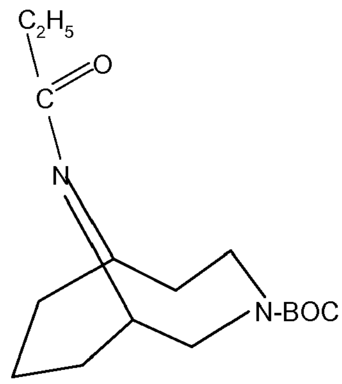

(13a)

Yield: 76%; $R_f$: 0.32 (ligroin-AcOEt 7:3); $^1H$-NMR ($CDCl_3$): δ (ppm) 1.17 (t,3H,J=8.8Hz), 1.44 (s,3H), 1.48 (s,6H), 1.50-2.60 (m,11H), 2.90-3.20 (m,3H), 3.82-4.40 (m,1H), 4.73-5.17 (m,1H).

**EXAMPLE 14**

Synthesis of 10-propionyl-3,10-diazabicyclo-[4.3.1]decane

To a solution in dichloromethane (7 ml)of 3-BOC-10-propionyl-3,10-diazabicy-clo[4.3.1] decane obtained in Example 13 (0.32 g), cooled at 0˚C, trifluoroacetic acid (0.83 ml) in 3 ml of dichloromethane, is added. The mixture is kept under stirring at room temperature for 3 hours. The solvent is then evaporated and the residue treated with an aqueous solution of saturated $NaHCO_3$, then extracted with $CH_2Cl_2$. The organic phase is separated, anhydrified on sodium sulphate and evaporated. 0.21 g of a light oil corresponding to the compound 10-propionyl-3,10-diazabicyclo [4.3.1]decane (14a) are obtained.

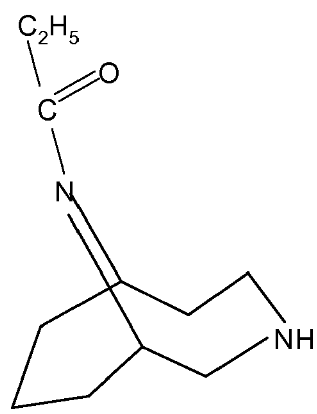

(14a)

Yield: quantitative; $R_f$: 0.20 ($CH_2Cl_2$-MeOH 8:2); $^1H$-NMR ($CDCl_3$): δ (ppm) 1.17 (t,3H,J=7.2Hz), 1.31-2.60 (m,11H), 1.84 (bs,1H), 2.76-3.12 (m,3H), 3.92-4.40 (m,1H), 4.71-5.17 (m,1H).

**EXAMPLE 15**

General method for preparing 3-propionyl-9-alkyl-3,9-diazabi-cyclo[4.2.1]nonane (I-A) compounds

A mixture formed of the compound 3-propionyl-3,9-diazabi cyclo[4.2.1]nonane (4a) obtained in Example 4 (0.6 mmoles), $K_2CO_3$ (0.72 mmoles), acetone (7 ml) and an organic chloride (0.72 mmoles) of general formula (II-A):

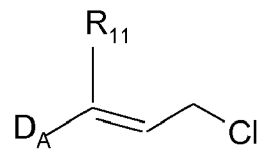

(II-A)

wherein $D_A$ and $R_{11}$ have the meanings reported in Table 1, is heated at reflux for 24 hours. The mixture is then cooled to room temperature, the formed solid filtered and acetone evaporated. The residue has been purified by flash chromatography ($CH_2Cl_2$/acetone 8:2) obtaining the compounds 3-propionyl-9-alkyl-3,9-diazabicyclo[4.2.1]nonanes of formula I-Aa up to I-Ad, as oils, characterized by the following general chemical structure (I-A):

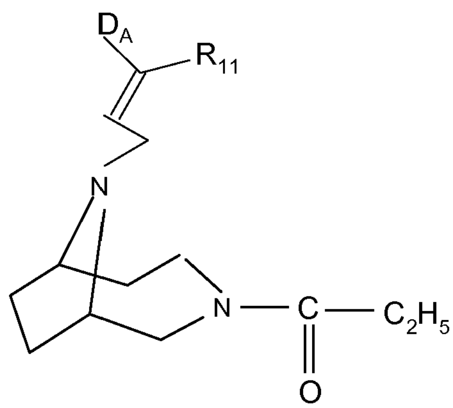

(I-A)

In Table 1 for each of the synthesized compounds I-Aa, I-Ab, I-Ac and I-Ad, it is indicated: $D_A$, $R_{11}$, reaction yield in per cent (Yield %), the melting point in centigrade degrees (m.p. in ˚C) of the corresponding fumarate salts, the empirical formula, the wave length (λ) of the IR band corresponding to the group -C(O)-, the significant peaks of the $^1$H-NMR analysis in $CDCl_3$ ($^1$H-NMR δ ppm).

**EXAMPLE 16**

General method for preparing 3-alkyl-9-propionyl-3,9-diazabi-cyclo[4.2.1]nonanes (I-B)

The same procedure described in the preparation of 3-propionyl-9-alkyl-3,9-diazabicyclo[4.2.1]nonanes (Example 15) has been used for the preparation of 3-alkyl-9-propionyl-3,9-diazabicyclo[4.2.1]nonanes by using the compound 9-propionyl-3,9-diazabicyclo[4.2.1]nonane (5a) obtained in Example 5 instead of the compound 3-propionyl-3,9-diazabicyclo[4.2.1]nonane (4a). The compound (5a) was reacted with the organic chlorides of general formula (II-A) (see Example 15), wherein $D_A$ and $R_{11}$ have the values reported in Table 1. In the Table the compounds 3-alkyl-9-propionyl-3,9-diazabicyclo [4.2.1]-nonanes of formula I-B: I-Ba, I-Bb, I-Bc and I-Bd which have been synthesized with this method, are reported. They are in the physical form of oils and are characterized by the general chemical structure (I-B).

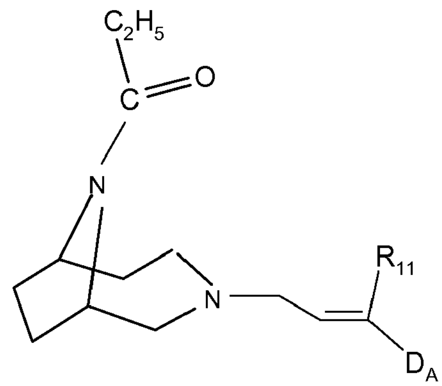

(I-B)

**EXAMPLE 17**

General method for the preparation of 3-propionyl-10-alkyl-3,10-diazabicyclo[4.3.1]decanes (I-C)

The same method described in Example 15 has been used for the preparation of the compounds 3-proprionyl-10-alkyl-3,10-diazabicyclo[4.3.1]decanes characterized by the following general chemical structure (I-C):

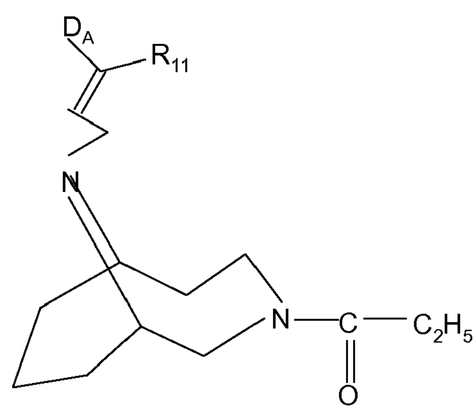

(I-C)

With respect to the Example 5, in this case in the synthesis it is used the compound 3-propionyl-3,10-diazabicyclo [4.3.1]decane (10a) obtained in Example 10 in place of 3-propionyl-3,9-diazabicyclo[4.2.1]nonane (4a). The compounds of general formula I-C have been synthesized by using the organic chlorides of general formula (II-A), see Example 15, wherein $D_A$ and $R_{11}$ have the meanings reported in Table 1 for each of the compounds synthesized according to this method: I-Ca, I-Cb, I-Cc.

**EXAMPLE 18**

General method for the preparation of 3-alkyl-10-propionyl-3,10-diazabicyclo[4.3.1]decanes (I-D)

The same method described for the preparation of 3-propionyl-9-alkyl-3,9-diazabicyclo[4.2.1]nonanes (Example 15) is carried out, by using the compound 10-propionyl-3,10-diazabicyclo [4.3.1]decane (14a) obtained in Example 14 in place of the compound 3-propionyl-3,9-diazabicyclo-[4.2.1]nonane (4a). The organic chlorides used are those of general formula (II-A), wherein $D_A$ and $R_{11}$ have the meanings reported in Table 1. The obtained compounds 3-alkyl-10-propionyl-3,10-diazabicyclo[4.3.1]decanes have formula (I-D) and are in the physical form of oils.

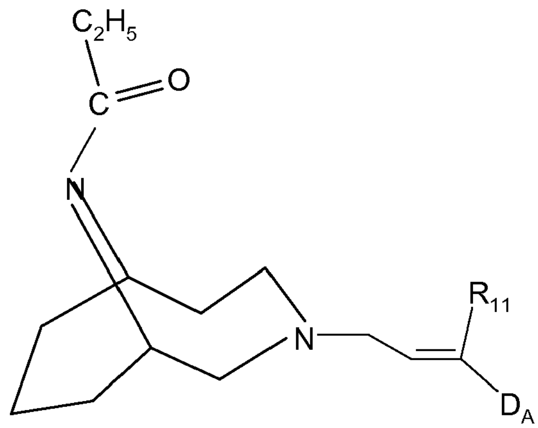

(I-D)

The following compounds: I-Da, I-Db and I-Dc are reported in the Table.

Table 1

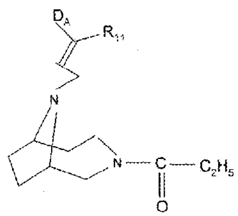
(I-A)

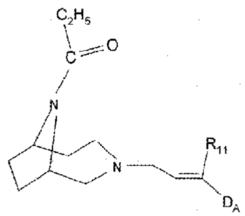
(I-B)

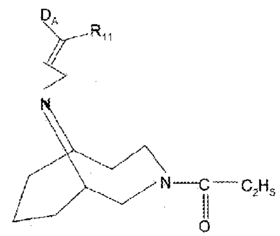
(I-C)

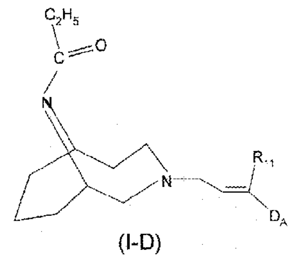
(I-D)

| Example | $R_{11}$ | $D_A$ | Yield % | m.p. (˚C) | Formula (analysis) | IR λ ($cm^{-1}$) | $^1$H-NMR δ ppm |
|---|---|---|---|---|---|---|---|
| (**I-Aa**) | $CH_3$ | Phenyl | 30 | 230-232 | $C_{20}H_{28}N_2O$ (C,H,N) | 1658 | 1.16 (t,3H,J=7.4Hz), 1.20-2.50 (m,7H), 2.04 (s, 3H), 2.79 (dd,1H,J=2.6 e 13.2Hz), 3.18-4.03 (m, 5H), 3.35 (d,2H,J=6.2Hz), 4.40 (bd,1H,J=12.4Hz), 5.88 (t. 1H,J=6.0Hz), 7.15-7.50 (m, 5H) |
| (**I-Ab**) | $CH_3$ | 3,4-dichloro phenyl | 31 | 188-189 | $C_{20}H_{26}Cl_2N_2O$ (C,H,N) | 1660 | 1.16 (t,3H,J=7.6Hz), 1.20-2.53 (m,7H), 2.00 (s, 3H), 2.81 (bd,1H,J=12.8Hz), 3.12-4.04 (m, 7H), 4.38 (bd, 1H,J=12.2Hz), 5.88 (t,1H, J=6.0Hz), 7.23 (d,1H, J=9.0Hz), 7.38 (d,1H, J=8.2Hz), 7.46 (s,1H) |
| (**I-Ac**) | $CH_3$ | 4-Cl-Naphthyl | 76 | 97-101 | $C_{24}H_{29}ClN_2O$ (C,H,N) | 1650 | 1.16 (t,3H,J=7.6Hz), 1.36-1.88 (m,8H), 2.08 (s, 3H), 2.71-2.87 (m,1H), 3.18-4.24 (m,6H), 4.43 (d, 1H,12.0Hz), 5.44-5.78 (m, 1H), 7,36-8,42 (m,6H) |

(continued)

| Example | $R_{11}$ | $D_A$ | Yield % | m.p. (°C) | Formula (analysis) | IR λ ($cm^{-1}$) | $^1$H-NMR δ ppm |
|---|---|---|---|---|---|---|---|
| (**I-Ad**) | Phenyl | Phenyl | 27 | 176-177 | $C_{25}H_{30}N_2O$ (C,H,N) | 1659 | 1.13 (t,3H,J=7.4Hz), 1.20-2.46 (m,7H), 2.77 (dd, 1H,J=2.6 and 12.8Hz), 3.10-4.00 (m, 5H), 3.22 (d, 2H,J=6.6Hz), 4.34 (bd,1H, J=12.2Hz), 6.20 (dt,1H, J=2.2 e 6.8Hz), 7.00-7.46 (m, 10H) |
| (**I-Ba**) | $CH_3$ | Phenyl | 39 | 228-230 | $C_{20}H_{28}N_2O$ (C,H,N) | 1644 | 1.16 (t,3H,J=7.4Hz), 1.40-3.04 (m,12H), 2.04 (s, 3H), 3.24 (d,2H,J=6.6Hz), 4.02-4.18 (m,1H), 4.55-4.75 (m,1H), 5.83 (t,1H,J=6.6Hz), 7.20-7.50 (m,5H) |
| (**I-Bb**) | $CH_3$ | 3,4-dicloro-Fenile | 29 | 186-188 | $C_{20}H_{26}Cl_2N_2O$ (C,H,N) | 1643 | 1.16 (t,3H,J=7.6Hz), 1.40-2.83 (m.12H). 2.00 (s, 3H), 3.23 (d,2H,J=6.6Hz), 4.05-4.25 (m,1H), 4.55-4.75 (m,1H), 5.83 (t,1H,J=6.2Hz), 7.20 (dd,1H,J=2.2 and 8.6Hz), 7.38 (dd,1H,J=1.2 and 8.4Hz), 7.45 (d,1H, J=2.0Hz) |
| (**I-Bc**) | $CH_3$ | 4-Cl-Naphthyl | 50 | 95-99 | $C_{24}H_{29}ClN_2O$ (C,H,N) | 1650 | 1.22 (t,3H,J=7.4 Hz), 1.20-2.00 (m,8H), 2.19 (s, 3H), 2.19-2.92 (m,4H), 3.30 (d,2H.J=7,4Hz), 4.22-4.32 (m,1H), 4.58-4.68 (m,1H), 5.79 (m,1H), 7.14-8.33 (m, 6H) |

(continued)

| Example | $R_{11}$ | $D_A$ | Yield % | m.p. (°C) | Formula (analysis) | IR λ ($cm^{-1}$) | $^1$H-NMR δ ppm |
|---|---|---|---|---|---|---|---|
| (**I-Bd**) | Phenyl | Phenyl | 41 | 184-186 | $C_{25}H_{30}N_2O$ (C,H,N) | 1642 | 1.15 (t,3H,J=7.4Hz), 1.40-2.82 (m,12H), 3.10-3.22 (m,2H), 4.04-4.28 (m,1H), 4.55-4.75 (m,1H), 6.15 (t,1 H,J=6.6Hz), 7.10-7.47 (m,10H) |
| (**I-Ca**) | $CH_3$ | Phenyl | 64 | 106-108 | $C_{21}H_{30}N_2O$ (C,H,N) | 1640 | 1.18 (t,3H.J=7,6Hz), 1.20-2.55 (m,10H), 2.07 (s, 3H), 2.87-4.34 (m,8H), 5.75-5.92 (m,1H), 7.10-7.43 (m,5H) |
| (**I-Cb**) (**I-Cb**) | $CH_3$ | 3.4-dichloro-phenyl | 67 | 184-185 | $C_{21}H_{28}Cl_2N_2O$ (C,H,N) | 1655 | 1.18 (t,3H,J=7.6Hz), 1.20-2.54 (m,10H), 2 02 (s, 3H), 2.83-4.13 (m,8H), 5.69-6.01 (m,1H), 7.17-7.53 (m,3H) |
| (**I-Cc**) | Phenyl | Phenyl | 68 | 137-139 | $C_{25}H_{32}N_2O$ (C,H,N) | 1660 | 1.15 (t,3H,J=7.6Hz), 1.20-2.49 (m,10H), 2.87-4.06 (m,8H). 6.05-6.23 (m,1H), 7.08-7.44 (m,10H) |
| (**I-Da**) | $CH_3$ | Phenyl | 64 | 144-146 | $C_{21}H_{30}N_2O$ (C,H,N) | 1650 | 1.17 (t,3H,J=7.6Hz), 1.34-3.00 (m,14H), 2.03 (s, 3H), 3.15-3.23 (m,2H), 3.80-4.38 (m,1H), 4.60-5.17 (m,1H), 5.76-5.95 (m,1H), 7.02-7.53 (m,5H) |
| (**I-Db**) | $CH_3$ | 3,4-dichloro-phenyl | 58 | 175-176 | $C_{21}H_{28}Cl_2N_2O$ (C,H,N) | 1660 | 1.17 (t,3H,J=6.2Hz), 1.18-2.95 (m,14H), 1.99 (s, 3H), 3.15-3.24 (m,2H), 3.80-4.40 (m, 1H), 4.62-5.11 (m, 1H), 5.72-5.92 (m, 1H), 7.14-7.47 (m,3H) |

(continued)

| Example | $R_{11}$ | $D_A$ | Yield % | m.p. (˚C) | Formula (analysis) | IR λ ($cm^{-1}$) | $^1H$-NMR δ ppm |
|---|---|---|---|---|---|---|---|
| (**I-Dc**) | Phenol | Phenyl | 52 | 169-170 | $C_{26}H_{32}N_2O$ (C,H,N) | 1634 | 1.14 (t,3H,J=7.4Hz), 1.25-2.57 (m,12H), 2.57-2.86 (m,2H), 3.12 (dd, 2H.J=1.6 and 5.8Hz), 3.80-4.32 (m,1H), 4 64-5.13 (m,1H), 6.16 (t,1H,J=6.0Hz), 7.10-7.40 (m,10H) |

**EXAMPLE 19**

Affinity towards the opioidergic receptors μ, δ, k

The affinity of the compounds synthesized in the preceeding examples towards the opioidergic receptors μ, δ, k has been evaluated in vitro by radioreceptorial binding studies by using the method reported hereinunder.

The technique of the receptorial binding allows to establish if and with what affinity and specificity, a specific compound binds to a particular receptor.

To evaluate the affinity of a specific compound to a particular receptor it is necessary to challenge (in a particular preparation of the tissue wherein those specific receptors are present) the compound to be tested with a radioactive labelled compound with known affinity . The property of the tested compound to displace the radioactive compound gives an index of the affinity of the compound for the binding to that specific receptor. The radioactivity value found in the receptor- compound complex allows furthermore to calculate with great precision the amount of compound bound to the receptor. By this method it is therefore possible to quickly identify the affinity of a new compound towards a specific receptor and thus determine its pharmacological activity. By repeating the same experimental design it is possible to evaluate the affinity of the compound towards other kinds of receptors and thus establish its specificity degree.

The receptorial binding technique, besides being used for the screening of new molecules with pharmacological activity, can give useful information on possible changes at the receptorial level, correlated for example with a prolonged exposure to drugs and/or to particular pathologies. In these situations, indeed, changes in the amount of the present receptors, or conformational changes can occur, which alter the affinity of the agonists or antagonists with consequence on the normal function of the receptors themselves.

The experimentation has been carried out according to the guide lines of the European Community for the animal experimentation (EEC n. 86/609), by using laboratory animals (male mices CD1 Charles River Italy, Calco, LC, Italy) lodged twenty in a cage, under standard stabulation conditions (temperature 22±2°C, relative humidity 60%, artificial light with a light/dark cycle of 12 hours). The food and water were at disposal ad libitum.

The binding experiments were carried out according to the following methods.

Receptors k: CD1 male mice weighing 20-25 g were used. The animals were sacrificed by cervical dislocation and the complete brain (excluding the cerebellum) was quickly dissected and kept in ice. The tissue was homogeneized in 40 volumes (w/v) of Tris-HCl buffer (50 mM, pH 7.4) by an Ultra-Turrax, then centrifuged for 20 minutes at 48,000 x g in a centrifuge kept at 4°C. The resulting supernatant was suspended again in the same buffer and incubated at 37°C for 30 minutes in a bath kept under oscillation. At the end of the incubation the suspension was centrifuged 48,000 x g for 15 minutes and the pellets suspended again in 10 volumes of the Tris-HCl buffer. The binding experiment was carried out in a volume of 1 ml at the temperature of 25°C with about 800-1000 μg of proteins for sample. The incubation was carried out for 60 minutes in the presence of different concentrations of the ligand $^3$H-U 69.593 (41.7 Ci/mmole). The non specific binding was determined in the presence of U69593 (10 μM). The incubation was stopped by quick filtering by means of a filtration instrument (Brandell® , Gaithersburg, MD, USA) by using GF/C filters (Whatman®).

Receptors μ and δ: the experiments were carried out according to the method described by Unterwald (1995) by using CD1 male mice weighing 20-25 g, lodged 20 for cage under standard stabulation conditions (temperature 22±2°C, relative humidity 60%, artificial light with light dark cycle of 12 hours). After the sacrifice, total brain (excluding the cerebellum)of the animals was quickly removed from the animals. The so obtained tissues were quickly homogenized, by Polytron®, in 50 volumes of Tris HCl buffer (50 mM), pH 7.4 and the homogenate then centrifuged at 48,000 x g for 20 minutes at 4°C. The resulting pellets were suspended in 50 volumes of the same buffer and the suspension incubated at 37°C for 45 minutes, in a bath kept under oscillation, so to make easier the separation of the endogenous opioids from the receptors. At the end of the incubation the suspensions were centrifuged at 48,000 x g, for 20 minutes at 4°C and the resulting pellets suspended in 40 volumes of the Tris HCl buffer (50 mM), pH 7.4. The obtained suspension of cerebral membranes was used for the binding tests.

The binding experiment was carried out in a 2 ml volume, at the temperature of 25°C, with 50-100 μg of proteins in each sample; the incubation was carried out for 60 minutes in the presence of 1 nM [$^3$H]-DAMGO (54.5 Ci/mmole) or 1 nM [$^3$H]-DPDPE (45 Ci/mmole), respectively for the study of the receptors μ and δ.

The non specific binding was determined in the presence of naloxone (10 μM). For drawing of the competition curves at least eight different concentrations of each compound were used. As a reference compound morphine was used at concentrations comprised between $10^{-10}$ and $10^{-5}$ M.

The incubation was interrupted by quick filtration by GF/B filters (Whatman®), by means of a filtration instrument (Brandel®, Gaithersburg, MD, USA). The filters were washed three times with 5 ml of cold Tris HCl buffer (50 mM), pH 7.4.

The radioactivity was determined by a scintillator in liquid phase (Tricarb® 2100, Packard, Meridien, IL, USA), by using three ml of scintillating fluid (Ultima Gold MV, Packard, Meridien, IL, USA).

The protein determination was carried out by the Bradford method (1976) by using the protocol and the reactants supplied by Bio-Rad (Milano, Italy).

The affinity of the compounds towards the receptors μ, δ, κ has been expressed in Ki terms.

The results of the binding experiments are shown in Table 2.

Table 2

| Affinity of the compounds of the invention towards the opioidergic receptors μ, δ and k (affinity values expressed as $K_i$) | | | |
|---|---|---|---|
| Compound (Example) | $K_i$ μ (nM) | $K_i$ δ(nM) | $K_i$ k(nM) |
| I-Aa | 5.7 ± 1.0 | 425 ± 25 | 55 ± 25 |
| I-Ab | 7.0 ± 1.5 | 867 ± 44 | 100 ± 5 |
| I-Ac | 56.7 ± 13 | 2000 ± 250 | 2500 ± 125 |
| I-Ad | 10.7 ± 0.3 | 142 ± 25 | 103 ± 7 |
| I-Ba | 8.3 ± 1.2 | 933 ± 33 | 900 ± 100 |
| I-Bb | 4.1 ± 0.35 | 850 ± 58 | 393 ± 23 |
| I-Bc | 68.3 ± 10.9 | >5,000 | >10,000 |
| I-Bd | 20.7 ± 2.33 | 333 ± 5 | 1133 ± 186 |
| I-Ca | 17.0 ± 1.3 | 1667 ± 166 | 410 ± 10 |
| I-Cb | 4.25 ± 0.3 | 1117 ± 216 | 425 ± 38 |
| I-Cc | 3.16 ± 0.6 | 187 ± 29.7 | 40 ± 0.1 |
| I-Da | 40 ± 2.8 | 3875 ± 125 | 1467 ± 317 |
| I-Db | 17.5 ± 1.4 | 3250 ± 166 | 1167 ± 120 |
| I-Dc | 23.3 ± 1.6 | 1688 ± 312 | 1933 ± 233 |

## EXAMPLE 20

Microemulsion preparation

5.05 mg of the compound I-Aa obtained in Example 15 was dissolved at 25˚C in 5.08 mg of the triglyceride Miglyol® 812S (Sasol Germany GmbH), that is a mixture of triglycerides of the caprylic and capric acid. To the obtained oily solution 45.10 mg of the non-ionic surfactant Solutol® HS15 (Basf) (polyethylenglycol 660 hydroxystearate) and 2.445 g of physiological solution have been added. The obtained sample was heated for 5 minutes at 40˚C and then cooled at room temperature. The final sample, completely liquid and isotropic at 25˚C, was a microemulsion having the following composition (% by weight):

| | |
|---|---|
| compound I-Aa | 0.2% |
| Oil (Miglyol® 812S) | 0.2% |
| Solutol® HS15 | 1.8% |
| Aqueous phase (physiological solution) | 97.8% |

## EXAMPLE 21

Microemulsion preparation

The procedure of the Example 20 has been repeated but using 10.09 mg of the compound I-Aa and 2.450 g of physiological solution. The obtained microemulsion, completely liquid and isotropic at 25˚C, had the following composition (% by weight):

| | |
|---|---|
| Compound I-Aa | 0.4% |
| Oil (Miglyol® 812S) | 0.2% |
| Solutol® HS15 | 1.8% |
| Aqueous phase (physiological solution) | 97.6% |

**EXAMPLE 22**

Microemulsion preparation

5.05 mg of the compound I-Ba obtained in Example 16 were mixed at 40°C with 2.50 mg of the triglyceride Miglyol® 810 (Sasol Germany GmbH), that is a triglyceride of the capric/caprylic acid, 2.52 mg of the monoglyceride Imwitor® 308 (Sasol Germany GmbH), a lipidic mixture containing a percentage by weight higher than 80% of glyceryl monocaprylate monoester, and 45.10 mg of the non-ionic surfactant Solutol HS15 (Basf). The obtained sample, cooled at room temperature, is limpid. To this sample 2.445 g of physiological solution were added. The obtained sample was heated for 5 minutes at 40°C and then cooled at room temperature. The obtained microemulsion, completely liquid and isotropic at 25°C, has the following composition (% by weight):

| | |
|---|---|
| Compound I-Ba | 0.2% |
| Triglyceride (Miglyol® 810) | 0.1% |
| Monoglyceride (Imwitor® 308) | 0.1% |
| Solutol® HS15 | 1.8% |
| Aqueous phase (physiological solution) | 97.8% |

**EXAMPLE 23**

Microemulsion preparation

The procedure of Example 22 has been repeated but using 10.07 mg of the compound I-Ba and 2.450 g of physiological solution. The microemulsion appears completely liquid and isotropic at 25°C and has the following composition (% by weight):

| | |
|---|---|
| Compound I-Ba | 0.4% |
| Triglyceride (Miglyol® 810) | 0.1% |
| Monoglyceride (Imwitor® 308) | 0.1% |
| Solutol® HS15 | 1.8% |
| Aqueous phase (physiological solution) | 97.6% |

**EXAMPLE 24**

Microemulsion preparation

5.01 mg of the compound I-Ba obtained in Example 16 have been solubilized in 40.00 mg of soya oil (Aldrich) at 25°C. The obtained oily solution was slowly added, under stirring, to 0.956 g of an aqueous solution, maintained at 60°C, obtained by solubilizing 0.205 g of non-ionic surfactant Brij® 97 (Aldrich), polyoxyethylen-10-oleyl ether $C_{18:1}E_{10}$, in 0.751 g of physiological solution. The final sample was cooled at room temperature obtaining a completely liquid and isotropic microemulsion at 25°C, having the following composition (% by weight):

| | |
|---|---|
| Compound I-Ba | 0.5% |
| Soya oil | 4.0% |
| Brij® 97 | 20.5% |
| Aqueous phase (physiological solution) | 75.0% |

**EXAMPLE 25**

Microemulsion preparation

2.03 mg of compound I-Cb obtained in Example 17 were solubilized in 30.02 mg of soya oil (Aldrich) at 25°C. The obtained oily solution was slowly added, under stirring, to 0.968 g of an aqueous solution, kept at 60°C, obtained by solubilizing 0.400 g of non-ionic surfactant Solutol® HS15 (Basf) in 0.568 grams of physiological solution. The final sample was cooled to room temperature obtaining a completely liquid and isotropic microemulsion at 25°C having the

following composition (% by weigh):

| | |
|---|---|
| Compound I-Cb | 0.2% |
| Soya oil | 3.0% |
| Solutol® HS15 | 40.0% |
| Aqueous phase (physiological solution) | 56.8% |

**EXAMPLE 26**

Microemulsion preparation

3.03 mg of compound I-Db obtained in Example 18 were added to 1.0 g of a microemulsion obtained by mixing at 25°C the following ingredients: 0.070 g of soya lecithin, 0.049 g of Solutol® HS15 (Basf), 0.114 g of PEG® 400, 0.278 g of physiological solution, 0.049 g of ethanol, 0.440 g of triglyceride Miglyol® 810 (Sasol Germany GmbH). The obtained microemulsion at 25°C appears completely liquid and isotropic and has the following composition (% by weight):

| | |
|---|---|
| Compound I-Db | 0.3% |
| Soya lecithin | 7.0% |
| Peg 400 | 11.4% |
| Solutol® | 4.9% |
| Physiological solution | 27.7% |
| Ethanol | 4.9% |
| Oil (Migliol® 810) | 43.8% |

**EXAMPLE 27**

Microemulsion preparation

30 mg of the opioidergic compound 9-(3,3-diphenylprop-2-enyl)-3-propionyl-3,9-diazabicyclo[3.3.1]nonane were synthes-ized according to the procedure indicated by G. A. Pinna et al. in Bioorganic & Medicinal Chemistry, 10 (2002) 1929-1937 (see in the publication the opioidergic ligand 2d) and has the following chemical structure:

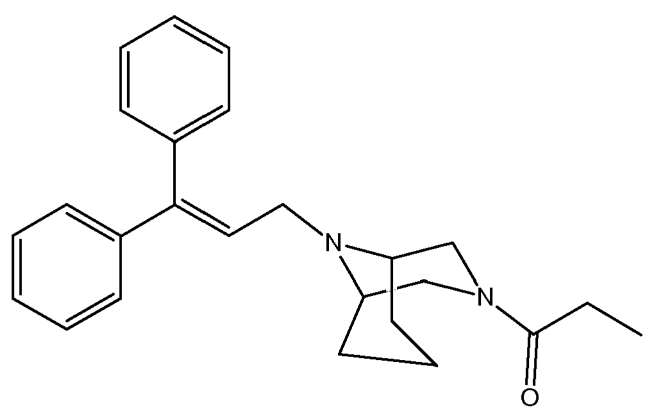

2.52 mg of the compound were solubilized in 5.08 mg of the triglyceride Miglyol® 812S (Sasol Germany GmbH) at 25°C. 45.10 mg of the non-ionic surfactant Solutol® HS15 (Basf) and 2.445 g of physiological solution have been added to the obtained oily solution. The obtained sample was heated for 5 minutes to 40°C and then cooled at room temperature. The obtained microemulsion is completely liquid and isotropic at 25°C and has the following composition (% by weight):

| | |
|---|---|
| Opoidergic compound | 0.1% |
| Oil (Miglyol® 812S) | 0.2% |

(continued)

| | |
|---|---|
| Solutol® HS15 | 1.8% |
| Aqueous phase (physiological solution) | 97.9% |

**EXAMPLE 28**

Microemulsion preparation

According to the procedure indicated in Example 1 of USP 5,672,601, 20 mg of the opioidergic compound $N^8$-acetyl-$N^3$-Cinnamyl-3,8-diazabicyclo[3.2.1] octane were sinthesized, of the following structure:

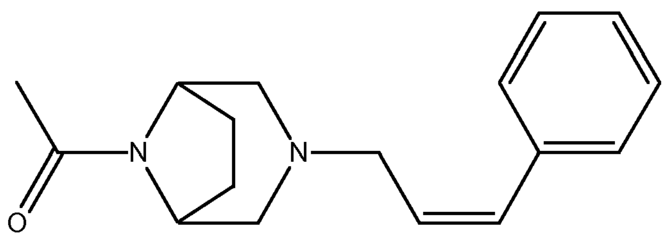

4,03 mg of the compound were solubilized in 5.08 mg of the triglyceride Miglyol® 812S (Sasol Germany GmbH) at 25˚C. 45.10 mg of the non-ionic surfactant Solutol® HS15 (Basf) and 2.445 g of physiological solution have been added under stirring to the obtained oily solution. The obtained sample was heated for 5 minutes to 40˚C and then cooled at room temperature. The obtained microemulsion is completely liquid and isotropic at 25˚C and has the following composition (% by weight):

| | |
|---|---|
| Opioidergic compound | 0.2% |
| Oil (Miglyol® 812S) | 0.2% |
| Solutol® HS15 | 1.8% |
| Aqueous phase (physiological solution) | 97.8% |

**EXAMPLE 29**

Microemulsion preparation

50 mg of the opioidergic compound 1-{6-[3-(4-chloro-phenyl)-but-2-enyl]-3,6-diaza-bicyclo[3.1.1]ept-3-yl}-propan-1-one have been synthesized according to the procedure indicated in patent application WO2005/108402. The compound, corresponding to the opoidergic ligand IAf shown at page 24 of said patent application, has the following chemical structure:

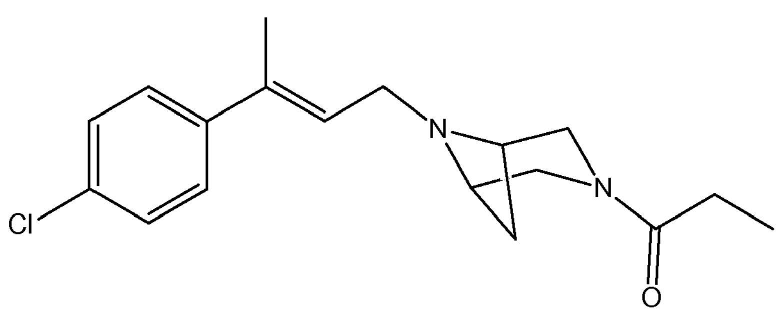

2,73 mg of said compound were solubilized in 5.08 mg of the triglyceride Miglyol® 812S (Sasol Germany GmbH) at 25˚C. 45.10 mg of the non-ionic surfactant Solutol® HS15 (Basf) and 2.445 g of physiological solution have been

added to the obtained oily solution. The obtained sample was heated for 5 minutes to 40°C and then cooled at room temperature. The obtained microemulsion appears completely liquid and isotropic at 25°C and has the following composition (% by weight):

| | |
|---|---|
| Opioidergic compound | 0.1% |
| Oil (Miglyol® 812S) | 0.2% |
| Solutol® HS15 | 1.8% |
| Aqueous phase (physiological solution) | 97.9% |

**EXAMPLE 30**

Microemulsion preparation

The opioidergic derivative active principle was prepared by starting from a mixture formed of compound 3-Propionyl-3,9-diazabicyclo[4.2.1]nonane obtained in Example 4 (0.6 mmoles), $K_2CO_3$ (0.72 mmoles), acetone (7 ml) and 0.72 mmoles of the chloride of formula:

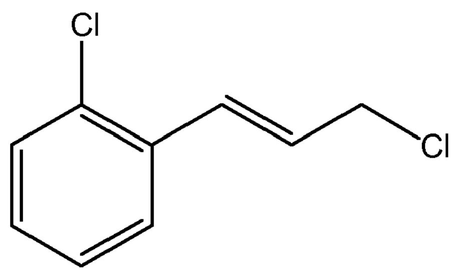

The mixture is heated, at reflux, for 24 hours. The mixture is then brought again to room temperature, the solid is filtered and acetone evaporated. The residue was then purified by flash chromatography ($CH_2Cl_2$/acetone 8:2) obtaining the following compound under the form of an oil:

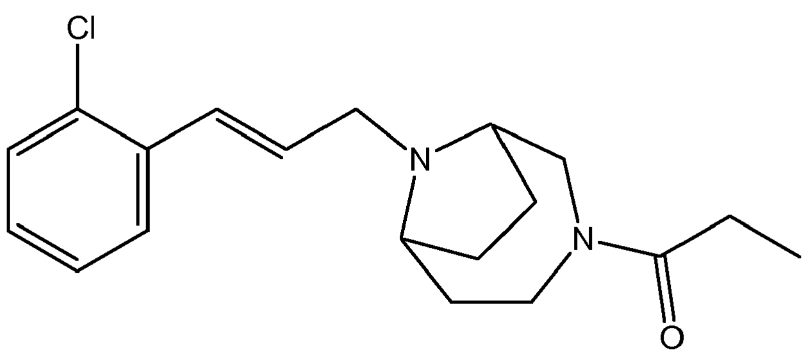

The procedure reported in Example 23 has been repeated for preparing the microemulsion, by substituting the compound I-Ba obtained in Example 16 with the above mentioned opioidergic derivative. The obtained microemulsion, completely liquid and isotropic at 25°C, has the following composition (% by weight):

| | |
|---|---|
| Opioidergic compound | 0.4% |
| Triglyceride (Miglyol® 810) | 0.1% |
| Monoglyceride (Imwitor® 308) | 0.1% |
| Solutol® HS15 | 1.8% |
| Aqueous phase (physiological solution) | 97.6% |

**EXAMPLE 31**

Microemulsion preparation

The opioidergic derivative active principle was prepared by repeating Example 16 by using instead of the organic chloride of formula (IIa), that used in Example 30. It was thus obtained the derivative having the following chemical structure:

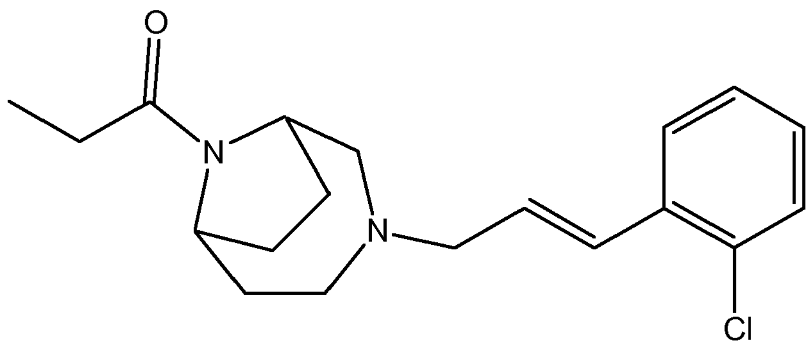

The procedure reported in Example 23 has been repeated for preparing the microemulsion, by substituting the compound I-Ba obtained in Example 16 with the above mentioned opioidergic derivative. The obtained microemulsion is completely liquid and isotropic at 25˚C and has the following composition (% by weight):

| | |
|---|---|
| Opioidergic compound | 0.4% |
| Triglyceride (Miglyol® 810) | 0.1% |
| Monoglyceride (Imwitor® 308) | 0.1% |
| Solutol® HS15 | 1.8% |
| Aqueous phase (physiological solution) | 97.6% |

**EXAMPLE 32**

Microemulsion preparation

0.082 g of compound I-Aa obtained in Example 15 have been solubilized in 0.082 g of the triglyceride Miglyol® 812S (Sasol Germany GmbH) a 25˚C. to the obtained oily solution, 0.736 g of the non-ionic surfactant Solutol® HS15 (Basf) and 0.010 g of physiological solution were added, under stirring,. The sample was heated for 5 minutes to 40˚C and then cooled at room temperature. The obtained microemulsion is completely liquid and isotropic at 25˚C and has the following composition (% by weight):

| | |
|---|---|
| Compound I-Aa | 8.2% |
| Oil (Miglyol® 812S) | 8.2% |
| Solutol® HS15 | 73.6% |
| Aqueous phase (physiological solution) | 10.0% |

**EXAMPLE 33**

Evaluation of the analgesic effect of the microemulsions containing the opioidergic compounds

One of the most important therapeutic indications of the compounds agonist of the opioidergic receptors is pain relief. Morphine is the reference compound of this class of opioidergic derivatives and its use is recommended in cases of acute and chronic pain.

To evaluate the therapeutic properties of the present microemulsions in the pain treatment, two behavioural assays have been adopted in animal models, commonly used for the evaluation of the pain threshold in laboratory animals: the Hot Plate test and the Tail Flick test, have been carried out.

Evaluation of the analgesic effect of the compounds by the Hot-Plate test

The analgesic effect has been evaluated in accordance with the procedure indicated by Ruiu S. et al. (J. Pharmacol. Exp. Ther. 306(1) (2003) 363-370), by determining the time (latency in seconds) of response to the pain in mice (male CD1, 20-25 g) placed on a plate maintained at the constant temperature of 55 ± 0.2°C.
In the experiment the following behaviours have been considered as pain signs: leg lifting (forelegs or hindlegs), licking of the same, jumping, etc.
When one of said signs was shown by the animal, it was immediately removed from hot plate, and the relevant latency time registered.
To avoid lesions to leg tissues, a maximum latency time of 14 seconds was fixed, after which the animal was in any case removed from the plate.
The values obtained from the experimentation have been expressed as MPE % (maximum possible effect %), according to the formula:

$$\text{MPE} = \frac{[\text{Latency Test (sec.)} - \text{Basic latency (sec.)}]}{\text{Cut off (sec.)} - \text{Basic Latency (sec.)}} \times 100$$

wherein latency test is the number of seconds elapsed before the appearance of pain signs in the animal undergoing pharmacological treatment. By basic latency it is meant the seconds elapsed before the appearance of pain signs, in the same animal before drug adminisration.
In particular it has been evaluated the analgesic potency of the microemulsions of Examples 20, 21, 23. Said microemulsions have been administered by intraperitoneal route (i.p.) ar the following dosages, referred to the opioidergic compound active principle contained in said microemulsions:

Microemulsion Example 20: 10 mg/kg;
Microemulsion Example 21: 20 mg/kg;
Microemulsion Example 23: 20 mg/kg.

The blanks or references were formed of the same microemulsions without the opioidergic compounds, and by morphine (10 mg/kg), When the blank was morphine, it was solubilized in a microemulsion of the same composition as that under screening.
For evaluating the duration of the analgesic effect, the % MPE values have been registered (average ± SEM) obtained at different times (15, 30, 60, 120, 240, 360 min.) from the administration of the microemulsions and of the references, see Table 3. The values in the Table correspond to the ± SEM average of at least 6 animals for each experimental group.

Evaluation of the analgesic effect of the compounds by the Tail-Flick test

For this study, male CD1 mice weighing 20-25 g have been used. The test consists in evaluating the time elapsed between the exposure of a portion of the mouse tail, put at 2 cm from the tail tip, to a small heat source and the moment when the animal removes the tail from the heat source (Ruiu S. et al. in J. Pharmacol. Exp. Ther.; 306(1) (2003) 363-370). This time interval has been automatically calculated by a specific equipment for the Tail-Flick test (Instrument for Tail Flick, Ugo Basile, Italy), wherein the heat source is an infrared lamp that can be regulated. The equipment automatically records the time during which the mouse tail has remained in contact with the heat source (latency time). To avoid lesions to the tail tissues, it has been fixed a maximum latency time (12 sec), after which the mouse tail was in any case removed from the heat source.
As for the Hot-Plate test, the analgesic potency of the microemulsions of the Examples 20, 21 and 23 has been evaluated. Said microemulsions have been administered by intraperitoneal route (i.p.) at the following dosages, referred to the opioidergic active principle contained in said microemulsions:

Microemulsion Example 20: 10 mg/kg;
Microemulsion Example 21: 20 mg/kg;
Microemulsion Example 23: 20 mg/kg.

The blanks or references were formed of the same microemulsions without the opioidergic compounds, and

by morphine (10 mg/kg). When the blank was morphine, it was solubilized in a microemulsion of the same composition as that under screening.

The response values to the Tail-Flick test have been expressed as % MPE (± SEM average) and the analgesic effect of the compounds has been evaluated at different times (15, 30, 60, 120, 240, 360 min.) from the administration of the microemulsions and of the reference compositions (Table 4). The values in the Table correspond to the ± SEM average of at least 6 animals for each experimental group.

Results

The results obtained in the Hot Plate and Tail Flick tests are reported respectively in Tables 3 and 4. The Tables show that the microemulsions containing the opioidergic active principles of the present invention significantly increase the pain threshold. As a matter of fact the MPE % values obtained within 120 minutes from the administration are notably higher than those of the corresponding carriers. Therefore the microemulsions containing the above described opioidergic active principles of the present invention show analgesic properties.

At 30 minutes from the administration, the analgesic effect resulted equal to or higher than that of the morphine. Besides, with reference in particular to the administered dose of 20 mg/kg, it has been noticed that the analgesic effect is maintained in the time.

In the Tables, by the expression "carriers of the microemulsion" it is meant the microemulsion composition without the active ingredient.

TABLE 3

| Evaluation of the analgesic effect by the Hot Plate Test | | | | | |
|---|---|---|---|---|---|
| | MPE% | | | | |
| | 30 min | 60 min | 120 min | 240 min | 360 min |
| Carrier of the microemulsion of Example 20 | 8.8±1.5 | -2.7±4.0 | 4.8±2.0 | 6.0±3.0 | 3.2±2.7 |
| Morphine in the carrier of the microemulsion of Example 20 (10mg/kg) | 41.1±9.1 | 56.5±8.4 | 30.6±4.9 | 23.0±4.3 | 9.5±4.4 |
| Microemulsion of Example 20 (opioid 10 mg/kg) | 53.5±13.5 | 28.1±7.8 | 6.9±3.9 | 3.7±3.0 | 4.6±1.3 |
| Carrier of the microemulsions of Examples 21 and 23 | 8.0±1.5 | 2.0±1.0 | 1.8±0.9 | 3.0±1.0 | 3.0±2.0 |
| Morphine in the carrier of the microemulsions of Examples 21 and 23 (10mg/kg) | 48.3±14.6 | 53.2±16.7 | 37.5±8.5 | 7.6±8.6 | 6.8±13.8 |
| Microemulsion of 'Example 21 (opioid 20 mg/kg) | 59.2±13.5 | 44.9±9.8 | 26.9±10.3 | 5.6±4.6 | 0.4±2.1 |
| Microemulsion of 'Example 23 (opioid 20 mg/kg) | 75.9±9.2 | 53.5±16.3 | 7.1±2.8 | 7.0±3.0 | -3.2±2.7 |

TABLE 4

| Evaluation of the analgesic effect by the Tail Flick test | | | | | |
|---|---|---|---|---|---|
| | MPE% | | | | |
| | 30 min | 60 min | 120 min | 240 min | 360 min |
| Carrier of the microemulsion of Example 20 | 2.5±1.9 | 4.9±4.7 | -2.1±3.5 | 9.5±1.9 | -4.9±3.5 |
| Morphine in the carrier of the microemulsion of Example 20 (10mg/kg) | 57.2±10.9 | 65.7±8.4 | 42.2±12.4 | 21.6±7.0 | 2.3±3.2 |
| Microemulsion of Example 20 (opioid 10 mg/kg) | 69.5±13.1 | 21.2±6.3 | 13.1±4.5 | 4.1±4.5 | -2.8±3.1 |
| Carrier of the microemulsions of the Examples 21 and 23 | 2.8±1.3 | 3.9±2.7 | -1.1±2.0 | 6.0±1.8 | 2.9±1.5 |

(continued)

| Evaluation of the analgesic effect by the Tail Flick test | | | | | |
|---|---|---|---|---|---|
| | MPE% | | | | |
| | 30 min | 60 min | 120 min | 240 min | 360 min |
| Morfphine in the carrier of the microemulsions of Examples 21 and 23 (10mg/kg) | 54.3±19.6 | 71.4±9.7 | 38.9±10.9 | 1.6±5.0 | -1.3±2.2 |
| Microemulsion of Example 21 (opioid 20 mg/kg) | 85.2±8.1 | 55.2±10.5 | 47.9±13.3 | 18.1±12.6 | -4.0±3.0 |
| Microemulsion of Example 23 (opioid 20 mg/kg) | 91.9±5.5 | 92.2±7.8 | 10.4±6.4 | 3.0±2.7 | -1.4±1.0 |

**EXAMPLE 34**

Microemulsion preparation

5 mg of the compound I-Ba (active principle) obtained in Example 16 were solubilized at 70°C in 65 mg of stearic acid (oil). At complete dissolution of the active principle in the stearic acid, 280 mg of sodium taurocholate (surfactant and 650 mg of distilled water have been added. The final sample, obtained at 70°C, is completely limpid, liquid and isotropic. The final composition (% by weight) of the obtained microemulsion is the following:

| | |
|---|---|
| Stearic acid (oil): | 6.5% |
| Sodium taurocholate (surfactant) | 28% |
| Water: | 65% |
| Active principe: | 0.5% |

**EXAMPLE 35**

Microemulsion preparation

5 mg of the compound I-Aa (active principle) obtained in Example 15 were solubilized at 70°C in 95 mg of stearic acid (oil). At complete dissolution of the active principle in the oil, 0.1875 g of sodium taurocholate and 0.0625 g of the commercial sample of soya lecithin Epikuron® 200 (surfactants) and 650 mg of distilled water have been added.

The final sample, obtained at 70°C, appears completely limpid, liquid and isotropic. The final composition (% by weight) of the obtained microemulsion is the following:

| | |
|---|---|
| Stearic acid (oil): | 9.5% |
| Sodium taurocholate (surfactant): | 18.75% |
| Lecithin (surfactant): | 6.25% |
| Water: | 65% |
| Active principle: | 0.5% |

**EXAMPLE 36**

Microemulsion preparation

5 mg of compound I-Aa (active principle) obtained in Example 15 were solubilized at 70°C in 15 mg of Dynasan® 116 (tripalmitine). 190 mg of n-butanol, 63 mg of soya lecithin (Epikuron® 200), 127 mg of sodium taurocholate and 600 mg of physiological solution were added to the oily solution obtained at 70°C.

The final sample obtained at 70°C is completely limpid, liquid and isotropic. The final composition (% by weight) of the obtained microemulsion is the following:

| | |
|---|---|
| Tripalmitine (oil): | 1.5% |
| Sodium taurocholate (surfactant) | 6.3% |

(continued)

| | |
|---|---|
| Lecithin (surfactant): | 12.7% |
| n-butanol: | 19% |
| Physiological solution (aqueous phase): | 60% |
| Active principle: | 0.5% |

**EXAMPLE 37**

Microemulsion preparation

5 mg of compound I-Aa (active principle) obtained in Example 15 were solubilized at 70˚C in 9 mg of Miglyol® 812S and 36 mg of tripalmitine. 175 mg of n-butanol, 140 mg of soya lecithin (Epikuron® 200), 35 mg of sodium taurocholate and 600 mg of physiological solution were then added to the obtained oily solution.

The final sample obtained at 70˚C is completely limpid, liquid and isotropic. The final composition (% by weight) of the obtained microemulsion is the following:

| | |
|---|---|
| Tripalmitine (oil): | 3.6% |
| Miglyol® 812S (olio): | 0.9% |
| Sodium taurocholate (surfactant) | 3.5% |
| Lecithin (surfactant): | 14% |
| n-butanol: | 17.5% |
| Physiological solution (aqueous phase): | 60% |
| Active principle: | 0.5% |

**EXAMPLE 38**

The microemulsion of Example 20 has been heated to 75˚C in a closed vessel for 15 minutes. Phase separation between the aqueous phase and the oily phase has been observed. The sample was then stirred and cooled at 25˚C, restoring the initial microemulsion.

**EXAMPLE 39 Comparative**

The preparation of the microemulsion described in Example 20 has been repeated, but not using the oil Miglyol® 812S. The ratios by weight among the other components were maintained equal to those of Example 20. The ratio between the surfactant (Solutol® HS15) and the active principle (compound Ex. I-Aa) is equal to 9.00. At the end of the preparation it was not obtained a microemulsion but an heterogeneous composition, formed of an opalescent liquid phase containing a solid residue.

The aspect of the composition did not change by diluting the obtained composition with physiological solution so as to obtain the same concentration of compound I-Aa of the microemulsion of Example 20 (0.2%). The composition aspect remains unaltered even by increasing the ratio by weight surfactant/compound I-Aa from 9 to 20 by addition of Solutol® HS15.

Therefore, in order to obtain a microemulsion in the absence of component O), the ratio component PA/components) in this example should be further increased above 20. In Example 20, as reported above, said ratio was of 9,00.

**EXAMPLE 40 Comparative**

The preparation of the microemulsion described in Example 22 was repeated, but without using the Miglyol® 810 and Imwitor® 308 oils. The ratios by weight among the other components were maintained equal to those of Example 22. The ratio between the surfactant (Solutol® HS15) and the active principle (compound Ex. I-Ba) is 9.00. At the end of the preparation an heterogeneous composition was obtained that, as in Example 39 comparative, was constituted of an opalescent liquid phase containing a solid residue.

The aspect of the composition did not change by diluting the obtained composition with physiological solution to obtain a concentration of compound I-Ba equal to that of the microemulsion of Example 22 (0.2%). Even by increasing the ratio by weight surfactant/compound I-Ba from 9 to the double (18) by addition of Solutol® HS15 the situation did

not change.

**EXAMPLE 41 Comparative**

The preparation of the microemulsion described in Example 27 was repeated, but without using the Miglyol® 812S oil. The ratios by weight among the other components were maintained equal to those of Example 27. The ratio between the surfactant (Solutol® HS15)/active principle (opioidergic compound) is 18.00. At the end of the preparation an heterogeneous composition is obtained, formed of an opalescent liquid phase containing a solid body.

The aspect of the composition did not change by diluting with physiological solution to obtain a concentration of the opioidergic compound equal to that of the microemulsion of Example 27 (0.1 %). Even by increasing the ratio by weight surfactant/opioidergic compound from 18 to 30 by addition of Solutol® HS15, the situation did not change.

**EXAMPLE 42 Comparative**

The preparation of the microemulsion described in Example 28 was repeated, but without using the Miglyol® 812S oil. The ratios by weight among the other components were maintained equal to those of Example 29. The ratio between the surfactant (Solutol® HS15)/active principle (opioidergic compound) is 9.00. At the end of the preparation an heterogeneous composition is obtained, formed of an opalescent liquid phase containing a solid residue.

The aspect of the composition did not change by diluting with physiological solution to obtain the same concentration of the opioidergic compound of the microemulsion of Example 28 (0.2%). The increase of the ratio by weight surfactant/opioidergic compound from 8 to 20 by addition of Solutol® HS15, did not affect the composition, that remained heterogeneous.

**EXAMPLE 43 Comparative**

The preparation of the microemulsion of Example 29 was repeated, but without using the Miglyol® 812S oil.

The ratios by weight among the other components were maintained equal to those of Example 29. The ratio between the surfactant (Solutol® HS15)/active principle (opioidergic compound) is 18.00. At the end of the preparation an heterogeneous composition is obtained, formed of an opalescent liquid phase containing a solid residue.

The aspect of the composition did not change by diluting with physiological solution to obtain the same concentration of the opioidergic compound of the microemulsion of Example 29 (0.1%). The aspect remains unaltered even by increasing the ratio by weight surfactant/opioidergic compound from 18 to 25 by addition of Solutol® HS15.

## Claims

**1.** Pharmaceutical compositions in the form of microemulsions comprising the following components, in amounts expressed as % by weight:

S) from 0.01 to 95% of one or more pharmaceutically acceptable compounds, selected from the following classes:

- surfactants, selected from non-ionic, anionic, cationic and amphoteric, optionally containing fluorine atoms,
- polymers forming organized structures, as aggregates, micelles, liquid crystals, vesicles, in the liquid in which they are solubilized;

O) from 0.01 to 95% of one or more oils selected from the following classes of pharmaceutically acceptable compounds:

- $C_4$-$C_{32}$ acid esters, optionally containing one or more unsaturations of ethylenic type,
- $C_4$-$C_{32}$ acids, optionally containing one or more unsaturations of ethylenic type,

PA) from 0.001 to 90% of diazabicyclic compounds having formula A':

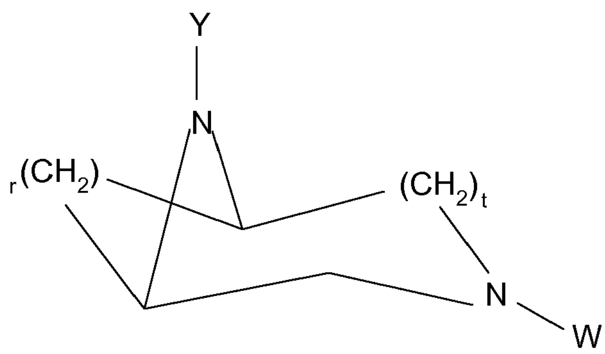

A'

wherein:

t is an integer equal to 1 or 2,
r is an integer equal to 1, 2 or 3 and has the following values, depending on t:

- r= 1, 2 or 3 when t=1,
- r= 2, 3 when t=2,

when t=1, one between the substituents W, Y of the nitrogen atoms of the diazabicyclic ring is an acyl group -C(O)-$R_B$, with $R_B$ = $C_1$-$C_{10}$ alkyl group, linear or branched when possible, the other substituent is selected from:

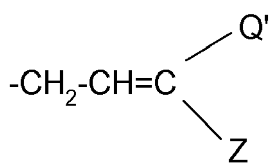

(XIV)

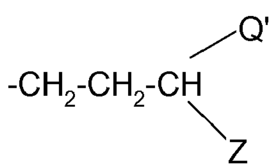

(XV)

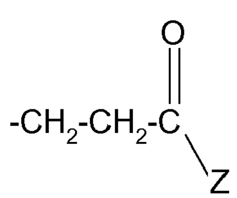

(XVI)

wherein:

Z is selected from:

- $C_6$-$C_{10}$ aryl,

- $C_5$-$C_7$ cycloalkyl,
- aromatic heterocycle with a 5 or 6 atom ring, containing at least one heteroatom selected from nitrogen, oxygen, sulphur,

Q' is selected among: hydrogen, $C_1$-$C_4$ alkyl, linear or branched when possible, $C_5$-$C_7$ cycloalkyl or phenyl,

when t=2, one of the substituents W, Y is an acyl group -C(O)-$R_B$, $R_B$ being as defined above; the other substituent is selected from:

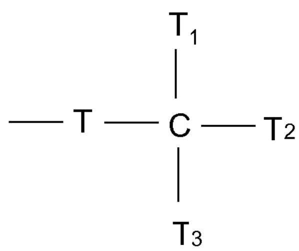
(XVII)

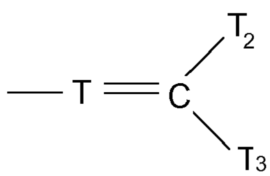
(XVIII)

-T≡C-$T_4$ (XIX)

wherein:

- T is a saturated or unsaturated $C_2$-$C_9$ aliphatic chain, linear or branched when possible,
- $T_1$ is selected from: hydrogen, isothiocyanate, CN, OR', C(O)OR', C(O)R', C(O)NR'R", NR'R", wherein R' and R", equal to or different from each other, are selected from hydrogen, $C_1$-$C_7$ alkyl, $C_1$-$C_7$ alkoxy, $C_1$-$C_7$ alkylthio, $C_1$-$C_7$ haloalkyl, $C_1$-$C_7$ haloalkoxy, linear or branched when possible, $C_3$-$C_{15}$ cycloalkyl, aryl, heteroaryl,
- $T_2$ and $T_3$, equal to or different from each other, are selected from:

  - hydrogen, with the following provisos:

    - when one substituent between W, Y has formula (XVII), at least one of $T_1$, $T_2$ and $T_3$ is not hydrogen,
    - when one substituent between W, Y has formula (XVIII), at least one of $T_2$ and $T_3$ is not hydrogen,
    - linear or branched when possible $C_1$-$C_{10}$ alkyl,
    - aryl or heteroaryl,
    - $C_3$-$C_{15}$ cycloalkyl,

  - $T_4$ has the same meanings as $T_2$ and $T_3$ but excluding hydrogen,

AD) from 0 to 60% by weight of one or more compounds selected from the following classes:

- modifiers of water and/or oil polarity,
- modifiers of the curvature of the film of component S),
- co-surfactants,

WA) from 0.01 to 99.9% of water or a saline aqueous solution, optionally buffered,

the sum of the components being 100%,
wherein the ratio by weight S)/PA) is lower than at least 10%, preferably lower than at least 30%, with respect to the ratio by weight S)/PA) of microemulsions having the same composition but not including component O).

**2.** Compositions according to claim 1, wherein the liquids in which the polymers component S) are solubilized, are water and/or oil.

**3.** Compositions according to claims 1-2, wherein $R_B$ of component PA) optionally contains $C_3$-$C_{10}$ cycloalkyl rings or $C_6$-$C_{10}$ aryl rings, or saturated or unsaturated $C_3$-$C_{10}$ heterocyclic rings containing one or more heteroatoms, preferably selected from N, S and O.

**4.** Compositions according to claims 1-3, wherein, when Z is a $C_6$-$C_{10}$ aryl or an aromatic heterocycle having a 5 or 6 atom ring, said aryl and aromatic heterocycle are optionally substituted with one or more groups, equal to or different from each other, selected from: $C_1$-$C_3$ alkoxy, $C_1$-$C_2$ haloalkoxy, $C_1$-$C_3$ alkyl, halogen, carboxy, cyano, nitro, -CONHZ', wherein Z' is $C_1$-$C_4$ alkyl, linear or branched when possible.

**5.** Compositions according to claims 1-4, wherein, when Z is an aromatic heterocycle having a 5 or 6 atom ring optionally fused with a benzene ring.

**6.** Compositions according to claims 1-5, wherein Q' is $C_5$-$C_7$ cycloalkyl or phenyl and said groups are optionally substituted with one or more groups, equal to or different from each other, selected from: $C_1$-$C_3$ alkoxy, $C_1$-$C_2$ haloalkoxy, $C_1$-$C_3$ alkyl, halogen, carboxy, cyano, nitro, -CONHZ', wherein Z' has the meaning of $C_1$-$C_4$ alkyl, linear or branched when possible.

**7.** Compositions according to claims 1-6, wherein $T_1$ is OR', C(O)OR', C(O)R', C(O)NR'R", NR'R", R' and/or R" being $C_3$-$C_{15}$ cycloalkyl, the cycloalkyl optionally containing one or more heteroatoms, preferably selected from O, S, N.

**8.** Composition according to claims 1-7, wherein $T_1$ is OR', C(O)OR', C(O)R', C(O)NR'R", NR'R", R' and/or R" are selected from $C_3$-$C_{15}$ cycloalkyl, optionally containing one heteroatom selected from 0,5 ml, aryl and heteroaryl, these groups are optionally substituted with one or more groups selected from hydroxy, halogen, linear or branched when possible $C_1$-$C_{10}$ alky.

**9.** Compositions according to claims 1-8, wherein $T_2$ and $T_3$ are $C_1$-$C_{10}$ alkyl, linear or branched, optionally substituted with one or more groups, equal to or different from each other, selected from hydroxy, halogen, CN, $C_3$-$C_{15}$ cycloalkyl, said cycloalkyl optionally containing one or more heteroatoms, preferably selected from O, S and N, aryl or heteroaryl, wherein each of the cycloalkyl, aryl and heteroaryl is optionally substituted with one or more groups selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

**10.** Compositions according to claims 1-9, wherein $T_2$ and $T_3$ have the meaning of aryl or heteroaryl and each of the aryl or heteroaryl is optionally substituted with one or more of the following groups, equal to or different from each other, selected from:

- $C_1$-$C_{10}$ alkyl, linear or branched when possible, optionally substituted with one or more groups, equal to or different from each other, selected from hydroxy, halogen, CN;
- $C_3$-$C_{15}$ cycloalkyl, optionally containing one or more heteroatoms, preferably selected from O, S, N, said cycloalkyl optionally substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy,
- alkylaryl or aryl or heteroaryl or alkylheteroaryl, that are optionally substituted with one or more groups, equal to or different from each other, selected from hydroxy, halogen, linear or when possible branched $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

**11.** Compositions according to claims 1-10, wherein $T_2$ and $T_3$ have the meaning of $C_3$-$C_{15}$ cycloalkyl, said cycloalkyl optionally containing one or more heteroatoms, preferably selected from O, S, N, and is optionally substituted with one or more of the following groups, equal to or different from each other, selected from:

- linear or branched when possible $C_1$-$C_{10}$ alkyl, , optionally substituted with one or more groups, equal to or

different from each other, selected from hydroxy, halogen, CN,
- $C_3$-$C_{15}$ cycloalkyl, optionally containing one or more heteroatoms, preferably selected from O, S, N, said cycloalkyl optionally substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy,
- arylalkyl, aryl, heteroaryl, heteroarylalkyl, optionally substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

**12.** Compositions according to claims 1-11, wherein one of the W, Y substituents of compounds PA) is selected from $-C(O)-CH_3$ or $-C(O)-C_2H_5$, and the other substituent:

- when t=1, is selected from (XIV) or (XV),
- when t=2, is selected from (XVII) wherein $T_1$=H, or (XVIII).

**13.** Compositions according to claims 1-12, wherein one of the W, Y substituents of compounds PA) is selected from $-C(O)-CH_3$ or $-C(O)-C_2H_5$, and the other substituent:

- when t=1, is selected from (XIV) or (XV) wherein at least one of Q', Z has the meaning of aryl,
- when t=2, is selected from (XVII) wherein $T_1$=H, or (XVIII), wherein at least one of $T_2$, $T_3$ is aryl.

**14.** Compositions according to claims 1-13, comprising the following components in % by weight:

- component S) from 0.01 to 60%,
- component O) from 0.01 to 90%,
- component PA) from 0.001 to 50%,
- component AD) from 0 to 20%,
- component WA) from 0.1 to 99,9%,

the sum of the components being 100%.

**15.** Compositions according to claims 1-13 comprising the following components in % by weight:

- component S) from 0.01 to 50%,
- component O) from 0.01 to 90%,
- component PA) from 0.05 to 50%,
- component AD) from 0 to 10%,
- component WA) from 10 to 99,9%,

the sum of the components being 100%.

**16.** Compositions according to claims 1-13 comprising the following components in % by weight:

- component S) from 0.01 to 50%,
- component O) from 0.01 to 50%,
- component PA) from 0.05 to 50%,
- component AD) from 0 to 10%,
- component WA) from 20 to 99.9%,

the sum of the components being 100%.

**17.** Compositions according to claims 1-16, wherein the surfactants component S) are selected from non ionic and anionic surfactants.

**18.** Compositions according to claims 1-17, wherein in component AD):

- the modifiers of the water and/oil polarity are polyethylenglycols and aliphatic alcohols,
- the modifiers of the curvature of the film of the component S) are $C_2$-$C_5$ aliphatic alcohols,

- the co-surfactants are aliphatic alcohols, preferably having at least 6 carbon atoms.

**19.** Compositions according to claims 1-18 diluted with water and/or with aqueous solutions and/or with oil to obtain a pharmaceutically effective concentration of the active principle.

**20.** A process for preparing the compositions of claims 1-19, comprising the following steps:

(IP) solubilization of the diazabicyclic compound of formula A' in oil,
(IIP) addition of component S) to the solution in oil,
(IIIP) optional addition of component AD) to the oily phase,
(IVP) addition of water or of saline solution to the oily phase, obtaining a limpid solution.

**21.** A process according to claim 20, comprising the following steps:

(I') solubilization of the diazabicyclic compound of formula A' in oil,
(II') addition of component S) to the aqueous phase,
(III') optional addition of component AD) to the aqueous phase,
(IV') mixing of the oily phase of step (I') with the aqueous phase of step (II') or optionally with (III').

**22.** A process according to claims 20-21, carried out at temperatures in the range 0-80°C.

**23.** Homopiperazine-comprising nonane and decane diazabicyclic derivatives with affinity for the opioidergic μ and/or δ and/or k receptors and/or for all their receptorial subclasses, and activity on the central and/or the peripheral system, having formula (I), including the isomeric forms and their mixtures, wherein the ring atoms are optionally in different isotopic forms:

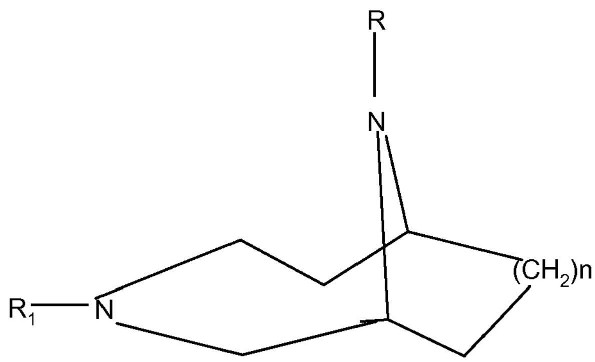

(I)

wherein:

- n is an integer equal to 1 or 2;
- one of R, $R_1$ substituents of the nitrogen atoms of the diazabicyclic ring is a -C(O)-$R_B$ group, $R_B$ = $C_1$-$C_{10}$ alkyl group, linear or branched when possible,
- the other substituent is selected from the following groups (II)-(X):

structure (II):

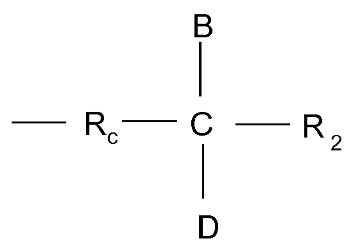

(II)

wherein:

- $R_c$ is a bivalent saturated aliphatic chain $C_3$-$C_{10}$, linear or branched when possible,
- B is a group selected from hydrogen, isothiocyanate, CN, OR', C(O)OR', C(O)R', C(O)NR'R", NR'R", wherein R' and R", equal to or different from each other, are selected from hydrogen, linear or branched when possible $C_1$-$C_7$ alkyl, $C_1$-$C_7$ alkoxy, $C_1$-$C_7$ alkylthio, $C_1$-$C_7$ haloalkyl, $C_1$-$C_7$ haloalkoxy, $C_3$-$C_{15}$ cycloalkyl, aryl or heteroaryl,
- D and $R_2$, equal to or different from each other, are selected from:

    - hydrogen, with the proviso that in formula (II) at least one substituent among B, D and $R_2$ is not hydrogen;
    - $C_1$-$C_{10}$ alkyl, linear or branched when possible,
    - aryl or heteroaryl,
    - $C_3$-$C_{15}$ cycloalkyl,

formula (III):

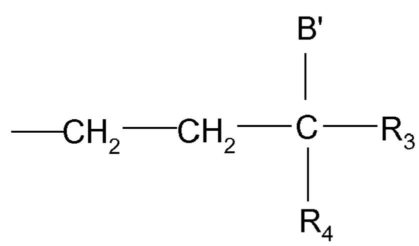

(III)

wherein:

- B' has the same meanings as B,
- $R_3$ is hydrogen, or alkyl, aryl, heteroaryl or cycloalkyl as defined in $R_2$,
- $R_4$ has the following meanings:

    - $C_1$-$C_{10}$ alkyl, linear or branched when possible,
    - $C_3$-$C_{15}$ cycloalkyl, optionally containing one or more heteroaroms, preferably selected from O, S, N,

when B' and $R_3$ are not both hydrogen, $R_4$ has the further meaning of aryl or heteroaryl,
formula (IV):

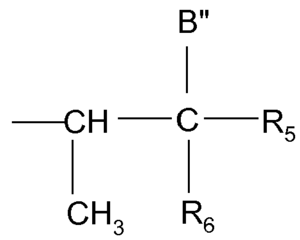

(IV)

wherein:

- B" has the same meaning as B',
- $R_5$ has the same meaning as $R_3$,
- $R_6$ has the same meaning as $R_4$, or is aryl or heteroaryl,

formula(V):

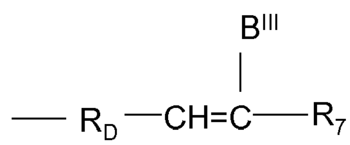

(V)

wherein:

- $R_D$ is a bivalent saturated or unsaturated aliphatic chain $C_2$-$C_8$, linear or branched when possible,
- $B^{III}$ and $R_7$, equal to or different from each other, have the same meanings as $R_2$, with the proviso that $B^{III}$ and $R_7$ are not both hydrogen;

formula (VI):

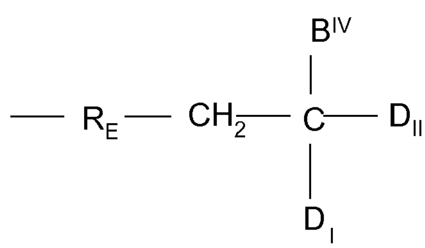

(VI)

wherein:

- $R_E$ is an unsaturated bivalent aliphatic chain $C_2$-$C_8$, linear or branched when possible;
- $B^{IV}$ has the same meaning of B as defined above;
- $D_I$ and $D_{II}$ have the same meanings as $R_2$, with the proviso that in formula (VI) at least one among $B^{IV}$, $D_{II}$ and $D_I$ is different from hydrogen;

formula (VII):

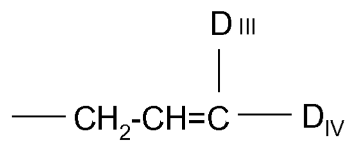

(VII)

wherein:

- $D_{III}$ and $D_{IV}$, equal to or different from each other, have the same meanings as $R_2$ but excluding hydrogen;

formula (VIII):

$-CH_2-CH=CH- D_v$ (VIII)

wherein $D_V$ has the same meanings as $R_4$ but excluding aryl or heteroaryl;
formula (IX):

$-R_F-C \equiv C-R_8$ (IX)

wherein $R_F$ is a saturated or unsaturated bivalent aliphatic chain $C_2$-$C_8$, linear or branched when possible and $R_8$ has the same meanings as $R_2$ but excluding hydrogen;
formula (X):

$-CH_2-C \equiv C-R_9$ (X)

wherein $R_9$ has the same meanings as $R_4$ but excluding aryl or heteroaryl.

**24.** Compounds according to claim 23, wherein B in formula (II) is selected from OR', C(O)OR', C(O)R', C(O)NR'R", NR'R", wherein R' and/or R" are selected from $C_3$-$C_{15}$ cycloalkyl optionally containing one or more heteroatoms preferably selected from O, S or N, aryl and heteroaryl, each of cycloalkyl, aryl and heteroaryl is optionally substituted with one or more groups selected from hydroxy, halogen, linear o branched when possible $C_1$-$C_{10}$ alkyl.

**25.** Compounds according to claims 23-24, wherein D and/or $R_2$ in formula (II) are $C_1$-$C_{10}$ alkyl, linear or branched when possible, the alkyl optionally substituted with one or more groups, equal to or different from each other, selected from hydroxy, halogen, CN, $C_3$-$C_{15}$ cycloalkyl, aryl, heteroaryl, wherein:

- $C_3$-$C_{15}$ cycloalkyl optionally contains one or more heteroatoms, preferably selected from O, S, N,
- $C_3$-$C_{15}$ cycloalkyl, aryl and heteroaryl are optionally substituted with one or more groups selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

**26.** Compounds according to claims 23-25 wherein, when D and/or $R_2$ of formula (II) are $C_3$-$C_{15}$ cycloalkyl, optionally containing one or more heteroatoms, preferably selected from O, S and N, or when D and/or $R_2$ of formula (II) and/or $R_6$ of formula (IV) are aryl, heteroaryl, each of aryl, heteroaryl and cycloalkyl is optionally substituted with one or more of the following, equal to or different from each other, selected from:

- linear or branched when possible $C_1$-$C_{10}$ alkyl, optionally substituted with one or more groups, equal to or different from each other, selected from hydroxy, halogen, CN,
- $C_3$-$C_{15}$ cycloalkyl optionally containing one or more heteroatoms, preferably selected from O, S, N, the cycloalkyl optionally substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy,

- arylalkyl or aryl or heteroaryl or heteroarylalkyl optionally substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

**27.** Compounds according to claims 23-26, wherein in formula (III) $R_4$ is linear or branched when possible $C_1$-$C_{10}$ alkyl, optionally substituted with one or more groups, equal to or different from each other, selected from hydroxy, halogen, CN, aryl, heteroaryl or $C_3$-$C_{15}$ cycloalkyl optionally containing one or more heteroatoms preferably selected from O, S, N, wherein each of cycloalkyl, aryl or heteroaryl is optionally substituted with one or more groups equal to or different from each other selected from hydroxy, halogen, $C_1$-$C_{10}$ alkyl, linear or branched when possible, arylalkyl, aryl, heteroalkyl, heteroarylalkyl, each of arylalkyl, aryl, heteroaryl, heteroarylalkyl being optionally substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

**28.** Compounds according to claims 23-27, wherein in formula (III) when $R_4$ has the meaning of $C_3$-$C_{15}$ cycloalkyl, the cycloalkyl is optionally substituted with one or more of the following groups, equal to or different from each other:

- $C_1$-$C_{10}$ alkyl, linear or branched when possible, optionally substituted with one or more groups, equal to or different from each other, selected from the following: hydroxy, halogen, CN,
- $C_3$-$C_{15}$ cycloalkyl, optionally containing one or more heteroatoms, equal to or different from each other preferably selected from O, S, N, said cycloalkyl optionally substituted with one or more of the following groups, equal to or different from each other: hydroxy, halogen, $C_1$-$C_{10}$ alkyl, linear or branched when possible, arylalkyl, aryl, heteroaryl, heteroarylalkyl, said arylakyl, aryl, heteroaryl, heteroarylalkyl being optionally substituted with one or more groups, equal to or different from each other, selected from: hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy,
- arylalkyl or aryl or heteroaryl or heteroarylalkyl, optionally substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

**29.** Compounds according to claims 23-28, wherein in formula (III) B' and $R_3$ are not both hydrogen and $R_4$ is aryl or heteroaryl, each of aryl and heteroaryl being optionally substituted with one or more of the following groups, equal to or different from each other:

- $C_1$-$C_{10}$ alkyl, linear or branched when possible, optionally substituted with one or more groups, equal to or different from each other, selected from the following: hydroxy, halogen, CN,
- $C_3$-$C_{15}$ cycloalkyl, optionally containing one or more heteroatoms, equal to or different from each other, preferably selected from O, S, N, the cycloalkyl is optionally substituted with one or more of the following groups, equal to or different from each other: hydroxy, halogen, $C_1$-$C_{10}$ alkyl, linear or branched when possible, arylalkyl, aryl, heteroaryl, heteroarylalkyl, the arylalkyl, aryl, heteroaryl, heteroarylalkyl being optionally substituted with one or more groups, equal to or different from each other, selected from: hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy,
- arylalkyl, aryl, heteroaryl, heteroarylalkyl, wherein each of said groups are optionally substituted with one or more groups, equal to or different from each other, selected from hydroxy, halogen, linear or branched when possible $C_1$-$C_{10}$ alkyl, $C_1$-$C_7$ alkoxy, $C_1$-$C_7$ alkylthio, $C_1$-$C_7$ haloalkyl, $C_1$-$C_7$ haloalkoxy.

**30.** Compounds according to claims 23-29, wherein one between R, $R_1$ is a -C(O)-$R_B$ group and the other is selected from the groups (II) - (X) wherein:

- in formula (II) $R_c$ is a bivalent saturated aliphatic chain $C_3$-$C_7$, linear or branched when possible, B is hydrogen,
- in formula (III) B' is hydrogen, $R_3$ is a substituent selected from alkyl, aryl, heteroaryl or cycloalkyl as defined in $R_2$, $R_4$ is as above defined including aryl and heteroaryl,
- in formula (IV) B" is hydrogen,
- in formula (V) $R_D$ is a saturated or unsaturated bivalent aliphatic chain $C_2$-$C_5$, linear or branched when possible, $B^{III}$ and $R_7$, equal to or different from each other, have the same meaning of $R_2$ with the proviso that $B^{III}$ and $R_7$ are not both hydrogen,
- in formula (VI) $R_E$ is an unsaturated bivalent aliphatic chain $C_2$-$C_5$, linear or branched when possible, $B^{IV}$ is hydrogen, $D_{II}$ and $D_I$ have the same meaning of $R_2$, with the proviso that in formula (VI) at least one of $D_{II}$, $D_I$ is different from hydrogen,

formulae (VII) to (X) are as defined above.

**31.** Compounds according to claims 23-29, wherein one of R, $R_1$ is -C(O)-$R_B$, $R_B$ being a $C_1$-$C_4$ alkyl group, linear or branched when possible, the other is selected from formulae (II) to (X), wherein:

- in formula (II) $R_c$ is a bivalent saturated aliphatic chain $C_3$-$C_7$, linear or branched when possible, B is hydrogen,
- in formula (III) B' is hydrogen, $R_3$ is a substituent selected from alkyl, aryl, heteroaryl or cycloalkyl as defined in $R_2$, $R_4$ is as above defined including aryl and heteroaryl,
- in formula (IV) B" is hydrogen,
- in formula (V) $R_D$ is a saturated or unsaturated bivalent aliphatic chain $C_2$-$C_5$, linear or branched when possible, $B^{III}$ and $R_7$, equal to or different from each other, have the same meaning of $R_2$, with the proviso that $B^{III}$ and $R_7$ are not both hydrogen,
- in formula (VI) $R_E$ is an unsaturated bivalent aliphatic chain $C_2$-$C_5$, linear or branched when possible, $B^{IV}$ is hydrogen, $D_{II}$ and $D_I$ have the same meanings as $R_2$, with the proviso that in formula (VI) at least one substituent between $D_{II}$ and $D_I$ is different from hydrogen,

formulae from (VII) to (X) are as defined above.

**32.** Compounds according to claims 23-29, wherein in formula (I) one between R, $R_1$ is -C(O)-$R_B$, $R_B$ being selected from methyl, ethyl, the other selected from formulae (II)-(X) wherein:

- in formula (II) $R_c$ is a bivalent saturated alkyl chain $C_3$, linear or branched when possible, B is hydrogen,
- in formula (III) B' is hydrogen, $R_3$ is a substituent selected from alkyl, aryl, heteroaryl or cycloalkyl as defined in $R_2$, $R_4$ is as above defined including aryl and heteroaryl,
- in formula (IV) B" is hydrogen,
- in formula (V) $R_D$ is a saturated or unsaturated bivalent aliphatic chain $C_2$-$C_5$, linear or branched when possible, $B^{III}$ and $R_7$, equal to or different from each other, have the same meanings as $R_2$, with the proviso that $B^{III}$ and $R_7$ are not both hydrogen,
- in formula (VI) $R_E$ is an unsaturated bivalent aliphatic chain $C_3$, linear or branched when possible, $B^{IV}$ is hydrogen, $D_{II}$ and $D_I$ have the same meanings as $R_2$, with the proviso that in formula (VI) at least one of $D_{II}$, $D_I$ is different from hydrogen,

formulae (VII) to (X) being as defined above.

**33.** Compounds according to claims 23-29, wherein in formula (I) one among R, $R_1$ is -C(O)-$R_B$, $R_B$ being selected from methyl, ethyl, the other is selected from the following formulae:

- formula (III) wherein B' is hydrogen, $R_3$ is selected from alkyl, aryl, heteroaryl or cycloalkyl as defined in $R_2$, $R_4$ is as above defined including aryl and heteroaryl,
- formula (VII) wherein $D_{III}$ and $D_{IV}$, equal to or different from each other, have the same meanings as $R_2$ but excluding hydrogen,
- formula (VIII) wherein $D_V$ has the same meanings as $R_4$ but excluding aryl or heteroaryl,
- formula (X) wherein $R_9$ has the same meanings as $R_4$ but excluding aryl or heteroaryl.

**34.** Compounds according to claims 23-33, having the following formulae:

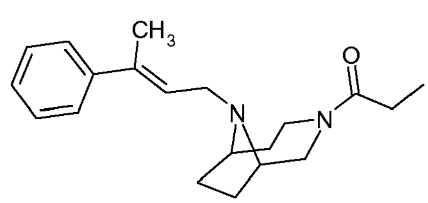

(XX)

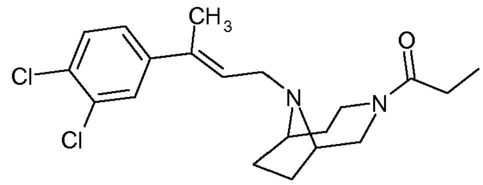

(XXI)

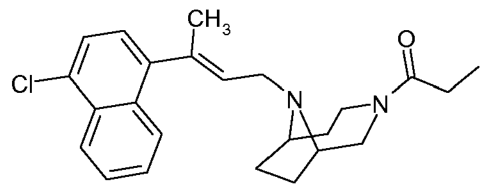

(XXII)

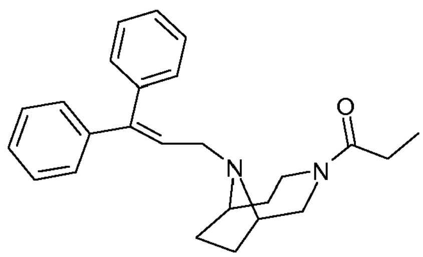

(XXIII)

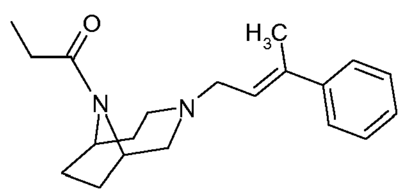

(XXIV)

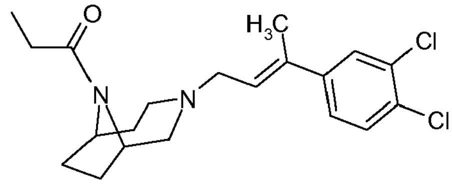

(XXV)

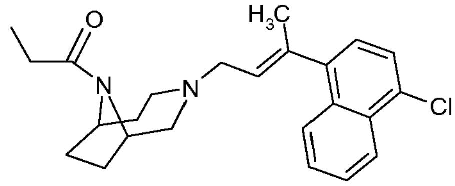

(XXVI)

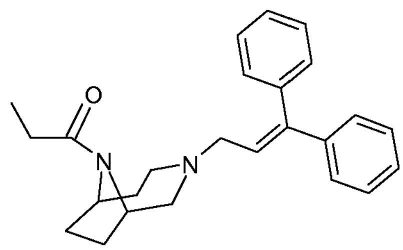

(XXVII)

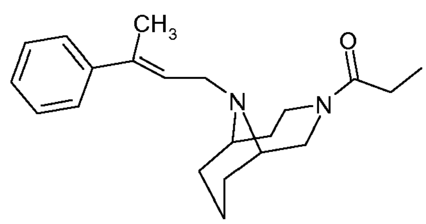

(XXVIII)

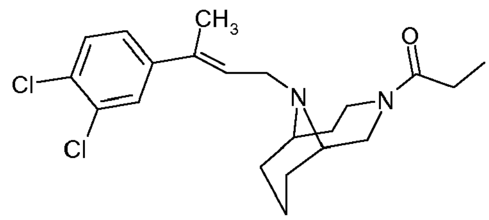

(XXIX)

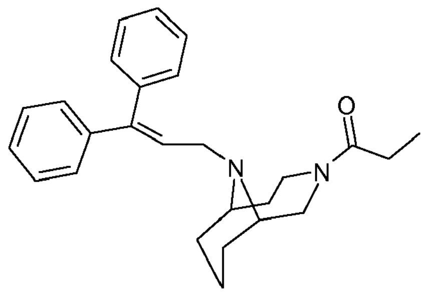

(XXX)

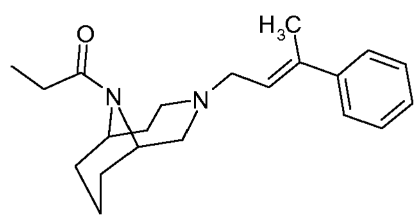

(XXXI)

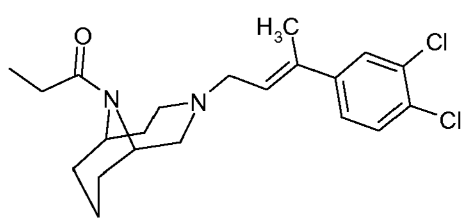

(XXXII)

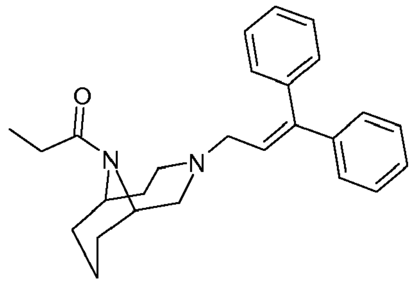

(XXXIII)

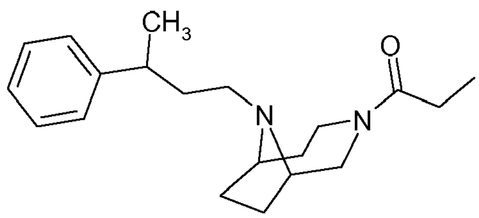

(XXXIV)

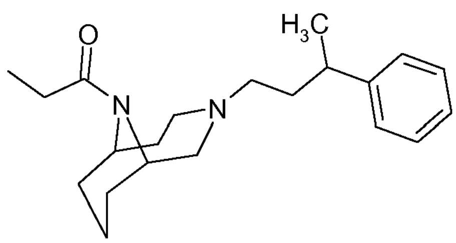

(XXXV)

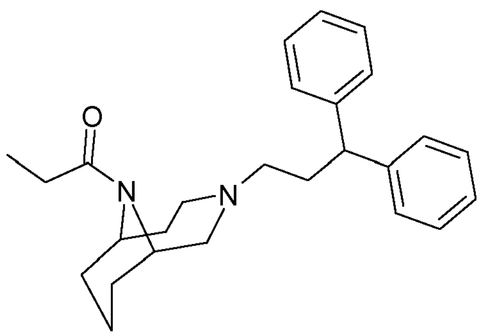

(XXXVI)

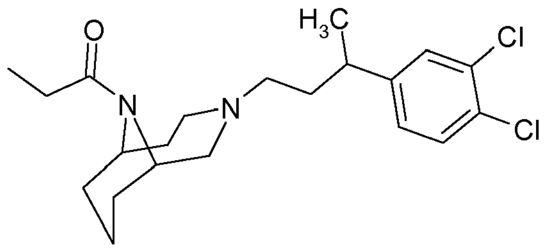

(XXXVII)

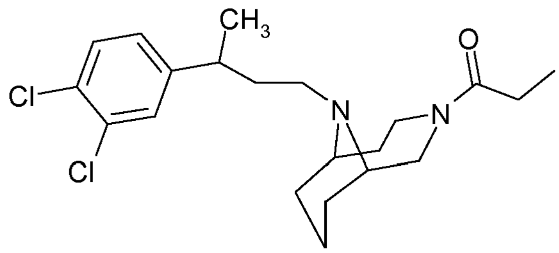

(XXXVIII)

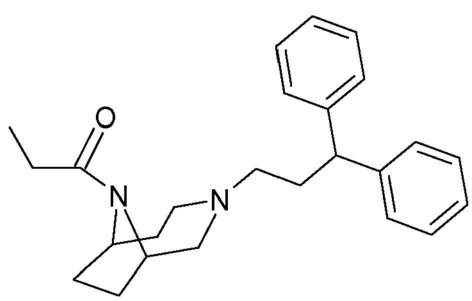

(XXXIX)

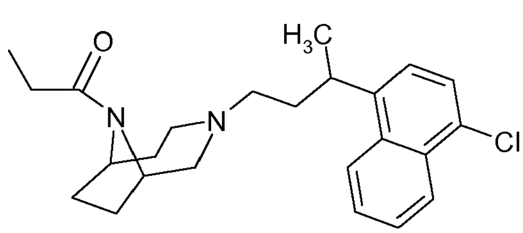

(XXXX)

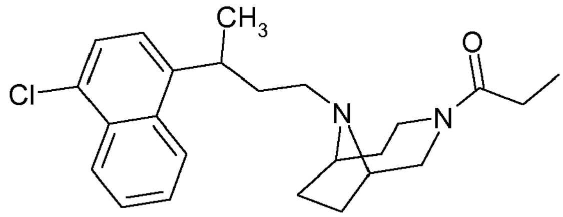

(XXXXI)

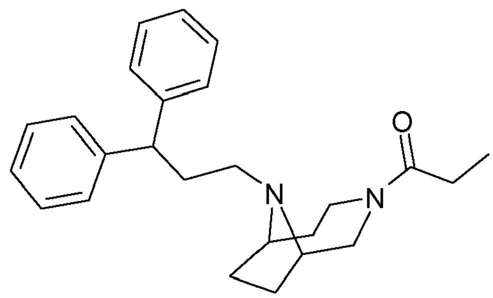

(XXXXII)

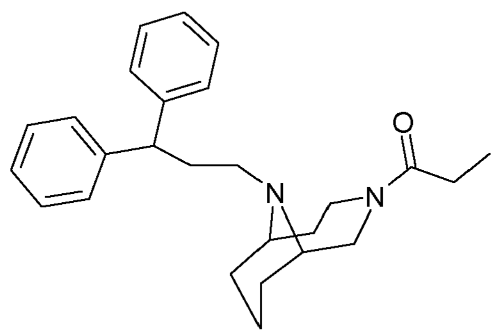

(XXXXIII)

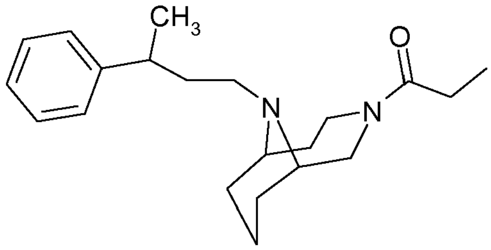

(XXXXIV)

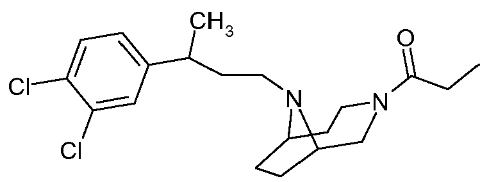

(XXXXV)

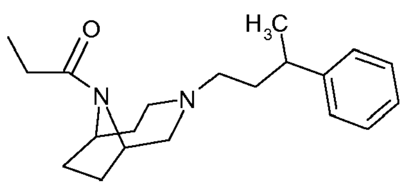

(XXXXVI)

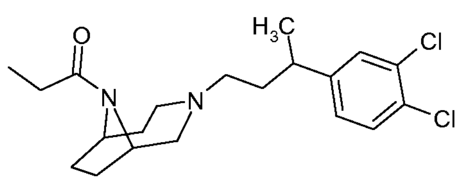

(XXXXVII)

**35.** Isomeric forms, both geometrical and stereoisomers, and their corresponding mixtures, of the compounds according to claims 23-34.

**36.** Microemulsions according to claims 1-19 and of the compounds of claims 23-35.

**37.** Use of the microemulsions according to claim 36 for preparing pharmaceutical compositions.

**38.** Use according to claim 37, wherein the microemulsions comprise the compounds according to claims 23-35.

**39.** Pharmaceutical compositions comprising the microemulsions of claim 36.

**40.** Pharmaceutical compositions according to claim 39 for the therapy and prophylaxis in mammals and in an individual of the diseases and disorders in which the opioidergic receptors μ and/or δ and/or k are involved.

**41.** Pharmaceutical compositions according to claim 40, comprising the compounds according to claims 23-35.

**42.** Use of the compositions according to claim 39 for preparing drugs for the prophylaxis and therapy of pain, post operating pain, chronic pain, neuropathic pain, treatment of abuse (as for example heroin and cocaine abuse), alcoholism, constipation, diarrhoea and other disorders of the gastrointestinal tract, nausea, vomit, dermatitis, obesity and other disorders connected with appetite, depression, smoke dependence (tabagism), sexual disfunctions, shocks, cerebral trauma, spinal damages, eye pathologies and disorders as for example glaucoma and intraocular hypertension, tumours as for example breast cancer.

**43.** Use according to claim 42, wherein the compositions comprise the compounds according to claims 23-35.

**44.** Hydrats and solvents of the compounds of claims 23-35.

Europäisches Patentamt
European Patent Office
Office européen des brevets

# EUROPEAN SEARCH REPORT

Application Number
EP 09 16 1266

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MUNRO, GORDON ET AL: "The novel compound (.+-.)-1-[10-((E)-3-Phenyl-allyl)-3,10-diaza- bicyclo[4.3.1]dec-3-yl]-propan-1-one (NS7051) attenuates nociceptive transmission in animal models of experimental pain; a pharmacological comparison with the combined .mu.-opioid receptor agonist and monoamine reuptake inhibitor trama" NEUROPHARMACOLOGY , 54(2), 331-343 CODEN: NEPHBW; ISSN: 0028-3908, 2008, XP022422948 compound NS7051 * tables 1,3 * * figures * * page 342, left-hand column, lines 1-17 * ----- | 23-33,35 | INV. A61K9/107 C07D487/08 C07D471/08 A61K31/551 A61P25/04 |
| X | RAZDAN, BALKISHEN ET AL: "Studies on azabicyclo systems. Syntheses and spasmolytic activity of analogs of 9-methyl-3,9-diazabicyclo[4.2.1]nonane and 10-methyl-3,10-diazabicyclo[4.3.1]decane" EUROPEAN JOURNAL OF MEDICINAL CHEMISTRY , 22(6), 573-7 CODEN: EJMCA5; ISSN: 0223-5234, 1987, XP023871200 * compound 5 * ----- | 23,31 | TECHNICAL FIELDS SEARCHED (IPC) A61K C07D A61P |
| X | BLACKBURN-MUNRO ET AL: "176 Pharmacological comparison of the combined mu-opioid receptor agonist and monoamine reuptake inhibitor NS7051 with tramadol in animal experimental pain models" EUROPEAN JOURNAL OF PAIN, SAUNDERS, LONDON, GB, vol. 11, no. 1, 12 May 2007 (2007-05-12), page 77, XP022076140 ISSN: 1090-3801 * abstract * ----- -/-- | 23-33,35 | |

1

The present search report has been drawn up for all claims

| Place of search | Date of completion of the search | Examiner |
|---|---|---|
| Munich | 13 November 2009 | Villa Riva, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 16 1266

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | WO 2004/011468 A (NEUROSEARCH AS [DK]; PETERS DAN [DK]; OLSEN GUNNAR M [DK]; NIELSEN ELS) 5 February 2004 (2004-02-05) formula (I) * page 4, lines 10-12 * * page 9, lines 31-33 * * page 13, compound b * ----- | 23-33 | |
| X | US 3 117 132 A (WAGNER HANS A) 7 January 1964 (1964-01-07) * column 1, lines 9-20 * * claim 5 * ----- | 23-30,35 | |
| X | AUDOUZE, KARINE ET AL: "New Ligands with Affinity for the .alpha.4.beta.2 Subtype of Nicotinic Acetylcholine Receptors. Synthesis, Receptor Binding, and 3D-QSAR Modeling" JOURNAL OF MEDICINAL CHEMISTRY , 49(11), 3159-3171 CODEN: JMCMAR; ISSN: 0022-2623, 2006, XP009082276 * compounds 35e,f,g and 35 * ----- | 23-35 | TECHNICAL FIELDS SEARCHED (IPC) |
| D,A | US 2003/195217 A1 (CIGNARELLA GIORGIO [IT] ET AL) 16 October 2003 (2003-10-16) * the whole document * ----- | 1-44 | |
| D,A | US 5 672 601 A (CIGNARELLA GIORGIO [IT]) 30 September 1997 (1997-09-30) * the whole document * ----- | 1-44 | |
| D,A | WO 2005/108402 A (UNIV MILANO [IT]; UNI DEGLI STUDI DI SASSARI [IT]; PINNA GERARD AIME [] 17 November 2005 (2005-11-17) * the whole document * ----- | 1-44 | |

-/--

The present search report has been drawn up for all claims

| Place of search | Date of completion of the search | Examiner |
|---|---|---|
| Munich | 13 November 2009 | Villa Riva, A |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 16 1266

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | PINNA G A ET AL: "N-3(9)-Arylpropenyl-N-9(3)propionyl-3,9-diazabicyclo[3.3.1]nonanes as mu-Opioid Receptor Agonists. Effects on mu-Affinity of Arylalkenyl Chain Modifications" BIOORGANIC AND MEDICINAL CHEMISTRY,, vol. 10, 1 January 2007 (2007-01-01), pages 1929-37, XP002551152 * the whole document * ----- | 1-44 | |

TECHNICAL FIELDS SEARCHED (IPC)

The present search report has been drawn up for all claims

1

| Place of search | Date of completion of the search | Examiner |
|---|---|---|
| Munich | 13 November 2009 | Villa Riva, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 09 16 1266

This annex lists the patent family members relating to the patent documents cited in the above-mentioned European search report.
The members are as contained in the European Patent Office EDP file on
The European Patent Office is in no way liable for these particulars which are merely given for the purpose of information.

13-11-2009

| Patent document cited in search report | Publication date | Patent family member(s) | Publication date |
|---|---|---|---|
| WO 2004011468 A | 05-02-2004 | AT 390427 T | 15-04-2008 |
| | | AU 2003280306 A1 | 16-02-2004 |
| | | CA 2489165 A1 | 05-02-2004 |
| | | CN 1668621 A | 14-09-2005 |
| | | DE 60319989 T2 | 17-07-2008 |
| | | EP 1527075 A1 | 04-05-2005 |
| | | HK 1077827 A1 | 23-05-2008 |
| | | JP 2005536522 T | 02-12-2005 |
| | | MX PA05001002 A | 16-05-2005 |
| | | NZ 537182 A | 28-07-2006 |
| | | US 2005239773 A1 | 27-10-2005 |
| US 3117132 A | 07-01-1964 | NONE | |
| US 2003195217 A1 | 16-10-2003 | AU 3737701 A | 27-08-2001 |
| | | WO 0160823 A1 | 23-08-2001 |
| | | EP 1259511 A1 | 27-11-2002 |
| | | IT MI20000293 A1 | 20-08-2001 |
| US 5672601 A | 30-09-1997 | AU 1808595 A | 11-09-1995 |
| | | WO 9523152 A1 | 31-08-1995 |
| | | EP 0746560 A1 | 11-12-1996 |
| | | IT 1274018 B | 14-07-1997 |
| WO 2005108402 A | 17-11-2005 | EP 1751164 A1 | 14-02-2007 |
| | | JP 2007537182 T | 20-12-2007 |
| | | US 2007225492 A1 | 27-09-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20030195217 A
- WO 2004011468 A
- US 5672601 A
- WO 2005108402 A
- WO 06113468 A
- US 20050203123 A
- WO 05092836 A
- WO 05066164 A
- WO 04089372 A
- WO 04060321 A
- WO 0242309 A
- WO 0146198 A
- US P5672601 A
- US 2003196217 A
- WO 200411468 A
- US P6028084 A


### Non-patent literature cited in the description


- *Bioorganic & Medicinal Chemistry,* 2002, vol. 10, 1929-1937
- *Eur. J. Pharmacol.,* 1996, vol. 296, 199-207
- *Veterinary Ophthalmology,* 2003, vol. 6 (1), 73-76
- *Exp. Eye Res.,* 08 January 2007, vol. 4 (1), 185-190
- *British Journal of Anaesthesia,* 1998, vol. 81, 606-607
- *Neuropeptides,* 1999, vol. 33 (5), 360-368
- **R.K. Mitra.** Physicochemical investigations of microemulsification of eucalyptus oil and water using mized surfactants (AOT+ Brij-35) and butanol. *J. Colloid and Interface Science,* 2005, vol. 283, 565-577
- **C.E. McNamee et al.** Physicochemical Characterization of PEG 1500-12-acyloxy-stearate micelles and liquid cristalline phases. *Langmuir,* 2005, vol. 21, 8146-8154
- **Remington.** *The Science and Practice of Pharmacy,* 1995, vol. II, 1457
- **G. A. Pinna et al.** *Bioorganic & Medicinal Chemistry,* 2002, vol. 10, 1929-1937
- **Ruiu S. et al.** *J. Pharmacol. Exp. Ther.,* 2003, vol. 306 (1), 363-370